# EUROPEAN PATENT APPLICATION

(11) **EP 4 445 912 A1**
(43) Date of publication of application: **16.10.2024**
(21) Application number: 22903520.9
(22) Date of filing: 07.12.2022
(51) Int. Cl.: A61K 39/395, C07K 14/735, A61K 35/17

(54) **P329G ANTIBODY TARGETING BCMA, COMBINATION OF SAME WITH CHIMERIC ANTIGEN RECEPTOR CELL, AND USE THEREOF**

(30) Priority: 07.12.2021 CN 202111487287
(71) Applicant: Innovent Cells Pharmaceuticals (Suzhou) Co., Ltd., Suzhou, Jiangsu 215123 (CN)
(72) Inventor: XU, Wei, Suzhou, Jiangsu 215123 (CN); WEI, Huafeng, Suzhou, Jiangsu 215123 (CN); DAROWSKI, Diana Binia, Suzhou, Jiangsu 215123 (CN); XU, Dan, Suzhou, Jiangsu 215123 (CN); YAO, Ying, Suzhou, Jiangsu 215123 (CN); PRINZ, Bianka, Suzhou, Jiangsu 215123 (CN); BOLAND, Nadthakarn, Suzhou, Jiangsu 215123 (CN); GEOGHEGAN, James, Suzhou, Jiangsu 215123 (CN)
(74) Representative: Sagittarius IP
(86) International application number: PCT/CN2022/137265
(87) International publication number: WO 2023/104099

(57) **Abstract**

A pharmaceutical combination comprising (i) a first component selected from an immune effector cell (e.g., a T cell and an NK cell) expressing a molecular switch-regulated CAR polypeptide, a nucleic acid molecule encoding the CAR polypeptide, a vector comprising the nucleic acid molecule, and any combination thereof; and (ii) a second component, which is an antibody that contains a P329G mutation and specifically binds to a B-cell maturation antigen (BCMA) protein. The present invention also relates to a kit comprising the pharmaceutical combination, and the use of the pharmaceutical combination in the treatment of BCMA-related diseases in a subject.

## Description

### FIELD OF THE INVENTION

The present invention generally relates to the field of antibody engineering and cellular immunology. Particularly, the present invention relates to a combination of an antibody that comprises a P329G mutation and specifically binds to a B-cell maturation antigen (BCMA) protein, with an immune effector cell (e.g., a T cell, an NK cell) engineered to express a molecular switch-regulated chimeric antigen receptor, and to a use of the combination for the treatment of BCMA expression-related diseases, such as cancers that express or overexpress BCMA.

### BACKGROUND

B-cell maturation antigen (BCMA, also named as CD269, TNFRSF17) is a member of the tumor necrosis factor receptor superfamily (TNFRSF). BCMA is a type III transmembrane protein with a characteristic cysteine-rich domain (CRD) of TNFR family members in the extracellular domain (ECD), which forms a ligand-binding motif. The ligands of BCMA include B cell activating factor (BAFF) and B-cell proliferation-inducing ligand (APRIL). B-cell proliferation-inducing ligand (APRIL) binds to BCMA with higher affinity, which promotes tumor cell proliferation.

BCMA is predominantly expressed on the surface of mature B cells, i.e. plasma cells, and is not expressed in normal hematopoietic stem cells, nor in non-blood derived tissues. BCMA signaling is essential for the survival of long-acting bone marrow plasma cells but is not essential for overall B cell homeostasis. BCMA on the membrane surface can be shed by γ-secretase cleavage, and the resulting soluble BCMA (sBCMA) may reduce membrane surface BCMA signaling by blocking BAFF/APRIL ligand binding. BCMA is found to be overexpressed in Multiple Myeloma (MM) cells in preclinical models as well as in human tumors, and it upregulates classical and non-classical NF-κB signaling, promotes MM cell growth, survival, adhesion, and induces osteoclast activation, vascular production, metastasis, and immunosuppression. BCMA expression has become an important marker for the diagnosis of MM. In addition, an elevated serum level of sBCMA in MM patients is positively correlated with the number of MM cells in the bone marrow, and changes in its concentration are closely related to MM prognosis and treatment response.

Given that BCMA is only expressed in plasma cells and is not expressed in natural and memory B cells, BCMA has become a popular target for the treatment of MM. Multiple targeted drugs have been developed, including those for a chimeric antigen receptor T cell (CAR-T) immunotherapy, such as Bluebird's Abecma (idecabtagene vicleucel, ide-cel), which was approved by the FDA in March 2021 for relapsed refractory MM (RRMM) as fourth and above line treatment, and Ciltacabtagene autoleucel (Cilta-cel), jointly developed by Johnson & Johnson and Nanjing Legend, which has also submitted a Biologics License Application (BLA).

Abecma, as the first CAR-T cell therapy targeting BCMA, leads to the death of cancer cells expressing BCMA protein by recognizing and binding to BCMA proteins on multiple myeloma cancer cells. That is, Abecma recognizes and kills tumor cells by directly targeting the surface antigen BCMA proteins on tumor cells through the chimeric antigen receptor (CAR) on the CAR-T cells. The limitation of this conventional chimeric antigen receptor T cells is the lack of control over the activity of CAR-T cells after reinfusion into the body. After activation in vivo, these CAR-T cells will continue recognizing and killing cells expressing their target antigens, and in some cases (e.g., tumor patients with larger tumor loads), a large number of rapidly activated CAR-T cells will release massive amounts of inflammatory cytokines, causing patients to develop serious toxic side effects such as cytokine release syndrome (CRS) and neurotoxicity (NT). A meta-analysis of 84 clinical trials comprising 2592 patients shows that the incidences of grade 3 or higher CRS and NT after conventional CAR-T cell therapy are 29% and 28%, respectively. Although a large number of clinical trials show that most patients who experienced toxic effects recover quickly after receiving appropriate treatment and the antitumor efficacy is not significantly affected, the management of toxic effects consumes a large amount of clinical resources and increases the physical and psychological burden and economic burden of patients.

Since the vast majority of CAR-T cells (e.g., Abecma) target tumor-associated antigens (TAAs) rather than tumor-specific antigens (TSAs), and these TAAs are often expressed at low levels in many normal tissues, especially vital tissues and organs, in addition to being expressed on tumor cells, CAR-T cell recognition and killing of the normal tissues leads to "On-target/off tumor" toxicity, which may be tolerated by the body in the short term, but may lead to serious side effects if a recovery or a remission is not achieved in the long-term. For example, CD19 CAR-T cells targeting CD 19 are used clinically to treat CD 19-positive hematologic tumors, which in addition to removing tumor cells, also kill normal B cells. Although the continued presence of CAR-T cells in the body is closely related to a long-term relapse-free survival, it also leads to a long-term B cell dysplasia and humoral immune deficiency, and to easily caused infections. Clinical trials have shown that 27-36% of lymphoma patients develop bacterial infections within 30 days after receiving CD19 CAR-T cell therapy, and 9.2-28% of patients develop viral infections after one month. It requires a median time of 6.7 months for B-cell recovery, and 31-64% of patients require gamma-globulin replacement therapy. In addition, continuous activation of CAR-T cells in vivo tends to lead to functional depletion, impairing the antitumor effects and reducing survival of CAR-T cells in vivo, thereby reducing long-term therapeutic effects.

In order to reduce the "on-target/off tumor" toxicity of CAR-T cells, the following strategies are commonly used in the prior art. One strategy is to design a CAR to comprise an antigen-binding domain targeting a target antigen that is highly expressed on the tumor surface but not expressed or under-expressed in normal tissues, during the process of CAR design. Another strategy is to strictly control the dose of T cells administered, since excessive CAR-T cells, upon stimulation by an antigen, will proliferate exponentially and are more likely to cause on-target/off-tumor effects.

Further strategy is to introduce an inducible suicide gene such as an inducible Caspase-9 (iCasp9) suicide gene during the construction of a CAR, and when on-target/off-tumor toxicity is observed in patients, to administer AP1903 (a dimerizing chemical inducer that activates iCasp9) to cause apoptosis in CAR-T cells, thereby mitigating the toxicity (Zhang Huihui et al., Preclinical research on suicide gene as "safety switch" to control CAR-T cell toxicity, Chinese Journal of Cancer Biotherapy, 2021, 28(3): 225-231); there is also a report of using the herpes simplex virus thymidine kinase (HSV-TK) gene as a suicide gene to induce apoptosis in CAR-T cells through treatment with ganciclovir administration. Additionally, there is also a report of using an electroporation transfection method to make T cells transiently express CAR, exerting therapeutic effects through transient killing function (Kenderian SS et al., CD33-specific chimeric antigen receptor T cells exhibit potent preclinical activity against human acute myeloid leukemia[J] Leukemia. 2015; 29(8): 1637-1647). However, the above regulation methods are all carried out after toxicity occurs, and the toxicity relief effect depends on the effectiveness and efficiency of the drug in removing CAR-T cells. Moreover, the long-term anti-tumor efficacy is affected as the CAR-T cells cannot be recovered after removal.

### SUMMARY OF THE INVENTION

Conventional CAR-T cells targeting BCMA still face toxicity and relapse problems in the treatment of MM. A meta-study shows that the incidences of CRS and NT after treatment with conventional CAR-T cells targeting BCMA are 74% (95% CI, 56-91%) and 34% (95% CI, 24-43%), with the incidences of grade 3 or higher events at 25% (95% CI, 7-43%) and 12% (95% CI, 4-20%). In addition, the treated patients have an increased incidence of infections due to impaired humoral immunity caused by plasma cell deficiency. 69% of 128 patients with relapsed refractory multiple myeloma (RRMM) treated with ide-cel develop infections, with a 22% incidence of grade 3 or higher infections, and most patients require anti-infective, leukocyte-raising growth factor and immunoglobulin replacement therapies. Similarly, 56% and 20% of the 97 RRMM patients treated with Cilta-cel develop infections and grade 3 or higher infections, respectively. These clinical results suggest the need for the development of a controllable BCMA-targeted CAR-T cell therapy.

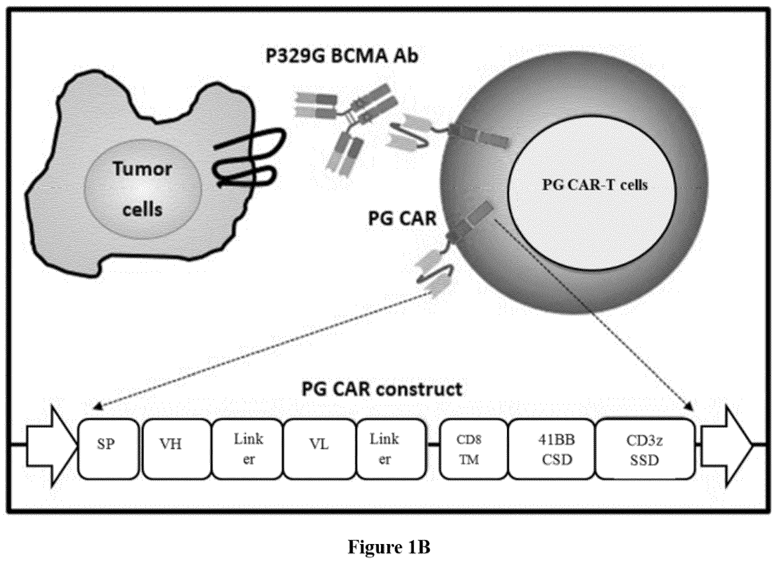

After studying, the present inventors have designed and constructed P329G CAR-T cells that target BCMA via binding to the Fc domain of an antibody comprising a P329G mutation and specifically binding to the BCMA molecule, and have validated their specific and controlled anti-tumor effects in vitro and in vivo. Unlike conventional CAR-T cells that target BCMA via direct recognization and kill MM cells, the pharmaceutical combination of the present invention comprises two components: a BCMA-specific P329G antibody and a P329G CAR-T cell. After the BCMA-specific P329G antibody recognizes BCMA-expressing tumor cells, the P329G CAR-T cell recognizes the Fc domain of the P329G antibody and re-targets to tumor cells, leading to tumor recognition and killing effect (see Figure 1B). In therapeutic approach using the pharmaceutical combination of the present invention, the P329G antibody acts as a bridge between P329G CAR-T cells and tumor cells, playing a "molecular switch" role to regulate P329G CAR-T cell activity.

Therefore, in a first aspect, the present invention provides a P329G mutated antibody capable of specifically binding to the BCMA molecule and acting as a "molecular switch" for immune effector cells (e.g., T cells, NK cells) expressing CAR polypeptides, including but not limited to ADI-38497 PG Ab (also referred to herein as "ADI-38497 PG antibody", "38497 PG Ab", "ADI-38497 PG IgG", "38497 PG IgG", "PG 38497 antibody"), ADI-38484 PG Ab (also referred to herein as "ADI-38484 PG antibody", "38484 PG Ab", "ADI-38484 PG IgG", "38484 PG IgG", "PG 38484 antibody").

In some embodiments, the present invention obtains an antibody or antigen-binding fragments that specifically binds to the BCMA molecule, comprising a heavy chain variable region and a light chain variable region, wherein,
(a) the heavy chain variable region comprises according to the Kabat numbering CDR H1 shown in amino acid sequence of SSSYYWT (SEQ ID NO: 25); or a variant of the CDR H1 with no more than 2 amino acid changes or no more than 1 amino acid change; CDR H2 shown in amino acid sequence of SISIAGSTYYNPSLKS(SEQ ID NO: 26); or a variant of the CDR H2 with no more than 2 amino acid changes or no more than 1 amino acid change; and CDR H3 shown in amino acid sequence of DRGDQILDV (SEQ ID NO: 27); or a variant of the CDR H3 with no more than 2 amino acid changes or no more than 1 amino acid change; the light chain variable region comprises according to the Kabat numbering CDR L1 shown in amino acid sequence of RASQSISRYLN(SEQ ID NO: 28); or a variant of the CDR L1 with no more than 2 amino acid changes or no more than 1 amino acid change; CDR L2 shown in amino acid sequence of AASSLQS(SEQ ID NO: 29); or a variant of the CDR L2 with no more than 2 amino acid changes or no more than 1 amino acid change; and CDR L3 shown in amino acid sequence of QQKYFDIT(SEQ ID NO: 30); or a variant of the CDR L3 with no more than 2 amino acid changes or no more than 1 amino acid change;
(b) the heavy chain variable region comprises according to the Kabat numbering CDR H1 shown in amino acid sequence of NDVIS (SEQ ID NO: 31); or a variant of the CDR H1 with no more than 2 amino acid changes or no more than 1 amino acid change; CDR H2 shown in amino acid sequence of VIIPIFGIANYAQKFQG(SEQ ID NO: 32); or a variant of the CDR H2 with no more than 2 amino acid changes or no more than 1 amino acid change; and CDR H3 shown in amino acid sequence of GRGYYSSWLHDI(SEQ ID NO: 33); or a variant of the CDR H3 with no more than 2 amino acid changes or no more than 1 amino acid change; the light chain variable region comprises according to the Kabat numbering CDR L1 shown in amino acid sequence of QASQDITNYLN(SEQ ID NO: 34); or a variant of the CDR L1 with no more than 2 amino acid changes or no more than 1 amino acid change; CDR L2 shown in amino acid sequence of DASNLET(SEQ ID NO: 35); or a variant of the CDR L2 with no more than 2 amino acid changes or no more than 1 amino acid change; and CDR L3 shown in amino acid sequence of QQAFDLIT(SEQ ID NO: 36); or a variant of the CDR L3 with no more than 2 amino acid changes or no more than 1 amino acid change;
wherein the amino acid change is an addition, deletion or substitution of an amino acid.

In some embodiments, the invention provides an antibody or antigen-binding fragments that specifically binds to the BCMA molecule, comprising a heavy chain variable region and a light chain variable region, wherein,
(a) the heavy chain variable region comprises according to the Kabat numbering CDR H1 shown in amino acid sequence of SSSYYWT (SEQ ID NO: 25); CDR H2 shown in amino acid sequence of SISIAGSTYYNPSLKS(SEQ ID NO: 26); and CDR H3 shown in amino acid sequence of DRGDQILDV (SEQ ID NO: 27); the light chain variable region comprises according to the Kabat numbering CDR L1 shown in amino acid sequence of RASQSISRYLN(SEQ ID NO: 28); CDR L2 shown in amino acid sequence of AASSLQS(SEQ ID NO: 29); and CDR L3 shown in amino acid sequence of QQKYFDIT(SEQ ID NO: 30);
(b) the heavy chain variable region comprises according to the Kabat numbering CDR H1 shown in amino acid sequence of NDVIS(SEQ ID NO: 31); CDR H2 shown in amino acid sequence of VIIPIFGIANYAQKFQG(SEQ ID NO: 32); and CDR H3 shown in amino acid sequence of GRGYYSSWLHDI(SEQ ID NO: 33); the light chain variable region comprises according to the Kabat numbering CDR L1 shown in amino acid sequence of QASQDITNYLN(SEQ ID NO: 34); CDR L2 shown in amino acid sequence of DASNLET(SEQ ID NO: 35); and CDR L3 shown in amino acid sequence of QQAFDLIT(SEQ ID NO: 36).

In some embodiments, the present invention obtains an antibody or antigen-binding fragments that specifically binds to the BCMA molecule comprising a heavy chain variable region and a light chain variable region, wherein,
(a) the heavy chain variable region comprises a sequence shown in SEQ ID NO: 2, or a sequence having at least 90%, 91%, 92%, 93%, 94%, 95%, 96%, 97%, 98% or 99% identity thereto, and the light chain variable region comprises a sequence shown in SEQ ID NO: 3, or a sequence having at least 90%, 91%, 92%, 93%, 94%, 95%, 96%, 97%, 98% or 99% identity thereto;
(b) the heavy chain variable region comprises a sequence shown in SEQ ID NO: 9, or a sequence having at least 90%, 91%, 92%, 93%, 94%, 95%, 96%, 97%, 98% or 99% identity thereto, and the light chain variable region comprises a sequence of SEQ ID NO: 10, or a sequence having at least 90%, 91%, 92%, 93%, 94%, 95%, 96%, 97%, 98% or 99% identity thereto.

In some embodiments, the present invention obtains an antibody or antigen-binding fragments that specifically binds to the BCMA molecule, comprising a heavy chain variable region and a light chain variable region, wherein,
(a) the heavy chain variable region comprises a sequence shown in SEQ ID NO: 2, and the light chain variable region comprises a sequence shown in SEQ ID NO: 3;
(b) the heavy chain variable region comprises a sequence shown in SEQ ID NO: 9, and the light chain variable region comprises a sequence shown in SEQ ID NO: 10.

In some embodiments, the antibody of the present invention that specifically binds to the BCMA molecule is an IgG1, IgG2, IgG3 or IgG4 antibody; preferably, an IgG1 or IgG4 antibody; more preferably, an IgG1 antibody.

In some embodiments, the antigen-binding fragment of the antibodies of the present invention that specifically binds to the BCMA molecule is Fab, Fab', F(ab')₂, Fv, single chain Fv, single chain Fab or diabody.

With respect to the antibody or the antigen-binding fragment that specifically binds to the BCMA molecule, it is an antibody having a mutated Fc domain, which is obtained by mutating the amino acid at position P329 according to the EU number to glycine (G), wherein the mutated Fc domain has a decreased binding to Fcy receptor, compared with the binding of a parent antibody Fc domain without the mutation to Fcy receptor.

In some embodiments, the mutated Fc domain is a mutated Fc domain of an IgG1, IgG2, IgG3 or IgG4 antibody, preferably, the mutated Fc domain is a mutated Fc domain of an IgG1 or IgG4 antibody; more preferably, the mutated Fc domain is a mutated Fc domain of an IgG1 antibody.

In some embodiments, the antibody or antigen-binding fragment that specifically binds to the BCMA molecule comprises a heavy chain constant region sequence shown in SEQ ID NO: 5, or a sequence having at least 90%, 91%, 92%, 93%, 94%, 95%, 96%, 97%, 98% or 99% identity thereto, and wherein the amino acid at P329 position according to the EU numbering is mutated into G

For example, the antibody or antigen-binding fragment comprises a heavy chain constant region sequence shown in SEQ ID NO: 5, or a sequence having at least 90%, 91%, 92%, 93%, 94%, 95%, 96%, 97%, 98% or 99% identity thereto, and wherein the amino acid at P329 position according to the EU numbering is mutated into G; and a light chain constant region sequence shown in SEQ ID NO: 6, or a sequence having at least 90%, 91%, 92%, 93%, 94%, 95%, 96%, 97%, 98% or 99% identity thereto.

In one embodiment, the antibody or antigen-binding fragment that specifically binds to the BCMA molecule comprises a heavy chain constant region sequence shown in SEQ ID NO: 5 and a light chain constant region sequence shown in SEQ ID NO: 6

In a second aspect, the present invention provides a nucleic acid encoding the antibody in the first aspect of the invention, a vector comprising the nucleic acid encoding the antibody, a host cell comprising the nucleic acid molecule or the vector, and a method for preparing the antibody. The method comprises the following steps: under conditions suitable for expressing the nucleic acid encoding the antibody that specifically binds to BCMA molecule or antigen-binding fragment thereof according to the first aspect of the invention, cultivating and introducing a host cell comprising an expression vector comprising the nucleic acid encoding the antibody that specifically binds to BCMA molecule or antigen-binding fragment thereof according to the first aspect of the invention, isolating the antibody that specifically binds to BCMA molecule or antigen-binding fragment thereof. Optionally, the method further comprises recovering the antibody that specifically binds to BCMA molecule or antigen-binding fragment thereof from the host cell. Preferably, the host cell is prokaryotic or eukaryotic, more preferably selected from Escherichia coli cells, yeast cells, mammalian cells or other cells suitable for producing the antibody or antigen-binding fragment thereof, and most preferably, the host cell is HEK293 cell or CHO cell.

In a third aspect, the present invention provides a pharmaceutical combination, comprising
(i) a first component, selected from an immune effector cell (e.g., a T cell and an NK cell) expressing a molecular switch-regulated CAR polypeptide, a nucleic acid molecule encoding the CAR polypeptide, a vector comprising the nucleic acid molecule, and any combination thereof; and
(ii) a second component, which is an antibody or an antigen binding fragment that comprises a P329G mutation and specifically binds to BCMA molecule (also designated as a P329G mutated antibody), for example, the P329G mutated antibody of the first aspect of the present invention, and

Optionally a pharmaceutically acceptable auxiliary material;
Wherein, the molecular switch-regulated CAR polypeptide comprises
(1) a humanized anti-P329G mutation scFv sequence, wherein the scFv sequence comprises the following sequences capable of specifically binding to an antibody Fc domain comprising a P329G mutation, but not capable of specifically binding to a parent antibody Fc domain without the mutation:
   (i) a heavy chain variable region, which comprises according to the Kabat numbering
      (a) a heavy chain complementarity determining region CDR H1 shown in amino acid sequence of RYWMN (SEQ ID NO: 19), or a variant of the CDR H1 with no more than 2 amino acid changes or no more than 1 amino acid change;
      (b) a CDR H2 shown in amino acid sequence of EITPDSSTINYAPSLKG(SEQ ID NO: 20); or a variant of the CDR H2 with no more than 2 amino acid changes or no more than 1 amino acid change; and
      (c) a CDR H3 shown in amino acid sequence of PYDYGAWFAS(SEQ ID NO: 21); or a variant of the CDR H3 with no more than 2 amino acid changes or no more than 1 amino acid change; and
   (ii) a light chain variable region, which comprises according to the Kabat numbering
      (d) a light chain complementarity determining region (CDR L)1 shown in amino acid sequence of RSSTGAVTTSNYAN(SEQ ID NO: 22); or a variant of the CDR L1 with no more than 2 amino acid changes or no more than 1 amino acid change;
      (e) a CDR L2 shown in amino acid sequence of GTNKRAP(SEQ ID NO: 23); or a variant of the CDR L2 with no more than 2 amino acid changes or no more than 1 amino acid change; and
      (f) a CDR L3 shown in amino acid sequence of ALWYSNHWV(SEQ ID NO: 24); or a variant of the CDR L3 with no more than 2 amino acid changes or no more than 1 amino acid change; wherein the amino acid change is an addition, deletion or substitution of an amino acid;
(2) a hinge region/a spacer region, selected from a group consisting of
   (i) a (G₄S)ₙ, (SG₄)ₙ, or G₄(SG₄)ₙ peptide linker, wherein "n" is an integer from 1 to 10, such as an integer from 1 to 4; for example, a sequence shown in SEQ ID NO: 14;
   (ii) a CD8α hinge region, or a variant thereof having 1-5 amino acid modifications, for example, a sequence shown in SEQ ID NO: 18 or a variant thereof having 1-2 amino acid modifications;
(3) a transmembrane region (TM), selected from a group consisting of CD8 transmembrane domain, or a variant thereof having 1-5 amino acid modifications, for example, a sequence shown in SEQ ID NO: 15, or a variant thereof having 1-2 amino acid modifications;
(4) a co-stimulatory signal domain (CSD), selected from a group consisting of 4-1BB co-stimulatory domain, or a variant thereof having 1-5 amino acid modifications, for example, a sequence shown in SEQ ID NO: 16, or a variant thereof having 1-2 amino acid modifications;
(5) a stimulatory signal domain (SSD), which is a CD3ζ signaling domain, or a variant thereof having 1-10 amino acid modifications, for example, a sequence shown in SEQ ID NO: 17, or a variant thereof having 1-10, or 1-5 amino acid modifications;
   wherein the amino acid modification is an addition, deletion or substitution of an amino acid.

In some embodiments, the molecular switch-regulated CAR polypeptide in the pharmaceutical combination of the present invention comprises
(1) a humanized anti-P329G mutation scFv sequence, wherein the scFv sequence comprises the following sequences capable of specifically binding to an antibody Fc domain comprising a P329G mutation, but not capable of specifically binding to a parent antibody Fc domain without the mutation:
   (i) a heavy chain variable region, which comprises according to the Kabat numbering
      (a) CDR H1 shown in amino acid sequence of RYWMN(SEQ ID NO: 19);
      (b) CDR H2 shown in amino acid sequence of EITPDSSTINYAPSLKG(SEQ ID NO: 20); and
      (c) CDR H3 shown in amino acid sequence of PYDYGAWFAS(SEQ ID NO: 21); and
   (ii) a light chain variable region, which comprises according to the Kabat numbering
      (d) CDR L1 shown in amino acid sequence of RSSTGAVTTSNYAN(SEQ ID NO: 22);
      (e) CDR L2 shown in amino acid sequence of GTNKRAP(SEQ ID NO: 23); and
      (f) CDR L3 shown in amino acid sequence of ALWYSNHWV(SEQ ID NO: 24);

   for example, (i) a heavy chain variable region, which comprises a sequence shown in SEQ ID NO: 12, or a sequence having at least 90%, 91%, 92%, 93%, 94%, 95%, 96%, 97%, 98% or 99% identity thereto, and
   (ii) a light chain variable region, which comprises a sequence shown in SEQ ID NO: 13, or a sequence having at least 90%, 91%, 92%, 93%, 94%, 95%, 96%, 97%, 98% or 99% identity thereto;
   for example, (i) a heavy chain variable region, which comprises a sequence shown in SEQ ID NO: 12, and (ii) a light chain variable region, which comprises a sequence shown in SEQ ID NO: 13;
(2) a hinge region/a spacer region, selected from a group consisting of
   (i) a (G₄S)ₙ, (SG₄)ₙ, or G₄(SG₄)ₙ peptide linker, wherein "n" is an integer from 1 to 4, for example, a sequence shown in SEQ ID NO: 14;
   (ii) a CD8α hinge region sequence shown in SEQ ID NO: 18, or a variant thereof having 1 amino acid modification
(3) a transmembrane region (TM), selected from a group consisting of CD8 transmembrane domain shown in SEQ ID NO: 15, or a variant thereof having 1 amino acid modification
(4) a co-stimulatory signal domain (CSD), selected from a group consisting of 4-1BB co-stimulatory domain shown in SEQ ID NO: 16, or a variant thereof having 1 amino acid modification
(5) a stimulatory signal domain (SSD), selected from a group consisting of CD3ζ signaling domain shown in SEQ ID NO: 17, or a variant thereof having 1 amino acid modification wherein the amino acid modification is an addition, deletion or substitution of an amino acid.

In some embodiments, the molecular switch-regulated CAR polypeptide in the pharmaceutical combination of the present invention further comprises a signal peptide sequence at N-terminus, for example, a signal peptide sequence shown in SEQ ID NO: 11.

In some embodiments, the molecular switch-regulated CAR polypeptide in the pharmaceutical composition of the present invention has an amino acid sequence shown in SEQ ID NO: 1, or a sequence having at least 90%, 91%, 92%, 93%, 94%, 95%, 96%, 97%, 98% or 99% identity thereto.

In a fourth aspect, the present invention provides a nucleic acid encoding the molecular switch-regulated CAR polypeptide in the pharmaceutical combination of the present invention, a vector comprising the nucleic acid encoding the CAR polypeptide, and a cell comprising the CAR nucleic acid molecule or the vector, or a cell expressing the CAR polypeptide, preferably the cell is an autologous T cell or an allogeneic T cell.

In some embodiments, the nucleic acid molecule encoding the molecular switch-regulated CAR polypeptide in the pharmaceutical combination of the present invention is a nucleic acid molecule encoding the amino acid sequence shown in SEQ ID NO: 1 or an amino acid sequence having at least 90%, 91%, 92%, 93%, 94%, 95%, 96%, 97%, 98%, or 99% identity thereto.

In some embodiments, the vector comprising the nucleic acid molecule encoding the molecular switch-regulated CAR polypeptide in the pharmaceutical combination of the present invention is selected from a group consisting of a DNA vector, an RNA vector, a plasmid, a lentiviral vector, an adenovirus vector, or a retroviral vector.

In some embodiments, the immune effector cell in the pharmaceutical combination of the present invention is a T cell, NK cell prepared from an autologous T cell, NK cell, or an allogeneic T cell, NK cell, and expressing the molecular switch-regulated CAR polypeptide in the pharmaceutical combination of the present invention, for example, the immune effector cell is prepared from T cell, NK cell isolated from human peripheral blood mononuclear cells (PBMC) and expressing the molecular switch-regulated CAR polypeptide described herein.

In some embodiments, the effector function of the P329G CAR-T cells mediated by antibody to fight against BCMA-expressing tumor cells was evaluated in an in vitro co-culture system using primary P329G CAR-T cells prepared from human PBMCs from several different donor sources and a humanized anti-BCMA antibody having a P329G mutation, whereby the P329G mutant anti-BCMA antibody can act as a "molecular switch" to regulate the recognition and killing activity of the P329G CAR-T cells against BCMA-positive tumor cells, and the in vitro effector function is comparable to that of conventional CAR-T cells directly targeting BCMA-positive tumor cells, but the activity of conventional CAR-T cells does not depend on the P329G mutated antibody.

Further in some embodiments, the invention validated the anti-tumor effect of the P329G CAR-T cells combined with the P329G mutated antibody in immunodeficient mice inoculated with BCMA-expressing positive human tumor cell line as tumor source, and investigated the dose and interval and the like of the antibody administration.

In vitro and in vivo experiments show that only in the presence of a P329G antibody, can P329G CAR-T cells "turn on" their activity and produce effector function to recognize and kill BCMA-positive tumor cells; by adjusting the dose and dosing interval of a P329G antibody, P329G CAR-T cells can be regulated on the expansion degree and the intensity of their anti-tumor activity in vivo. By regulating the dose and interval of the P329G antibody administration, the extent of expansion and the intensity of anti-tumor activity of the P329G CAR-T cells can be regulated. In view that the P329G antibody can exert anti-tumor effect by blocking the pro-tumor signaling transmitted by the binding of BCMA to its ligand, the BCMA-specific P329G antibody can also lead to a synergistic anti-tumor effect when combined with P329G CAR-T cells.

In some embodiments, for the pharmaceutical combination of the present invention, (i) the immune effector cell expressing the CAR polypeptide of the present invention is administered intravenously one or more times to a subject at a dose of 1×10⁶ cells/kg body weight - 10×10⁶ cells/kg body weight, for example 1×10⁶ cells/kg body weight, 2×10⁶ cells/kg body weight, 3×10⁶ cells/kg body weight, 5×10⁶ cells/kg body weight, 7×10⁶ cells/kg body weight, 9×10⁶ cells/kg body weight, 10×10⁶ cells/kg body weight; and
(ii) the P329G mutated antibody in the pharmaceutical combination of the present invention is administered, preferably parenterally, more preferably intravenously, to a subject in a form of a dose unit of 0.1-10mg/kg, preferably 0.1mg/kg, 0.3mg/kg, 0.5mg/kg, 1mg/kg, 3mg/kg, 5mg/kg, 7mg/kg, 9mg/kg, 10mg/kg.

In some embodiments, (i) and (ii) are administered separately, simultaneously, or sequentially, e.g., (ii) is administered on the first day, (i) is administered intravenously on the same day, and then (ii) is administered multiple times at a certain frequency, meanwhile an in vivo PK concentration of (i) and a desired therapeutic efficacy endpoint are monitored to determine whether to administer (i) multiple times; or
(i) is administered on the first day, (ii) is administered intravenously on the second day, and then (ii) is administered multiple times at a certain frequency, meanwhile an in vivo PK concentration of (i) and a desired therapeutic efficacy endpoint are monitored to determine whether to administer (i) multiple times; or
(ii) is administered on the first day, (i) is administered intravenously on the second day, and then (ii) is administered multiple times at a certain frequency, meanwhile an in vivo PK concentration of (i) and a desired therapeutic efficacy endpoint are monitored to determine whether to administer (i) multiple times;

For example, (i) and (ii) are each administered once, and then (ii) is administered multiple times at a frequency of once every 3-4 days, once a week, once every two weeks, once every three weeks, or once every four weeks, meanwhile an in vivo PK concentration of (i) and a desired therapeutic efficacy endpoint are monitored to determine whether to administer (i) multiple times.

In a fourth aspect, the present invention provides a use of the pharmaceutical combination of the present invention for the treatment of BCMA-related diseases in a subject, comprising administering to the subject a therapeutically effective amount of the pharmaceutical combination as defined in the preceding third aspect, wherein the BCMA-related disease is, for example, a cancer expressing or overexpressing BCMA, for example, the cancer is relapsed/refractory multiple myeloma (RRMM).

In a fifth aspect, the present invention provides for a use of the pharmaceutical combination of the present invention in the manufacture of a medicament for the treatment of a BCMA-related disease, for example, the BCMA-related disease is a cancer expressing or overexpressing BCMA, for example, the cancer is relapsed/refractory multiple myeloma (RRMM).

In a sixth aspect, the present invention provides a method for treating a disease associated with BCMA, the methods comprising administering to a subject a therapeutically effective amount of the pharmaceutical combination of the present invention, for example, the BCMA-related disease is a cancer expressing or overexpressing BCMA, for example, the cancer is relapsed/refractory multiple myeloma (RRMM).

In a seventh aspect, the present invention provides a kit of parts, comprising the pharmaceutical combination as defined in the preceding third aspect, preferably the kit of parts is in the form of a pharmaceutical dose unit.

In an eighth aspect, the present invention provides a pharmaceutical complex, which is formed by the binding of
(i) an immune effector cell (e.g., a T cell and an NK cell) expressing a molecular switch-regulated CAR polypeptide in the pharmaceutical combination of the present invention; to
(ii) an antibody or an antigen binding fragment that comprises a P329G mutation and specifically binds to BCMA molecule (also designated as a P329G mutated antibody), for example, the P329G mutated antibody of the first aspect of the present invention,

Wherein the complex is formed by binding of the humanized anti-P329G mutation scFv sequence in the extracellular domain of the CAR polypeptide in the immune effector cell to the Fc domain of the P329G mutated antibody;
For example, wherein the immune effector cell is a T cell prepared from an autologous T cell or an allogeneic T cell, and expressing the molecular switch-regulated CAR polypeptide in the pharmaceutical combination of the present invention, for example, the immune effector cell is prepared from T cell isolated from human PBMC and expressing the molecular switch-regulated CAR polypeptide of the present invention, for example, wherein the P329G mutated antibody is ADI-38497 PG Ab and/or ADI-38484 PG Ab.

The present invention also provides a use of the pharmaceutical complex for the treatment of a BCMA-related disease in a subject, preferably the BCMA-related disease is, for example, a cancer expressing or overexpressing BCMA, for example, the cancer is relapsed/refractory multiple myeloma (RRMM).

Thus, the present invention firstly, in a first aspect, obtains a high-affinity BCMA-specific P329G antibody through in vitro binding ability, affinity and Fc effector function assays, which is able to bind to both BCMA antigen and P329G CAR molecule and exerts a bridging effect. Secondly, in a third aspect, the present invention uses the constructed P329G CAR structural molecule and combines P329G CAR-T cells prepared by using the CAR molecule with BCMA-specific P329G antibody, and then co-cultures them with BCMA-positive MM cells in vitro to construct an in vitro co-culture system. In this system, the effect of P329G antibody as a "molecular switch" to regulate the activity of the P329G CAR-T cells is verified, i.e., P329G CAR-T cells can be activated, to proliferate, secrete effector cytokines and produce killing effect, only in the presence of the P329G mutated antibody, and these effects are dose dependent on the P329G antibody. The P329G CAR-T cell recognition and killing effects are enhanced with the increasing antibody doses. In contrast, WT antibody not carrying P329G mutation does not stimulate P329G CAR-T cell effector functions.

Furthermore, in vitro experiments of the present invention have shown that soluble BCMA antigen does not affect the activity of the P329G CAR-T cells combined with BCMA-specific P329G antibody, while soluble BCMA antigen leads to a significant inhibitory effect on conventional CAR-T cells. In subcutaneous as well as systemic tumor-bearing immunodeficient mouse models inoculated with MM cells having a high or a low BCMA expression, P329G CAR-T cells in combination with P329G antibody in the present invention produce a good antitumor effect, which is at least comparable to that of conventional CAR-T cells, but the in vivo expansion of the P329G CAR-T cells is significantly lower than that of conventional CAR-T cells, suggesting that P329G CAR-T cells achieve a substantial anti-tumor effect, meanwhile may induce lower CRS, NT and other acute toxic side effects. Further, by adjusting the dose and interval of P329G antibody administration, the degree of in vivo expansion and the intensity of the anti-tumor effect of the P329G CAR-T cells can be regulated. A sustained anti-tumor effect can be maintained after tumor clearance and discontinuation of P329G antibody administration. This provides a basis for clinic to "turn off" P329G CAR-T cell activity and maintain a sustained antitumor effect while restoring normal plasma cell number and humoral immunity to reduce long-term toxic side effects such as infection.

Toxicological experiments of the present invention also show that P329G CAR-T cells in combination with P329G antibody produced no significant toxicity, thus laying the foundation for clinical transformation.

### BRIEF DESCRIPTION OF THE DRAWINGS

When read together with the following drawings, the preferred embodiments of the present invention described in detail below will be better understood. For the purpose of illustrating the invention, the currently preferred embodiments are shown in the drawings. However, it should be understood that the present invention is not limited to the precise arrangement and means of the embodiments shown in the drawings.
Figure 1A shows the expressions of CARs on CD3⁺ cells, CD4⁺, and CD8⁺ T cell subsets after transduction of T cells with HuR968B, Blue21 CARs constructed in Example 1-1.
Figure 1B shows the action mechanism of targeting of BCMA-expressing target cells via P329G CAR-T cells mediated by P329G antibody. The structure of 8 CAR constructs is shown. In the figure, "SP" denotes signal peptide; "TMD" denotes transmembrane domain; "ICD" denotes intracellular domain; "CSD" denotes costimulatory domain; "SSD" denotes stimulatory signaling domain. In the PG CAR construct in the figure, the extracellular domain comprises an antigen-binding portion capable of specifically binding to a mutated Fc domain comprising a P329G mutation, and the antigen-binding portion comprises a heavy chain variable region (VH) and a light chain variable region (VL).

Figure 2A shows a schematic diagram of a method for determining antibody affinity using surface plasmon resonance (SPR).
Figure 2B shows the representative affinity profiles of ADI-38497 PG antibody with a recombinant BCMA from human, cynomolgus monkey, mouse, rat and rabbit, as determined by SPR.
Figure 2C shows the binding ability of P329G BCMA antibody to CHO-GS cells stably expressing human, cynomolgus monkey and mouse BCMA.
Figure 2D shows the binding activity of P329G BCMA antibody to positive BCMA-expressing multiple myeloma cell lines MM.1s, RPMI8226, U266, H929, L363 and AMO1.
Figure 3A shows a schematic diagram of the detection of the affinity of the single chain antibody-rabbit Fc fusion protein specific for P329G mutation to ADI-38497 P329G mutated antibody using surface plasmon resonance (SPR) assay.
Figure 3B shows representative affinity profiles of ADI-38497 PG antibody and wild-type antibody binding to anti-PG scFv fusion protein, as determined by SPR assay.
Figure 3C shows a schematic representation of the method for detecting antibody avidity using surface plasmon resonance (SPR) assay.
Figure 3D shows representative avidity profiles of ADI-38497 PG antibody and wild-type antibody binding to anti-PG scFv fusion protein (P329G CAR extracellular domain comprises anti-PG scFv), as determined by SPR assay.
Figure 3E shows the binding ability of ADI-38497 WT antibody and ADI-38497 PG antibody to P329G CAR-T cells.
Figure 4A shows the ability of ADI-38497 WT antibody and ADI-38497 PG antibody to mediate ADCC killing.
Figure 4B shows the ability of ADI-38497 WT antibody and ADI-38497 PG antibody to mediate ADCP killing.
Figure 4C shows the ability of ADI-38497 PG antibody to mediate target cell lysis.
Figure 5A shows that against H929 cells, ADI-38497 PG antibody comprising P329G mutation specifically mediates the activation of CAR⁺-T.
Figure 5B shows the activation effect of different BCMA antibodies (ADI-38497 PG antibody, ADI-38484 PG antibody, ADI-38497 WT antibody, GSK PG IgG) on HuR968B CAR-T cells against L363 target cells.
Figure 5C shows the proliferation of HuR968B CAR-T cells after stimulation by the coated ADI-38497 WT antibody or ADI-38497 PG antibody.
Figure 5D shows the release results of the effector cytokine secretion from HuR968B CAR-T cells co-cultured with H929 cells and RPMI8226 cells after the addition of different concentrations of BCMA antibodies (ADI-38497 PG antibody, ADI-38484 PG antibody, ADI-38497 WT antibody, or GSK PG IgG as a positive control).
Figure 5E shows the release results of the effector cytokine secretion from HuR968B CAR-T cells co-cultured with different tumor cells after the addition of different concentrations of BCMA antibodies (ADI-38497 PG antibody, ADI-38484 PG antibody, ADI-38497 WT antibody, or GSK PG IgG).
Figure 5F shows the killing effects of HuR968B CAR-T cells induced by different BCMA antibodies (ADI-38497 PG antibody, ADI-38484 PG antibody, ADI-38497 WT antibody, or GSK PG IgG) on tumor cells having different BCMA expression levels (H929⁺⁺ cells, RPMI8226⁺⁺⁺ cells, AMO1⁺ cells, and L363⁺ cells).
Figure 5G shows the killing effect of CAR-T cells induced by P329G BCMA antibody on tumor cells.
Figure 6 shows the effect of different concentrations of free BCMA protein on the killing effect of HuR968B CAR-T and Blue21 CAR-T cells.
Figure 7A and Figure 7B show the pharmacokinetic experiment results of ADI-38497 PG antibody in mice.
Figure 8A shows the therapeutic effects of different doses of PG antibody combined with PG CAR-T cells on immunodeficient tumor bearing mice inoculated subcutaneously with human H929 tumor cells having a higher BCMA expression. In the figure, "cCAR-T" indicates conventional CAR-T, i.e., Blue21 CAR-T.
Figure 8B shows the changes in body weight of mice treated with different doses of PG antibody combined with PG CAR-T cells in immunodeficient tumor bearing mice inoculated subcutaneously with human H929 tumor cells having a higher BCMA expression.
Figure 8C shows the expansion of PG CAR-T cells in mice treated with different doses of PG antibody combined with PG CAR-T cells in immunodeficient tumor bearing mice inoculated subcutaneously with human H929 tumor cells having a higher BCMA expression.
Figure 9A shows the therapeutic effect of different doses of PG antibody combined with PG CAR-T cells on immunodeficient tumor bearing mice inoculated subcutaneously with human L363 tumor cells having a lower BCMA expression. In the figure, "cCAR-T" indicates conventional CAR-T, i.e., Blue21 CAR-T.
Figure 9B shows the changes in body weight of mice treated with different doses of PG antibody combined with PG CAR-T cells in immunodeficient tumor bearing mice inoculated subcutaneously with human L363 tumor cells having a lower BCMA expression.
Figure 9C shows the expansion of PG CAR-T cells in mice treated with different doses of PG antibody combined with PG CAR-T cells in immunodeficient tumor bearing mice inoculated subcutaneously with human L363 tumor cells having a lower BCMA expression.
Figure 10A shows the therapeutic effect of PG antibody combined with different doses of PG CAR-T cells on immunodeficient tumor bearing mice inoculated subcutaneously with human H929 tumor cells.
Figure 10B shows the expansion of PG CAR-T cells in mice treated with PG antibody combined with different doses of PG CAR-T cells in immunodeficient tumor bearing mice inoculated subcutaneously with human H929 tumor cells.
Figure 11A shows the frequency of PG antibody administration in the experiment of Example 11-1.
Figure 11B shows the therapeutic effect of different frequencies of PG antibody administration when combined with PG CAR-T cells in immunodeficient tumor bearing mice inoculated subcutaneously with human H929 tumor cells.
Figure 11C shows the expansion of PG CAR-T cells in mice in the experiments of Example 11-1.
Figure 12A shows the fluorescence images of the therapeutic effects of different doses of PG antibody combined with PG CAR-T cells on immunodeficient tumor bearing mice inoculated with human H929-luc tumor cells via the tail vein.
Figure 12B shows the therapeutic effects of different doses of PG antibody combined with PG CAR-T cells on immunodeficient tumor bearing mice inoculated with human H929-luc tumor cells via the tail vein.
Figure 12C shows the changes in body weight of mice treated with different doses of PG antibody combined with PG CAR-T cells in immunodeficient tumor bearing mice inoculated with human H929-luc tumor cells via the tail vein.
Figure 13A shows the therapeutic effect of PG antibody combined with PG CAR-T cells on immunodeficient tumor-bearing mice inoculated subcutaneously with human H929 tumor cells.
Figure 13B shows the changes in body weight of mice treated with PG antibody combined with PG CAR-T cells in immunodeficient tumor-bearing mice inoculated subcutaneously with human H929 tumor cells.
Figure 13C and Figure 13D show the results of hematological and blood biochemical assays in mice treated with PG antibody combined with PG CAR-T cells in immunodeficient tumor-bearing mice inoculated subcutaneously with human H929 tumor cells.

### DETAILED DESCRIPTION

Unless otherwise defined, all technical and scientific terms used herein have the same meanings as those commonly understood by those skilled in the art to which the invention belongs. All publications, patent applications, patents and other references referred to herein are incorporated by reference in their entirety. Furthermore, the materials, methods and examples described herein are illustrative only and are not intended to be restrictive. Other features, objects and advantages of the present invention will be apparent from the description and drawings and from the appended claims.

### I. Definitions

For the purpose of interpretation of the present description, the following definitions will be used, and where appropriate, terms used in the singular may also include the plural, and vice versa. It is to be understood that the terms used herein are only intended to describe specific embodiments, and are not intended to be restrictive.

The term "about", when used in conjunction with a numerical value, means a numerical value that covers a range having a lower limit of 5% less than the specified numerical value and an upper limit of 5% greater than the specified numerical value.

As used herein, the terms "and/or" mean any one of the options or two or more of the options.

In the context, when the terms "comprise" or "comprising" are used, unless otherwise specified, the situation consisting of the elements, integers, or steps described is also covered. For example, when referring to a antibody variable region "comprising" a specific sequence, it is also intended to cover the antibody variable region consisting of the specific sequence.

The terms "BCMA" and "B cell maturation antigen" are used interchangeably, and include variants, isotypes, species homologues of human BCMA, and analogues having at least one identical epitope with BCMA (e.g., human BCMA). BCMA proteins may also include fragments of BCMA, such as extracellular domains and extracellular domain fragments, such as fragments that retain the ability to bind to any antibody of the invention.

As used herein, the terms "BCMA antibody", "antibody against BCMA", "antibody specifically binding to BCMA", "antibody specifically targeting to BCMA", and "antibody specifically recognizing BCMA" can be interchangeably used, meaning antibodies that can specifically bind to B cell maturation antigen (BCMA).

The term "antibody" is used herein in the broadest sense, refers to a protein comprising an antigen-binding site, and encompasses natural and artificial antibodies with various structures, including, but not limited to, monoclonal antibodies, polyclonal antibodies, multispecific antibodies (e.g., bispecific antibodies), single-chain antibodies, intact antibodies, and antibody fragments. Preferably, the antibody of the present invention is a single-domain antibody or a heavy-chain antibody.

"Antibody fragment" and "antigen-binding fragment" are used interchangeably herein and refer to a molecule different from an intact antibody, which comprises a portion of the intact antibody and binds to an antigen to which the intact antibody binds. Examples of the antibody fragment include, but are not limited to, Fab', F(ab')₂, Fv, single-chain Fv, single-chain Fab, and diabodies.

The term "scFv" refers to a fusion protein comprising at least one antibody fragment containing a light chain variable region and at least one antibody fragment containing a heavy chain variable region, wherein the light chain variable region and the heavy chain variable region are continuously linked, optionally via a flexible short polypeptide linker, and are capable of being expressed as a single-chain polypeptide, with the scFv retaining the specificity of the intact antibody from which it is derived. Unless otherwise indicated, the scFv, as used herein, may have VL and VH variable regions in any order (e.g., relative to the N-terminus and C-terminus of the polypeptide), and may comprise VL-linker-VH or VH-linker-VL.

"Complementarity determining region" or "CDR" or "highly variable region" is a region in an antibody variable domain that is highly variable in sequence and forms a structurally defined loop ("hypervariable loop") and/or comprises antigen-contacting residues ("antigen-contacting sites"). CDRs are primarily responsible for binding to antigen epitopes. CDRs of heavy and light chains are generally referred to as CDR1, CDR2, and CDR3, and are numbered sequentially from the N-terminus. CDRs located in a heavy chain variable domain of an antibody are referred to as CDR H1, CDR H2, and CDR H3, whereas CDRs located in a light chain variable domain of the antibody are referred to as CDR L1, CDR L2, and CDR L3. In a given amino acid sequence of a light chain variable region or a heavy chain variable region, the exact amino acid sequence boundary of each CDR can be determined using any one or a combination of many well-known antibody CDR assignment systems including, e.g., Chothia based on the three-dimensional structure of antibodies and the topology of the CDR loops (Chothia et al. (1989) Nature 342: 877-883; Al-Lazikani et al., Standard conformations for the canonical structures of immunoglobulins, Journal of Molecular Biology, 273: 927-948 (1997)), Kabat based on antibody sequence variability (Kabat et al., Sequences of Proteins of Immunological Interest, 4th Ed., U.S. Department of Health and Human Services, National Institutes of Health (1987)), AbM (University of Bath), Contact (University College London), International ImMunoGeneTics database (IMGT) (imgt.cines.fr/ on the World Wide Web), and North CDR definition based on the affinity propagation clustering using a large number of crystal structures.

Unless otherwise stated, the term "CDR" or "CDR sequence" used herein encompasses CDR sequences determined by any one of the schemes described above.

CDRs can also be determined based on having the same Kabat numbering positions as a reference CDR sequence (e.g., any of the exemplary CDRs of the present invention). In the present invention, the numbering positions of antibody variable regions and specific CDR sequences (including heavy chain variable region residues) are based on the Kabat numbering system.

Although CDRs vary from antibody to antibody, only a limited number of amino acid positions within the CDRs are directly involved in antigen binding. The smallest overlapping region can be determined using at least two of the Kabat, Chothia, AbM, and Contact schemes, thereby providing a "minimal binding unit" for antigen binding. The minimal binding unit may be a sub-portion of the CDR. As will be clear to those skilled in the art, residues in the remaining portions of the CDR sequences can be determined by the structure and protein folding of the antibody. Therefore, variants of any CDR presented herein are also considered. For example, in a variant of one CDR, the amino acid residue of the minimal binding unit may remain unchanged, while the remaining CDR residues defined according to Kabat or Chothia or AbM may be substituted with conservative amino acid residues.

The term "chimeric antibody" is an antibody molecule in which: (a) a constant region or a portion thereof is modified, substituted, or exchanged such that antigen-binding sites are linked to constant regions of different or modified classes and/or species, or disparate molecules imparting new properties (e.g., enzymes, toxins, hormones, growth factors, and drugs) to chimeric antibodies, etc.; or (b) a variable region or a portion thereof is modified, replaced, or exchanged by variable regions with different or modified antigen-binding specificities. For example, a murine antibody may be modified by substituting its constant region with a constant region from a human immunoglobulin. Due to the substitution with a human constant region, the chimeric antibody may retain its specificity for recognizing antigens, while having reduced antigenicity in humans as compared to the original murine antibody.

"Humanized" antibody refers to a chimeric antibody comprising amino acid residues from non-human CDRs and amino acid residues from human FRs. In some embodiments, in the humanized antibody, all or substantially all CDRs (e.g., CDRs) correspond to those of a non-human antibody, and all or substantially all FRs correspond to those of a human antibody. A humanized antibody may optionally comprise at least a portion of an antibody constant region derived from a human antibody. The "humanized form" of an antibody (such as a non-human antibody) refers to an antibody that has been humanized.

"Human antibody" refers to an antibody having an amino acid sequence which corresponds to the amino acid sequence of an antibody generated by a human or human cell or derived from a non-human source that utilizes human antibody libraries or other human antibody encoding sequences. This definition of a human antibody explicitly excludes humanized antibodies comprising non-human antigen-binding residues.

The term "Fc region" refers to the C-terminal region of an immunoglobulin heavy chain, including a natural sequence Fc region and a variant Fc region. Fc region of a human IgG heavy chain is typically defined as a region from the amino acid residue at position Cys226 or Pro230 to the carboxyl terminus, and lysine residue at C-terminal position 447 of the Fc region (according to the EU numbering system) may be present or absent. Thus, the complete antibody composition may comprise an antibody population in which K447 residue is absent, an antibody population in which no K447 residue is removed, or antibody populations mixing an antibody with K447 residue and an antibody without K447 residue.

In some embodiments, the Fc region of an immunoglobulin comprises two constant domains, namely CH2 and CH3, and in other embodiments, the Fc region of an immunoglobulin comprises three constant domains, namely CH2, CH3 and CH4.

The binding of IgG to Fcγ receptor or C1q depends on the residues located in the hinge region and the CH2 domain. Two regions in the CH2 domain are critical to the binding to FcyR and complement C1q, and have a unique sequence in IgG2 and IgG4. It has been shown that the replacements of residues at positions 233-236 in human IgG1 and IgG2 and of residues at positions 327, 330 and 331 in human IgG4 can substantially reduce ADCC and CDC activities (Armour et al., Eur. J. Immunol. 29(8), 1999, 2613-2624; Shields et al., J. Biol. Chem 276(9), 2001, 6591-6604).

The terms "functional Fc region", "Fc region having function" and other similar terms are interchangable, and refer to Fc region with the effector function of a wild type Fc region.

Similar terms such as "variant Fc region", "Fc mutant", "mutation carrying Fc region", "mutant Fc region", "Fc region variant", "Fc variant", "variant Fc" and "mutant Fc" can be used interchangeably, referring to an Fc region comprising at least one amino acid modification and is different from the natural sequence Fc region/wild type Fc region.

In some embodiments, the variant Fc region comprises an amino acid sequence that differs from the amino acid sequence of the natural sequence Fc region by one or more amino acid substitutions, deletions, or additions. In some embodiments, the variant Fc region has at least one amino acid substitution compared with the Fc region of wild-type IgG, and the at least one amino acid substitution is to replace the amino acid at position P329 according to the EU numbering with glycine (G).

"Fc receptor" or "FcR" means a molecule that binds to the Fc region of an antibody. In some embodiments, FcR is a natural human FcR. In some embodiments, FcR is a receptor that binds to IgG antibody, namely FcγR, including three receptors of FcγRI (CD64), FcγRII (CD32) and FcyRIII (CD16), as well as their allelic variants and alternative splicing forms. FcyRII receptors include FcyRIIA and FcyRIIB, and FcyRIII receptors include FcγRIIIA and FcγRIIIB_{∘}

The term "effector function" refers to those biological activities attributed to the Fc region of an immunoglobulin, which vary with immunoglobulin isotypes. Examples of immunoglobulin effector functions include Fc receptor binding, antibody dependent cell-mediated cytotoxicity (ADCC), antibody dependent cell phagocytosis (ADCP), cytokine secretion, immune complex mediated antigen presenting cell antigen-uptake, C1q binding, and complement dependent cytotoxicity (CDC), downregulation of cell surface receptors (such as B cell receptors), and B cell activation.

The term "antibody dependent cell-mediated cytotoxicity (ADCC)" is one of the main mechanisms by which certain cytotoxic effector cells (such as natural killer (NK) cells) mediate the killing of target cells and foreign host cells. In some embodiments, the chimeric antigen receptor of the present invention provides antibody dependent cytotoxicity of T lymphocytes, and enhances antibody dependent cytotoxicity of NK cells. The chimeric antigen receptor of the present invention induces activation, sustained proliferation of T cells expressing the chimeric antigen receptor via binding to an antibody (or other anti-tumor molecule comprising Fc region) that binds to tumor cells, and induces specific cytotoxicity of the T cells expressing the chimeric antigen receptor against target cancer cells.

The term "antibody dependent cell phagocytosis (ADCP)" refers to a cellular reaction, in which macrophages are induced to be activated by the binding of target-cell-binding-antibody to FcyRIIIa on the surface of macrophages, so as to internalize target cells then acidify and degrade in phagosomes. ADCP can also be mediated by FcyRIIa and FcyRI, but the proportion is relatively low.

The term "complement dependent cytotoxicity (CDC)" refers to the lysis of target cells in the presence of complements. The complement system is a part of the innate immune system, which is made up of a series of proteins. The proteins of the complement system are called "complements", which are represented by the abbreviation symbols C1, C2, C3, etc. They are a group of heat labile proteins that exist in human or vertebrate serum and tissue fluid and have enzymatic activity after activation. C1q is the first component of complement dependent cytotoxicity (CDC) pathway. It can bind six antibodies, but its binding with two IgGs is sufficient to activate the complement cascade. The activation of the classical complement pathway is initiated by the binding of the first component (C1q) of the complement system to an antibody (of a suitable subclass) that binds to a related antigen, then a series of complement cascade reactions are activated, forming holes in the target cell membrane, thus leading to target cell death. To evaluate complement activation, a CDC assay can be performed according to a method described in, for example, Gazzano-Santoro et al., J. Immunol. Methods 202:163 (1996).

The term "variable region" or "variable domain" refers to a domain of antibody heavy chain or light chain involved in the binding of antibody to antigen. The variable domains of the heavy and light chains of natural antibodies usually have similar structures, and each domain comprises four conservative framework regions (FR) and three complementarity determining regions (CDR). (See, for example, Kindt et al., Kuby Immunology, 6th ed., W. H. Freeman and Co., page 91 (2007)). A single VH or VL domain may be sufficient to provide antigen binding specificity.

As used herein, the terms "binding" or "specific binding" mean that the binding to antigen is selective and can be distinguished from unwanted or nonspecific interactions. The binding ability of an antibody to a specific antigen can be determined by enzyme-linked immunosorbent assay (ELISA), SPR or biolayer interferometry technology or other conventional binding assays known in the art.

The term "stimulation" refers to a primary response induced by binding of a stimulatory molecule (e.g., a TCR/CD3 complex) with its cognate ligand thereby mediating a signal transduction event, such as, but not limited to, signal transduction via the TCR/CD3 complex. Stimulation can mediate altered expression of certain molecules, such as downregulation of TGF-β, and/or reorganization of cytoskeletal structures, and the like.

The term "stimulatory molecule" refers to a molecule expressed by a T cell that provides the primary cytoplasmic signaling sequence(s) that regulate primary activation of the TCR complex in a stimulatory way for at least some aspect of the T cell signaling pathway. In an embodiment, the primary signal is initiated by, for instance, binding of a TCR/CD3 complex with an MHC molecule loaded with peptide, and which leads to mediation of a T cell response, including, but not limited to, proliferation, activation, differentiation, and the like. In the specific CARs of the present invention, the intracellular signal domain in any one or more of the CARs of the present invention comprises an intracellular signaling sequence, e.g., a primary signaling sequence of CD3ζ.

The term "CD3ζ" is defined as the protein provided as GenBan Acc. No. BAG36664.1, or equivalent thereof, and "CD3ζ stimulatory signaling domain" is defined as the amino acid residues from the cytoplasmic domain of the CD3ζ chain that are sufficient to functionally transmit an initial signal necessary for T cell activation. In one embodiment, the cytoplasmic domain of CD3ζ comprises residues 52 through 164 of GenBank Acc. No. BAG36664.1 or the equivalent residues from a non-human species (e.g., mouse, rodent, monkey, ape and the like) that are functional orthologs thereof. In one embodiment, the "CD3ζ stimulatory domain" is the sequence provided as SEQ ID NO: 17 or a variant thereof.

The term "co-stimulatory molecule" refers to the cognate binding partner on a cell that specifically binds with a co-stimulatory ligand, thereby mediating a co-stimulatory response by the cell, such as, but not limited to, proliferation. Co-stimulatory molecules are cell surface molecules other than antigen receptors or their ligands that are required for an efficient immune response. Co-stimulatory molecules include, but are not limited to an MHC class I molecule, TNF receptor proteins, Immunoglobulin-like proteins, cytokine receptors, integrins, signaling lymphocytic activation molecules (SLAM proteins), activating NK cell receptors, OX40, CD40, GITR, 4-1BB (i.e., CD137), CD27, and CD28. In some embodiments, the "co-stimulatory molecule" is 4-1BB (i.e., CD137). The co-stimulatory signaling domain refers to the intracellular portion of the co-stimulatory molecule.

The term "4-1BB" refers to a member of the TNFR superfamily with an amino acid sequence provided as GenBank Acc. No. AAA62478.2, or the equivalent residues from a non-human species, e.g., mouse, rodent, monkey, ape and the like; and a "4-1BB co-stimulatory domain" is defined as amino acid residues 214-255 of GenBank Acc. No. AAA62478.2, or the equivalent residues from a non-human species, e.g., mouse, rodent, monkey, ape and the like. In one embodiment, the "4-1BB co-stimulatory domain" is the sequence provided as SEQ ID NO: 16 or the equivalent residues from a non-human species (e.g., mouse, rodent, monkey, ape and the like).

The term "signaling pathway" refers to the biochemical relationship between multiple signaling molecules that play a role in propagating signals from one part of the cell to another part of the cell.

The term "cytokine" is a generic term for proteins that are released by a cell population and act as intercellular mediators on another cell. Examples of such cytokines are lymphokines; monokines; interleukin (ILs), such as IL-1, IL-1α, IL-2, IL-3, IL-4, IL-5, IL-6, IL-7, IL-8, IL-9, IL-11, IL-12, and IL-15; tumor necrosis factors such as TNF-α or TNF-β; and other polypeptide factors, including γ-interferon.

"Isolated" antibody is an antibody that has been separated from components of its natural environment. In some embodiments, the antibody of the present invention is purified to a purity greater than 95% or 99% as determined by, for example, electrophoresis (e.g., SDS-PAGE, isoelectric focusing (IEF), and capillary electrophoresis) or chromatography (e.g., ion exchange or reverse-phase HPLC). For a review of methods for assessing antibody purity, see, e.g., Flatman et al., J. Chromatogr., B848: 79-87 (2007).

"Isolated" nucleic acid refers to a nucleic acid molecule that has been separated from components of its natural environment. The isolated nucleic acid includes a nucleic acid molecule contained in a cell that typically comprises the nucleic acid molecule, but the nucleic acid molecule exists extrachromosomally or at a chromosomal location that is different from its natural chromosomal location. "Isolated nucleic acid encoding the antibody of the present invention" refers to one or more nucleic acid molecules encoding chains of the antibody of the present invention or fragments thereof, including such nucleic acid molecules in a single vector or separate vectors, and such nucleic acid molecules present at one or more positions in a host cell.

The calculation of sequence identity between sequences is performed as follows.

To determine the percent identity of two amino acid sequences or two nucleic acid sequences, the sequences are aligned for optimal comparison purposes (e.g., for optimal alignment, gaps can be introduced in one or both of the first and second amino acid sequences or nucleic acid sequences, or non-homologous sequences can be discarded for comparison). In one preferred embodiment, for comparison purposes, the length of the aligned reference sequence is at least 30%, preferably at least 40%, more preferably at least 50% or 60%, and even more preferably at least 70%, 80%, 90%, or 100% of the length of the reference sequence. Amino acid residues or nucleotides at corresponding amino acid positions or nucleotide positions are then compared. When a position in the first sequence is occupied by the same amino acid residue or nucleotide at the corresponding position in the second sequence, the molecules are identical at this position.

A mathematical algorithm can be used to compare two sequences and calculate percent identity between the sequences. In one preferred embodiment, the percent identity between two amino acid sequences is determined with the Needleman and Wunsch algorithm ((1970) J. Mol. Biol., 48: 444-453; available at http://www.gcg.com) that has been integrated into the GAP program of the GCG software package, using the Blossum 62 matrix or PAM250 matrix and a gap weight of 16, 14, 12, 10, 8, 6, or 4 and a length weight of 1, 2, 3, 4, 5, or 6. In another preferred embodiment, the percent identity between two nucleotide sequences is determined with the GAP program of the GCG software package (available at http://www.gcg.com), using the NWSgapdna.CMP matrix and a gap weight of 40, 50, 60, 70, or 80 and a length weight of 1, 2, 3, 4, 5, or 6. A particularly preferred parameter set (and one that should be used unless otherwise stated) is a Blossum 62 scoring matrix with a gap penalty of 12, a gap extension penalty of 4, and a frameshift gap penalty of 5.

The percent identity between two amino acid sequences or nucleotide sequences can also be determined with a PAM120 weighted remainder table, a gap length penalty of 12, and a gap penalty of 4, using the E. Meyers and W. Miller algorithm ((1989) CABIOS, 4: 11-17) that has been incorporated into the ALIGN program (version 2.0).

Additionally or alternatively, the nucleic acid sequences and protein sequences described herein can be further used as "query sequences" to perform searches against public databases to, e.g., identify other family member sequences or related sequences.

The terms "amino acid change" and "amino acid modification" are used interchangeably and refer to addition, deletion, substitution, and other modifications of amino acids. Any combination of additions, deletions, substitutions and other modifications of amino acids may be made, provided that the final polypeptide sequence possesses the desired properties. In some embodiments, the amino acid substitution in the antibody results in reduced binding of the antibody to the Fc receptor. For the purpose of changing, for example, the binding characteristics of the Fc region, non-conservative amino acid substitutions are particularly preferred, where one amino acid is substituted with another amino acid having different structural and/or chemical properties. Amino acid substitutions include substitutions with non-naturally occurring amino acids or naturally occurring amino acid derivatives of the twenty standard amino acids (e.g., 4-hydroxyproline, 3-methylhistidine, ornithine, homoserine, and 5-hydroxylysine). Amino acid changes can be made using genetic or chemical methods well known in the art. Genetic methods may include site-directed mutagenesis, PCR, gene synthesis, etc. Methods for altering side chain groups of amino acids by means other than genetic engineering, such as chemical modification, may be useful. Multiple names may be used herein to denote the same amino acid change. For example, a substitution from proline to glycine at position 329 of the Fc domain may be denoted as 329G, G329, G₃₂₉, P329G Pro329Gly, or simply "PG".

The terms "conservative sequence modification" and "conservative sequence change" refer to amino acid modifications or changes that do not significantly affect or change the binding characteristics of an antibody or an antibody fragment comprising the amino acid sequence. Such conservative modifications include amino acid substitutions, additions, and deletions. Modifications can be introduced into the antibody or the antibody fragment of the present invention by standard techniques known in the art, such as site-directed mutagenesis and PCR-mediated mutagenesis. Conservative substitutions are amino acid substitutions in which an amino acid residue is replaced with an amino acid residue having a similar side chain. Families of the amino acid residues having similar side chains have been defined in the art. These families include amino acids having basic side chains (e.g., lysine, arginine, and histidine), amino acids having acidic side chains (e.g., aspartic acid and glutamic acid), amino acids having uncharged polar side chains (e.g., glycine, asparagine, glutamine, serine, threonine, tyrosine, cysteine, and tryptophan), amino acids having non-polar side chains (e.g., alanine, valine, leucine, isoleucine, proline, phenylalanine, and methionine), amino acids having β-side chains (e.g., threonine, valine, and isoleucine), and amino acids having aromatic side chains (e.g., tyrosine, phenylalanine, tryptophan, and histidine). Thus, one or more amino acid residues within the CARs of the present invention may be replaced with other amino acid residues from the same side chain family, and the altered CARs may be tested using the functional assays described herein.

The term "autologous" refers to any substance that is derived from the same individual into whom the substance is later to be re-introduced.

The term "allogeneic" refers to any substance that is derived from a different animal of the same species as the individual into whom the substance is to be introduced. Two or more individuals are said to be allogeneic to one another when the genes at one or more loci are not identical. In some aspects, allogeneic substances from individuals of the same species may be genetically dissimilar enough to undergo antigenic interactions.

The term "xenogeneic" refers to a graft derived from an animal of a different species.

The term "apheresis" as used herein refers to the art-recognized extracorporeal method by which the blood of a donor or patient is removed from the donor or patient and passed through an apparatus that separates out selected particular components and returns the remainder to the circulation of the donor or patient, e.g., by retransfusion. Therefore, in the context of "an apheresis sample", it refers to a sample obtained using apheresis.

The term "immune effector cell" refers to a cell that is involved in an immune response, e.g., in the promotion of an immune effector response. Examples of immune effector cells include T cells, e.g., α/β T cells and γ/δ T cells, B cells, natural killer (NK) cells, natural killer T (NKT) cells, mast cells, and myeloid cell-derived phagocytes.

"Immune effector function" or "immune effector response" refers to, for example, a function or response of an immune effector cell that enhances or promotes an immune attack on a target cell. For example, the immune effector function or response refers to a property of a T cell or NK cell that promotes the killing of a target cell or inhibits the growth or proliferation of a target cell. In the case of T cells, primary stimulation and co-stimulation are examples of the immune effector function or response.

The term "effector function" refers to a specialized function of a cell. The effector function of a T cell may be, for example, cytolytic activity or helper activity, including secretion of cytokines.

The term "T cell activation" refers to one or more cellular responses of a T lymphocyte, in particular a cytotoxic T lymphocyte, selected from: proliferation, differentiation, cytokine secretion, release of cytotoxic effector molecules, cytotoxic activity, and expression of activation markers. The chimeric antigen receptor of the present invention is capable of inducing T cell activation. Suitable assays for measuring T cell activation are described in the examples and are known in the art.

The term "lentivirus" refers to a genus of the Retroviridae family. Lentiviruses are unique among retroviruses in their ability to infect non-dividing cells; they can deliver a significant amount of genetic information to a host cell, making them one of the most efficient methods of gene delivery vectors. HIV, SIV, and FIV are all examples of lentiviruses.

The term "lentiviral vector" refers to a vector derived from at least a portion of the lentiviral genome, in particular including a self-inactivating lentiviral vector as provided in Milone et al., Mol. Ther. 17(8): 1453-1464 (2009). Other examples of lentiviral vectors that can be used clinically include, for example, but are not limited to, the LENTIVECTOR^{®} gene delivery technology from Oxford BioMedica, the LENTIMAX^{™} vector system from Lentigen, etc. Non-clinical types of lentiviral vectors are also available and known to those skilled in the art.

The term "BCMA-related disease" refers to any disease induced, aggravated or otherwise associated with increased expression or activity of BCMA.

The terms "individual" or "subject" are used interchangeably, including mammals. Mammals include, but are not limited to, domesticated animals (e.g., cattle, sheep, cats, dogs, and horses), primates (e.g., humans and non-human primates such as monkeys), rabbits, and rodents (e.g., mice and rats). In particular, the individual or subject is a human.

The terms "tumor" and "cancer" are used interchangeably herein, including solid tumors and liquid tumors.

The terms "cancer" and "carcinoma" refer to a physiological disease in mammals in which cell growth is unregulated.

The term "tumor" refers to all neoplastic cell growth and proliferation, whether malignant or benign, and all pre-cancerous and cancerous cells and tissues. The terms "cancer", "carcinoma", and "tumor" are not mutually exclusive when referred to herein.

"Tumor immune escape" refers to a process by which tumors evade immune recognition and clearance. As such, as a therapeutic concept, tumor immunity is "treated" when such evasion diminishes, and the tumor is recognized and attacked by the immune system. Examples of tumor recognition include tumor binding, tumor shrinkage, and tumor clearance.

The term "half maximal effective concentration (EC₅₀)" refers to the concentration of a drug, antibody or toxin that induces a response of 50% between the baseline and the maximum after a particular exposure time.

The term "fluorescence activated cell sorting" or "FACS" refers to a specialized type of flow cytometry. It provides a method for sorting a heterogeneous mixture of biological cells, one cell at a time, into two or more containers based on the specific light scattering and fluorescence characteristics of each cell (FlowMetric. "Sorting Out Fluorescence Activated Cell Sorting". 2017-11-09). Instruments for performing FACS are known to those skilled in the art and are commercially available to the public. Examples of such instruments include FACS Star Plus, FACScan and FACSort instruments from Becton Dickinson (Foster City, CA), Epics C from Coulter Epics Division (Hialeah, FL), and MoFlo from Cytomation (Colorado Springs, Colorado).

The term "pharmaceutically acceptable auxiliary materials" refers to diluents, adjuvants (e.g., (complete and incomplete) Freund's adjuvants), excipients, buffers or stabilizers, etc., that are administered with the active substance.

As used herein, "treatment", "treat" or "treating" refers to slowing, interrupting, arresting, alleviating, stopping, lowering, or reversing the progression or severity of an existing symptom, condition, disorder, or disease. Desired therapeutic effects include, but are not limited to, preventing the occurrence or recurrence of diseases, alleviating symptoms, reducing any direct or indirect pathological outcomes of diseases, preventing metastasis, delaying disease progression, improving or alleviating conditions, and alleviating or improving prognosis. In some embodiments, the antibody molecule of the present invention is used to delay the progression of a disease or to slow the progression of a disease.

The term "effective amount" refers to an amount or dosage of the antibody or composition of the present invention which generates expected effects in a patient in need of treatment or prevention after administration to the patient in a single dose or multiple doses. The effective amount can be easily determined by an attending physician as a person skilled in the art by considering a variety of factors as follows: species such as mammals; weight, age, and general health condition; the specific disease involved; the extent or severity of the disease; response in an individual patient; specific antibody administered; mode of administration; bioavailability characteristics of the administered formulation; selected administration regimen; and use of any concomitant therapy.

"Therapeutically effective amount" refers to an amount effective to achieve a desired therapeutic result at a necessary dose for a necessary period of time. The therapeutically effective amount of an antibody or antibody fragment or a composition thereof may vary depending on a variety of factors such as disease state, age, sex, and weight of an individual, and the ability of the antibody or antibody portion to elicit a desired response in the individual. The therapeutically effective amount is also such an amount that any toxic or undesired effect of the antibody or antibody fragment or the composition thereof is inferior to the therapeutically beneficial effect. "Therapeutically effective amount" preferably inhibits a measurable parameter (e.g., tumor growth rate, tumor volume, etc.) by at least about 20%, more preferably at least about 40%, even more preferably at least about 50%, 60%, or 70%, and still more preferably at least about 80% or 90%, relative to an untreated subject. The ability of a compound to inhibit a measurable parameter (e.g., cancer) can be evaluated in an animal model system that predicts efficacy in human tumors.

The term "pharmaceutical combination" refers to a non-fixed combination product or a fixed combination product, including but not limited to a kit and a pharmaceutical composition. The term "non-fixed combination" means that the active ingredients (e.g., (i) P329G CAR-T cells, and (ii) a P329G mutated antibody directed against BCMA) are administered to a subject either simultaneously or sequentially (without particular time limitation, or at identical or different time intervals) as separate entities, wherein such administration provides effective treatment in the subject. The term "fixed combination" refers to a combination in which the P329G mutated antibody directed against BCMA and the P329G CAR-T cells of the present invention are administered to a patient simultaneously in the form of a specific single dose. The term "non-fixed combination" refers to a combination in which the 329G mutated antibody directed against BCMA and the P329G CAR-T cells of the present invention are simultaneously, concurrently, or sequentially administered to a patient as separate entities, without specific dosage and time limitation, wherein such administration provides a therapeutically effective level of the pharmaceutical combination of the present invention in the patient. In one preferred embodiment, the pharmaceutical combination is a non-fixed combination.

The term "combination therapy" or "combined therapy" means that two or more components are administered to treat a cancer as described herein. Such administration includes co-administration of these components in a substantially simultaneous manner. Alternatively, such administration includes co-administration or separate administration or sequential administration of the active ingredients in a variety of or separate containers (e.g., capsules, powder, and liquid). The powder and/or liquid can be reconstituted or diluted to a desired dose before administration. In some embodiments, the administration further includes using the P329G mutated antibody directed against BCMA and the P329G CAR-T cells of the present invention at approximately the same time or in a sequential manner at different times. In any case, the therapeutic regimen will provide the beneficial effect of the pharmaceutical combination in the treatment of conditions or symptoms described herein.

The term "vector" as used herein when referring to a nucleic acid refers to a nucleic acid molecule capable of proliferating another nucleic acid to which it is linked. The term includes vectors that serve as self-replicating nucleic acid structures as well as vectors binding to the genome of a host cell into which they have been introduced. Some vectors are capable of directing the expression of nucleic acids to which they are operatively linked. Such vectors are referred to as "expression vectors" herein.

The term "host cell" refers to a cell into which an exogenous polynucleotide has been introduced, including progeny of such cells. Host cells include "transformants" and "transformed cells", which include primary transformed cells and progeny derived therefrom, regardless of the number of passages. Progeny may not be exactly the same as parent cells in terms of nucleic acid content, and may comprise mutations. Mutant progenies having the same function or biological activities that are screened or selected from the initially transformed cells are included herein. Host cells are any type of cell system that can be used to produce the antibody molecule of the present invention, including eukaryotic cells, e.g., mammalian cells, insect cells, and yeast cells; and prokaryotic cells, e.g., *E. coli* cells. Host cells include cultured cells, as well as cells within a transgenic animal, a transgenic plant, or cultured plant tissue or animal tissue.

"Subject/patient sample" refers to a collection of cells, tissues, or body fluids obtained from a patient or a subject. The source of tissue or cell samples can be solid tissues, e.g., from fresh, frozen and/or preserved organ or tissue samples or biopsy samples or puncture samples; blood or any blood component; body fluids such as cerebrospinal fluids, amniotic fluids, peritoneal fluids, or interstitial fluids; and cells from a subject at any time during pregnancy or development. Tissue samples may comprise compounds that are naturally not mixed with tissues, such as preservatives, anticoagulants, buffers, fixatives, nutrients, and antibiotics. Examples of tumor samples include but are not limited to tumor biopsies, fine needle aspirates, bronchial lavage fluids, pleural fluids, sputa, urine, surgical specimens, circulating tumor cells, serum, plasma, circulating plasma proteins, ascites, primary cell cultures or cell lines derived from tumors or exhibiting tumor-like properties, and preserved tumor samples such as formalin-fixed, paraffin-embedded tumor samples or frozen tumors samples.

When referring to a disease, the term "treatment", "treat" or "treating" refers to alleviating the disease (i.e., slowing or arresting or reducing the development of the disease or at least one clinical symptom thereof), preventing or delaying the onset or development or progression of the disease.

### II. Molecular switch-regulated chimeric antigen receptor (CAR) of the present invention

The present invention relates to a chimeric antigen receptor polypeptide capable of specifically binding to a mutated Fc domain of an antibody directed against a BCMA molecule. Specifically, the chimeric antigen receptor of the present invention comprises a humanized anti-P329G mutation scFv sequence, and the scFv sequence is capable of specifically binding to the Fc domain of an antibody comprising the P329G mutation, but not to a parent antibody Fc domain without the mutation. The Fc domain of the antibody comprising the P329G mutation has a decreased binding to Fc receptor (e.g., Fcy receptor), compared with the binding of the Fc domain of a parent antibody without the mutation to Fc receptor.

The recombinant CAR construct of the present invention comprises a sequence encoding a CAR, wherein the CAR comprises a humanized anti-P329G mutation scFv sequence, and the scFv sequence specifically binding to Fc domain of a P329G mutated antibody.

In one embodiment, the scFv sequence in the CAR construct of the present invention comprises the sequence of:
(i) a heavy chain variable region, which comprises according to the Kabat numbering
   (a) a heavy chain complementary determinant region CDR H1 shown in amino acid sequence of RYWMN(SEQ ID NO: 19), or a variant of the CDR H1 with no more than 2 amino acid changes or no more than 1 amino acid change;
   (b) a CDR H2 shown in amino acid sequence of EITPDSSTINYAPSLKG(SEQ ID NO: 20); or a variant of the CDR H2 with no more than 2 amino acid changes or no more than 1 amino acid change; and
   (c) a CDR H3 shown in amino acid sequence of PYDYGAWFAS(SEQ ID NO: 21); or a variant of the CDR H3 with no more than 2 amino acid changes or no more than 1 amino acid change; and
(ii) a light chain variable region, which comprises according to the Kabat numbering
   (d) a light chain complementary determinant region (CDR L)1 shown in amino acid sequence of RSSTGAVTTSNYAN(SEQ ID NO: 22); or a variant of the CDR L1 with no more than 2 amino acid changes or no more than 1 amino acid change;
   (e) a CDR L2 shown in amino acid sequence of GTNKRAP(SEQ ID NO: 23); or a variant of the CDR L2 with no more than 2 amino acid changes or no more than 1 amino acid change; and
   (f) a CDR L3 shown in amino acid sequence of ALWYSNHWV(SEQ ID NO: 24); or a variant of the CDR L3 with no more than 2 amino acid changes or no more than 1 amino acid change; wherein the change of amino acid is an addition, deletion or substitution of an amino acid.

Further, the scFv may have a signal peptide sequence, e.g., the signal peptide sequence shown in SEQ ID NO: 11, attached at the N-terminus, and the scFv may have an optional hinge region/spacer region sequence as shown in SEQ ID NO: 14 or SEQ ID NO: 18, a transmembrane region as shown in SEQ ID NO: 15, a co-stimulatory signaling domain as shown in SEQ ID NO: 16, and an intracellular stimulatory signaling domain comprising SEQ ID NO: 17 or a variant thereof, attached at the C-terminus, e.g., wherein the domains are contiguous with and in the same reading frame to form a single fusion protein.

In some embodiments, the scFv domain comprises (i) a heavy chain variable region, which comprises a sequence shown in SEQ ID NO: 12, or a sequence having at least 90%, 91%, 92%, 93%, 94%, 95%, 96%, 97%, 98% or 99% identity thereto, and (ii) a light chain variable region, which comprises a sequence shown in SEQ ID NO: 13, or a sequence having at least 90%, 91%, 92%, 93%, 94%, 95%, 96%, 97%, 98% or 99% identity thereto.

In some embodiments, the scFv domain comprises (i) a heavy chain variable region shown in SEQ ID NO: 12 and (ii) a light chain variable region shown in SEQ ID NO: 13. In one embodiment, the scFv domain further comprises a (Gly4-Ser)n linker, wherein n is 1, 2, 3, 4, 5 or 6, preferably 3 or 4. The light chain variable region and heavy chain variable region of a scFv can be, e.g., in any of the following orientations: light chain variable region-linker-heavy chain variable region or heavy chain variable region-linker-light chain variable region.

In some embodiments, exemplary CAR constructs of the present invention comprise a signal peptide sequence, a humanized anti-P329G mutation scFv sequence, a hinge region/spacer region, a transmembrane domain, an intracellular co-stimulatory signaling domain, and an intracellular stimulatory signaling domain.

In one embodiment, the invention provides an amino acid sequence of a full-length CAR polypeptide as SEQ ID NO: 1, as shown in the sequence listing.

In some embodiments, the present invention provides a recombinant nucleic acid construct comprising a nucleic acid molecule encoding a CAR of the present invention, for example, comprising a nucleic acid molecule encoding an amino acid sequence shown in SEQ ID NO: 1 or an amino acid sequence having at least 90%, 91%, 92%, 93%, 94%, 95%, 96%, 97%, 98%, or 99% identity to SEQ ID NO: 1. The construct encoding the CAR of the present invention can be obtained by using recombinant methods well known in the art. Alternatively, Alternatively, the nucleic acid of interest can be produced synthetically, rather than by genetic recombination methods.

The present invention includes a retroviral vector construct and a lentiviral vector construct that can be directly transduced into cells and express CAR.

In some embodiments, the nucleic acid sequence of the CAR construct of the present invention is cloned into a lentiviral vector to create a full-length CAR construct in a single coding frame and uses the EF1α promoter for expression.

It will be appreciated by those of ordinary skill in the art that the CAR polypeptides of the present invention may further be modified such that they vary in amino acid sequence, but not in the desired activity. For example, the CAR polypeptide may be subjected to additional nucleotide substitutions that result in amino acid substitutions at "non-essential" amino acid residues. For example, a non-essential amino acid residue in a molecule may be replaced with another amino acid residue from the same side chain family. In another embodiment, a string of amino acids can be replaced with a structurally similar string that differs in order and composition of side chain family members, e.g., a conservative substitution, in which an amino acid residue is replaced with an amino acid residue having a similar side chain, may be made.

Families of amino acid residues having similar side chains have been defined in the art, including basic side chains (e.g., lysine, arginine, histidine), acidic side chains (e.g., aspartic acid, glutamic acid), uncharged polar side chains (e.g., glycine, asparagine, glutamine, serine, threonine, tyrosine, cysteine), nonpolar side chains (e.g., alanine, valine, leucine, isoleucine, proline, phenylalanine, methionine, tryptophan), β-branched side chains (e.g., threonine, valine, isoleucine), and aromatic side chains (e.g., tyrosine, phenylalanine, tryptophan, histidine).

In some embodiments, the present invention contemplates generating functionally equivalent CAR polypeptide molecules, for example, VH or VL of the humanized anti-P329G mutation scFv sequence comprised in the CAR may be modified to obtain a VH having at least 90%, 91%, 92%, 93%, 94%, 95%, 96%, 97%, 98% or 99% identity to SEQ ID NO: 12 and a VL having at least 90%, 91%, 92%, 93%, 94%, 95%, 96%, 97%, 98% or 99% identity to SEQ ID NO: 13.

The transmembrane domain comprised in the CAR of the present invention is an anchored transmembrane domain that is a component of the polypeptide chain and capable of being integrated in the cell membrane. The transmembrane domain may be fused to other extracellular and/or intracellular polypeptide domains, thereby these extracellular and/or intracellular polypeptide domains will also be confined to the cell membrane. In the chimeric antigen receptor (CAR) polypeptide of the present invention, the transmembrane domain confers membrane attachment to the CAR polypeptide of the present invention. The CAR polypeptide of the present invention comprises at least one transmembrane domain, which may be derived from a natural source or a recombinant source, comprising predominantly hydrophobic residues such as leucine and valine. In the case where the source is natural, the domain can be derived from the transmembrane domain of a membrane-bound protein or a transmembrane protein such as CD28, CD8 (e.g., CD8α, CD8β). In one embodiment, the transmembrane domain comprises the amino acid sequence of SEQ ID NO: 15.

In some embodiments, the transmembrane domain in the CAR of the present invention is connected to the extracellular region (i.e., the humanized anti-P329G mutation scFv sequence) of the CAR with the aid of a hinge region/spacer region. For example, in one embodiment, the hinge may be CD8α hinge region, CD28 hinge region. In some embodiments, the hinge or spacer region sequence comprises the amino acid sequence of SEQ ID NO: 18.

Further, the glycine-serine duplex also provides a particularly suitable linker as the hinge region/spacer region. For example, in one embodiment, the linker comprises an amino acid sequence of GGGGS (SEQ ID NO: 14).

The cytoplasmic domain comprised in the CAR of the present invention comprises an intracellular signaling domain. The intracellular signaling domain is capable of activating at least one effector function of the immune cell into which the CAR of the present invention has been introduced.

Examples of the intracellular signaling domain for use in the CAR of the present invention include cytoplasmic sequences of T cell receptors (TCRs) and co-receptors that function synergistically to initiate signal transduction upon binding of the Fc domain of the P329G mutated antibody to the extracellular domain, as well as any derivatives or variants of these sequences and any recombinant sequences with the same functional capacity.

Considering that signals generated through the TCR alone are not sufficient for full activation of the T cell, the CAR of the present invention is also designed to comprise a co-stimulatory signaling domain (CSD) capable of generating a co-stimulatory signal. T cell activation is mediated by two distinct classes of cytoplasmic signaling sequences: those that initiate antigen-dependent primary activation through the TCR (primary intracellular signaling domains) and those that act in an antigen-independent manner to provide a co-stimulatory signal (secondary cytoplasmic domain, e.g., a co-stimulatory domain).

In one embodiment, the CAR of the present invention comprises a primary intracellular signaling domain, e.g., a primary signaling domain of CD3ζ, e.g., the CD3ζ signaling domain shown in SEQ ID NO: 17.

The intracellular signaling domain in the CAR of the present invention also comprises a secondary signaling domain (i.e., a co-stimulatory signaling domain). The co-stimulatory signaling domain refers to the CAR portion comprising an intracellular domain of a co-stimulatory molecule. Co-stimulatory molecules are cell surface molecules other than antigen receptors or their ligands, which are required by lymphocytes for an effective response to an antigen. In some embodiments, the co-stimulatory molecules include, but are not limited to, CD28, 4-1BB (CD137), which induce co-stimulatory effects that enhance proliferation, effector function, and survival of human CART cells in vitro and enhance the antitumor activity of human T cells in vivo.

The intracellular signaling sequences in the CAR of the present invention may be linked to each other in a random order or in a specified order. Optionally, short oligopeptide linkers or polypeptide linkers may form bonds between the intracellular signaling sequences. In one embodiment, the glycine-serine duplex may be used as a suitable linker. In one embodiment, a single amino acid, e.g., alanine, glycine, may be used as a suitable linker.

In one embodiment, the intracellular signaling domain of the CAR of the present invention is designed to comprise the co-stimulatory signaling domain of 4-1BB and the stimulatory signaling domain of CD3ζ.

### III. Nucleic acid molecules encoding the CAR of the present invention, vectors and cells expressing the CAR of the present invention

The present invention provides nucleic acid molecules encoding the CAR constructs described herein. In one embodiment, the nucleic acid molecules are provided as DNA constructs.

The present invention also provides vectors inserted with a CAR construct of the present invention. Expression of a natural or synthetic nucleic acid encoding CAR is achieved by operatively linking a nucleic acid encoding the CAR polypeptide to a promoter and incorporating the construct into an expression vector. The vectors can be suitable for replication and integration in eukaryotes. Typical cloning vectors contain transcription and translation terminators, initiation sequences, and promoters useful for regulation of the expression of the desired nucleic acid sequence.

Numerous viral based systems have been developed for gene transfer into mammalian cells. For example, retroviruses provide a convenient platform for gene delivery systems. A selected gene can be inserted into a vector and packaged in retroviral particles using techniques known in the art. The recombinant virus can then be isolated and delivered to cells of the subject either in vivo or ex vivo. Numerous retroviral systems are known in the art. In some embodiments, lentivirus vectors are used.

Vectors derived from retroviruses (e.g., lentiviruses) are suitable tools for achieving long-term gene transfer since they allow long-term, stable integration of a transgene and its propagation in progeny cells. Lentiviral vectors have the additional advantage over vectors derived from onco-retroviruses (e.g., murine leukemia virus) in that they can transduce non-proliferating cells, such as hepatocytes. They also have the added advantage of low immunogenicity. A retroviral vector may also be, e.g., a gamma-retroviral vector. A gamma-retroviral vector may include, e.g., a promoter, a packaging signal (ψ), a primer binding site (PBS), one or more (e.g., two) long terminal repeats (LTR), and a transgene of interest, e.g., a gene encoding a CAR. A gamma-retroviral vector may lack viral structural gens such as gag, pol, and env.

An example of a promoter capable of expressing a CAR transgene in mammalian T cells is EF1α promoter. The natural EF1α promoter drives the expression of the alpha subunit of the elongation factor-1 complex, wherein the alpha subunit is responsible for the enzymatic delivery of the aminoacyl tRNA to the ribosome. The EF1α promoter has been used extensively in mammalian expression plasmids and has been shown to be effective in driving CAR expression from transgenes cloned into a lentiviral vector. see, e.g., Milone et al, Mol. Ther. 17(8): 1453-1464 (2009).

Another example of a promoter is the immediate early cytomegalovirus (CMV) promoter sequence. This promoter sequence is a strong constitutive promoter sequence capable of driving high levels of expression of any polynucleotide sequence operatively linked thereto. However, other constitutive promoter sequences may also be used, including, but not limited to the simian virus 40 (SV40) early promoter, mouse mammary tumor virus (MMTV), human immunodeficiency virus (HIV) long terminal repeat (LTR) promoter, MoMuLV promoter, an avian leukemia virus promoter, an Epstein-Barr virus immediate early promoter, a Rous sarcoma virus promoter, as well as human gene promoters such as, but not limited to, the actin promoter, the myosin promoter, the elongation factor-1α promoter, the hemoglobin promoter, and the creatine kinase promoter. Further, the invention should not be limited to the use of constitutive promoters. Inducible promoters are also contemplated as part of the invention.

In some embodiments, the present invention provides methods for expressing the CAR construct of the present invention in mammalian immune effector cells, such as mammalian T cells or mammalian NK cells, and the immune effector cells created thereby.

A source of cells, e.g., immune effector cells (e.g., T cells or NK cells), is obtained from a subject. The term "subject" is intended to include living organisms in which an immune response can be elicited (e.g., mammals). T cells can be obtained from a number of sources, including peripheral blood mononuclear cells, bone marrow, lymph node tissue, cord blood, thymus tissue, tissue from a site of infection, ascites, pleural effusion, spleen tissue, and tumors.

T cells can be obtained from a unit of blood collected from a subject using any number of techniques known to the skilled artisan, such as Ficoll^{™} separation. In one preferred aspect, cells from the circulating blood of an individual are obtained by apheresis. The apheresis product typically contains lymphocytes, including T cells, monocytes, granulocytes, B cells, other nucleated white blood cells, red blood cells, and platelets. In one embodiment, the cells collected by apheresis may be washed to remove the plasma fraction and to place the cells in an appropriate buffer or media for subsequent processing steps. In one aspect of the invention, the cells are washed with phosphate buffered saline (PBS).

A specific subpopulation of T cells, such as CD3+, CD28+, CD4⁺, CD8+, CD45RA+, and CD45RO+T cells, can be further isolated by positive or negative selection techniques. For example, in one embodiment, T cells are isolated by incubation with anti-CD3/anti-CD28-conjugated beads (e.g., DYNABEADS^{®} M-450 CD3/CD28 T), for a time period sufficient for positive selection of the desired T cells. In some embodiments, the time period ranges from 30 minutes to 36 hours or longer. Longer incubation times may be used to isolate T cells in any situation where there are few T cells, such in isolating tumor infiltrating lymphocytes (TIL) from tumor tissue or from immunocompromised individuals. Further, use of longer incubation times can increase the efficiency of capture of CD8⁺ T cells. Thus, by simply shortening or lengthening the time, T cells are allowed to bind to the CD3/CD28 beads and/or by increasing or decreasing the ratio of beads to T cells, subpopulations of T cells can be preferentially selected for at culture initiation or at other time points during the process.

Enrichment of the T cell population can be accomplished by a negative selection process using a combination of antibodies, wherein the antibodies are directed against surface markers unique to the negatively selected cells. One method is to sort and/or select cells with the aid of negative magnetic immunoadhesion or flow cytometry, using a mixture of monoclonal antibodies directed against cell surface markers present on the negatively selected cells.

In some embodiments, the immune effector cell may be an allogeneic immune effector cell, e.g., a T cell or an NK cell. For example, the cell may be an allogeneic T cell, e.g., an allogeneic T cell lacking expression of functional T cell receptor (TCR) and/or human leukocyte antigen (HLA) (e.g., HLA class I and/or HLA class II).

A T cell lacking a functional TCR can be, e.g., engineered such that it does not express any functional TCR on its surface, engineered such that it does not express one or more subunits that comprise a functional TCR (e.g., engineered such that it does not express or exhibits reduced expression of TCRα, TCRβ, TCRγ, TCRδ, TCRε, and/or TCRζ) or engineered such that it produces very little functional TCR on its surface.

A T cell described herein, for example, may be engineered such that it does not express any functional HLA on its surface. For example, a T cell described herein may be engineered such that cell surface expression of HLA (e.g., HLA class I and/or HLA class II) is downregulated. In some aspects, downregulation of HLA may be achieved by reducing or eliminating expression of beta-2 microglobulin (B2M).

In some embodiments, T cell may lack functional TCRs and functional HLAs, e.g., HLA class I and/or HLA class II.

In one embodiment, the cells transduced by the nucleic acid encoding the CAR of the present invention are proliferated, e.g., the cells are proliferated under culture for 2 hours to about 14 days.

The CAR-expressing immune effector cells obtained after in vitro proliferation can be tested for effector functions as described in Examples.

### IV. Antibodies that specifically bind to the BCMA molecule and antibodies further comprising mutated Fc domain

The present invention provides an antibody that binds to BCMA with high target specificity and high affinity, comprising a heavy chain variable region and a light chain variable region, wherein
(a) the heavy chain variable region comprises according to the Kabat numbering CDR H1 shown in amino acid sequence of SSSYYWT (SEQ ID NO: 25); or a variant of the CDR H1 with no more than 2 amino acid changes or no more than 1 amino acid change; CDR H2 shown in amino acid sequence of SISIAGSTYYNPSLKS(SEQ ID NO: 26); or a variant of the CDR H2 with no more than 2 amino acid changes or no more than 1 amino acid change; and CDR H3 shown in amino acid sequence of DRGDQILDV (SEQ ID NO: 27); or a variant of the CDR H3 with no more than 2 amino acid changes or no more than 1 amino acid change; the light chain variable region comprises according to the Kabat numbering CDR L1 shown in amino acid sequence of RASQSISRYLN(SEQ ID NO: 28); or a variant of the CDR L1 with no more than 2 amino acid changes or no more than 1 amino acid change; CDR L2 shown in amino acid sequence of AASSLQS(SEQ ID NO: 29); or a variant of the CDR L2 with no more than 2 amino acid changes or no more than 1 amino acid change; and CDR L3 shown in amino acid sequence of QQKYFDIT(SEQ ID NO: 30); or a variant of the CDR L3 with no more than 2 amino acid changes or no more than 1 amino acid change;
(b) the heavy chain variable region comprises according to the Kabat numbering CDR H1 shown in amino acid sequence of NDVIS (SEQ ID NO: 31); or a variant of the CDR H1 with no more than 2 amino acid changes or no more than 1 amino acid change; CDR H2 shown in amino acid sequence of VIIPIFGIANYAQKFQG(SEQ ID NO: 32); or a variant of the CDR H2 with no more than 2 amino acid changes or no more than 1 amino acid change; and CDR H3 shown in amino acid sequence of GRGYYSSWLHDI(SEQ ID NO: 33); or a variant of the CDR H3 with no more than 2 amino acid changes or no more than 1 amino acid change; the light chain variable region comprises according to the Kabat numbering CDR L1 shown in amino acid sequence of QASQDITNYLN(SEQ ID NO: 34); or a variant of the CDR L1 with no more than 2 amino acid changes or no more than 1 amino acid change; CDR L2 shown in amino acid sequence of DASNLET(SEQ ID NO: 35); or a variant of the CDR L2 with no more than 2 amino acid changes or no more than 1 amino acid change; and CDR L3 shown in amino acid sequence of QQAFDLIT(SEQ ID NO: 36); or a variant of the CDR L3 with no more than 2 amino acid changes or no more than 1 amino acid change;
wherein the amino acid change is the addition, deletion or conservative substitution of an amino acid.

In some embodiments, the BCMA molecule binding antibody of the present invention binds to BCMA from a mammal, such as human, cynomolgus monkey, mouse, rat and rabbit.

In some embodiments, the antibodies of the present invention that bind to BCMA molecule have one or more of the following properties.
(1) Specifically binding to BCMA;
(2) Binding to human BCMA and cross-reacting with cynomolgus monkey, mouse, rat, and rabbit BCMA;
(3) Killing BCMA-positive cancer cells by antibody-dependent cytotoxicity and/or antibody dependent cell phagocytosis (ADCP) in the absence of a mutated Fc domain, for example, in the case of having an unmutated parent antibody Fc domain.

In some embodiments, the BCMA molecule binding antibody of the present invention comprises a heavy chain variable region and a light chain variable region that specifically bind to BCMA, wherein
(a) the heavy chain variable region comprises a sequence shown in SEQ ID NO: 2 or a sequence having at least 90%, 91%, 92%, 93%, 94%, 95%, 96%, 97%, 98% or 99% identity thereto, and the light chain variable region comprises a sequence shown in SEQ ID NO: 3 or a sequence having at least 90%, 91%, 92%, 93%, 94%, 95%, 96%, 97%, 98% or 99% identity thereto;
(b) the heavy chain variable region comprises a sequence shown in SEQ ID NO: 9 or a sequence having at least 90%, 91%, 92%, 93%, 94%, 95%, 96%, 97%, 98% or 99% identity thereto, and the light chain variable region comprises a sequence shown in SEQ ID NO: 10 or a sequence having at least 90%, 91%, 92%, 93%, 94%, 95%, 96%, 97%, 98% or 99% identity thereto;
wherein the amino acid change in the sequence having at least 90%, 91%, 92%, 93%, 94%, 95%, 96%, 97%, 98% or 99% identity is preferably an amino acid substitution, more preferably an amino acid conservative substitution, and preferably, the amino acid change does not occur in the CDR regions.

In some embodiments, the BCMA molecule binding antibody of the present invention is IgG1, IgG2, IgG3 or IgG4 antibody; preferably, it is IgG1 or IgG4 antibody; more preferably, it is IgG1 antibody, for example, human IgG1 antibody.

In some embodiments, the BCMA molecule binding antibody provided in the present invention comprises a mutated Fc domain, wherein the amino acid at position P329 according to the EU numbering is mutated to glycine (G), and Fcγ receptor binding of the mutated Fc domain is reduced, compared with Fcy receptor binding of the Fc domain of the parent antibody that is not mutated. For example, the mutated Fc domain is a mutated Fc domain of an IgG1, IgG2, IgG3 or IgG4 antibody; preferably, the mutated Fc domain is a mutated Fc domain of an IgG1 or IgG4 antibody; more preferably, the mutated Fc domain is a mutated Fc domain of an IgG1 antibody. For example, the mutated Fc domain is a mutated Fc domain of a human IgG1 antibody.

BCMA molecule binding antibodies comprising P329G mutated Fc domain cannot exert antibody dependent cytotoxicity by binding to Fcy receptor, and cannot exert antibody dependent phagocytosis (ADCP).

In some embodiments, the present invention provides a nucleic acid encoding any of the above antibody that binds to BCMA molecule, or fragment thereof, or any chain thereof. In one embodiment, a vector comprising the nucleic acid is provided. In one embodiment, the vector is an expression vector. In one embodiment, a host cell comprising the nucleic acid or the vector is provided. In one embodiment, the host cell is eukaryotic. In another embodiment, the host cell is selected from yeast cells, mammalian cells (such as CHO cells or 293 cells), or other cells suitable for preparing the antibody or antigen-binding fragment thereof. In another embodiment, the host cell is prokaryotic.

For example, the nucleic acids of the present invention comprise nucleic acids encoding the antibody of the present invention that binds to BCMA molecule. In some embodiments, one or more vectors comprising the nucleic acids are provided. In one embodiment, the vector is an expression vector, such as a eukaryotic expression vector. Vectors include but are not limited to viruses, plasmids, cosmids, λ phages or yeast artificial chromosomes (YAC). In one embodiment, the vector is a pcDNA3.4 expression vector.

Once the expression vector or DNA sequence has been prepared for expression, the expression vector can be transfected or introduced into suitable host cells. Various techniques can be used for this purpose, for example, protoplast fusion, calcium phosphate precipitation, electroporation, retroviral transduction, viral transfection, biolistics, lipid-based transfection, or other conventional techniques. In the case of protoplast fusion, cells are cultured in a medium and screened for appropriate activity. Methods and conditions for culturing the resulting transfected cells and for recovering the resulting antibody molecules are known to those skilled in the art and may be changed or optimized according to the particular expression vector and the particular mammalian host cell used based on the present description and methods known in the prior art.

Additionally, cells having stably incorporated DNA in chromosomes thereof can be selected by introducing one or more markers permitting the selection of transfected host cells. The markers may, for example, provide prototrophy, biocidal (e.g., antibiotics) resistance, or heavy metal (e.g., copper) resistance, etc., for an auxotrophic host. Selectable marker genes may be connected directly to a DNA sequence to be expressed or introduced through co-transformation into the same cell. Additional elements may also be required for optimal synthesis of mRNA. The elements may include splicing signals, transcriptional promoters, enhancers, and termination signals.

In one embodiment, provided is a host cell comprising the polynucleotide of the present invention. In some embodiments, provided is a host cell comprising the expression vector of the present invention. In some embodiments, the host cell is selected from a yeast cell, a mammalian cell, and other cells suitable for preparing an antibody. Suitable host cells include prokaryotic microorganisms, such as *E. coli.* The host cells may also be eukaryotic microorganisms such as filamentous fungi or yeast, or various eukaryotic cells such as insect cells. Vertebrate cells may also be used as hosts. For example, a mammalian cell line engineered to be suitable for suspension growth may be used. Examples of useful mammalian host cell lines include monkey kidney CV1 line (COS-7) transformed by SV40; human embryonic kidney line (HEK 293 or 293F cells), 293 cell, baby hamster kidney cell (BHK), monkey kidney cell (CV1), African green monkey kidney cell (VERO-76), human cervical cancer cell (HELA), canine kidney cell (MDCK), buffalo rat liver cell (BRL 3A), human lung cell (W138), human liver cell (Hep G2), Chinese hamster ovary cell (CHO cell), CHO-S cell, NSO cell, and myeloma cell line such as Y0, NS0, P3X63, and Sp2/0. For reviews of mammalian host cell lines suitable for protein production, see, e.g., Yazaki and Wu, Methods in Molecular Biology, vol. 248 (B. K. C. Lo ed., Humana Press, Totowa, NJ), pp. 255-268 (2003). In one preferred embodiment, the host cell is a CHO cell or a HEK293 cell.

In one embodiment, the present invention provides a method for preparing antibodies (including P329G mutated antibodies) that bind to BCMA molecule, which comprises culturing a host cell comprising a nucleic acid encoding the antibody (including P329G mutated antibody) that binds to BCMA molecule or an expression vector comprising the nucleic acid under conditions suitable for expressing the nucleic acid encoding the antibody (including P329G mutated antibody) that binds to BCMA molecule, and optionally isolating the antibody (including P329G mutated antibody) that binds to BCMA molecule. In a certain embodiment, the method further comprises recovering the antibody (including P329G mutated antibody) that binds to BCMA molecule from the host cell (or host cell culture medium).

The antibody (including P329G mutated antibody) that binds to BCMA molecule prepared as described herein can be purified by known prior art such as high performance liquid chromatography, ion exchange chromatography, gel electrophoresis, affinity chromatography, size exclusion chromatography, etc. The actual conditions used to purify a particular protein also depend on factors such as net charge, hydrophobicity, hydrophilicity, etc., and these will be apparent to those skilled in the art. The purity of the antibody (including P329G mutated antibody) of the present invention that binds to BCMA molecule can be determined by any of a variety of well-known analytical methods, which include size exclusion chromatography, gel electrophoresis, high performance liquid chromatography, and the like.

The antibody (including P329G mutated antibody) provided herein that binds to BCMA molecule can be identified, screened, or characterized for physical/chemical properties and/or biological activities through a variety of assays known in the art. In one aspect, the antibody (including P329G mutated antibody) of the invention that binds to BCMA molecule is tested for the antigen-binding activity, for example, by known methods such as FACS, ELISA or Western blotting. The binding to BCMA can be determined by methods known in the art, and exemplary methods are disclosed herein. In some embodiments, the binding of the BCMA molecule-binding antibody (including P329G mutated antibody) of the present invention to BCMA (such as human BCMA) on cell surface is determined by SPR or biolayer interferometry.

The present invention also provides an assay for identifying the antibody (including P329G mutated antibody) having biological activity that binds to BCMA molecule. The biological activity can include, for example, ADCC effect, CDC effect, etc.

Cells used in any of the above *in vitro* assays include a cell line that naturally expresses BCMA or is engineered to express BCMA. The cell line that is engineered to express BCMA is a cell line that does not express BCMA under normal conditions but expresses BCMA after transfecting DNA encoding BCMA into the cells.

### V. Pharmaceutical combination of the present invention

For optimizing the safety and efficacy of CAR therapy, the molecular switch-regulated chimeric antigen receptor of the present invention is a controllable CAR, having the CAR activity under control. The present invention uses a mutated antibody having Pro329Gly (proline at position 329 of the antibody Fc region according to the EU numbering is mutated to glycine, abbreviated as P329G) as a safety switch in CAR therapy of the present invention. In the absence of the P329G mutated antibody, the CAR activity of the present invention is turned off; in the presence of the P329G mutated antibody, the CAR activity of the present invention is turned on; thus, the on and off of the CAR molecular activity of the present invention are regulated by the P329G mutated antibody.

In some embodiments, the present invention provides a pharmaceutical combination comprising (i) an immune effector cell (e.g., T cells, NK cells) expressing a molecular switch-regulated CAR polypeptide of the present invention; and (ii) a P329G mutated antibody that specifically binds to BCMA molecule. For example, the immune effector cell is a T cell expressing the molecular switch-regulated CAR polypeptide of the present invention, prepared from an autologous T cell or an allogeneic T cell, e.g., the immune effector cell is a T cell expressing the molecular switch-regulated CAR polypeptide of the present invention, prepared from a T cell isolated from human PBMC. In some embodiments, the P329G mutated antibody is ADI-38497 PG Ab and/or ADI-38484 PG Ab.

In some embodiments, the present invention provides a pharmaceutical combination comprising (i) a nucleic acid molecule encoding a molecular switch-regulated CAR polypeptide of the present invention or a vector comprising the nucleic acid component; and (ii) a P329G mutated antibody that specifically binds to BCMA molecule.

In some embodiments, the pharmaceutical combination of the present invention optionally further comprises a pharmaceutically acceptable auxiliary material suitable for formulation. For example, (ii) in the pharmaceutical combination may be formulated according to conventional methods (e.g., Remington's Pharmaceutical Science, latest edition, Mark Publishing Company, Easton, U.S.A.).

In some embodiments, the pharmaceutical combination of the present invention is used to treat a BCMA-related disease, such as a cancer that expresses or overexpresses BCMA, the cancer being, for example, relapsed/refractory multiple myeloma (RRMM).

### VI. Uses of the pharmaceutical combination of the present invention and methods of treatment using the pharmaceutical combination of the present invention

The present invention provides uses of the above pharmaceutical combination of the invention, for the treatment of BCMA-related disease in subjects, and the disease is, for example, a cancer expressing or overexpressing BCMA, and the cancer is, for example, relapsed/refractory multiple myeloma (RRMM).

In one embodiment, the pharmaceutical combination of the present invention is used in a subject to treat a cancer that expresses or overexpresses BCMA and can reduce the severity of at least one symptom or indication of the cancer or inhibit the growth of cancer cells. The cancer is, for example, relapsed/refractory multiple myeloma (RRMM).

The present invention provides methods for treating BCMA-related diseases (e.g., cancers expressing or overexpressing BCMA, the cancers being, for example, relapsed/refractory multiple myeloma (RRMM)) in subjects comprising administering a therapeutically effective amount of the pharmaceutical combination of the present invention to an individual in need thereof.

The present invention provides uses of the foregoing pharmaceutical combination of the present invention in the manufacture of a medicament for the treatment of a BCMA-related disease (e.g., cancers expressing or overexpressing BCMA, the cancers being, for example, relapsed/refractory multiple myeloma (RRMM)).

The pharmaceutical combination of the present invention can also be administered to individuals who have had their cancer treated with one or more prior therapies but later relapsed or metastasized, e.g., the cancer is relapsed/refractory multiple myeloma (RRMM).

In some embodiments, (i) an immune effector cell (e.g., T cell, NK cell) expressing a molecular switch-regulated CAR polypeptide of the present invention; and (ii) a P329G mutated antibody that specifically binds to BCMA molecule, in the pharmaceutical combination of the present invention are administered parenterally, transdermally, intracavitarily, intraarterially, intravenously or intrathecally, or directly injected into a tissue or tumor. In some embodiments, the (ii) P329G mutated antibody that specifically binds to BCMA molecule in the pharmaceutical combination of the present invention is administered before, simultaneously with, or after the (i) immune effector cells (e.g., T cells, NK cells) expressing the molecular switch-regulated CAR polypeptide of the present invention.

In some embodiments, the (i) immune effector cells expressing the molecular switch-regulated CAR polypeptide of the present invention in the pharmaceutical composition of the present invention, are T cells expressing the CAR polypeptide of the present invention, prepared from autologous T cells or allogeneic T cells; and the (ii) P329G mutated antibody that specifically binds to BCMA molecule in the pharmaceutical composition of the present invention is any antibody specifically binding to BCMA molecule, which comprises the P329G mutation. Preferably, the P329G mutated antibody is ADI-38497 PG Ab and/or ADI-38484 PG Ab.

When component (i) in the pharmaceutical combination of the present invention is immune effector cells (e.g., T cells, NK cells) expressing the molecular switch-regulated CAR polypeptide of the present invention, the present invention does not limit the order in which component (i) and component (ii) in the pharmaceutical combination of the present invention are administered to a subject, nor does it limit the timing between administrations of component (i) and component (ii) in the pharmaceutical combination of the present invention to a subject. Thus, components (i) and (ii) in the pharmaceutical combination of the present invention may be administered separately, simultaneously, or sequentially. When the two components are not administered simultaneously, the two components may be administered at a time interval of 1 hour, 2 hours, 4 hours, 6 hours, 12 hours, 24 hours, 48 hours, or 72 hours, or at any suitable time interval readily determined by those skilled in the art. For example, (i) is administered intravenously on the first day, (ii) is administered on the second day, and then (ii) is administered multiple times at a certain frequency, meanwhile an in vivo PK concentration of (i) and a desired therapeutic efficacy endpoint are monitored to determine whether to administer (i) multiple times; or (ii) is administered on the first day, (i) is administered intravenously on the second day, and then (ii) is administered multiple times at a certain frequency, meanwhile an in vivo PK concentration of (i) and a desired therapeutic efficacy endpoint are monitored to determine whether to administer (i) multiple times; or (i) and (ii) are administered separately at a time interval of 1 h, 2 h, 4 h, 6 h, or 12 h on the same day, and then (ii) is administered multiple times at a certain frequency, meanwhile an in vivo PK concentration of (i) and a desired therapeutic efficacy endpoint are monitored to determine whether to administer (i) multiple times.

In some embodiments, where component (i) in the pharmaceutical composition of the present invention is immune effector cells (e.g., T cells, NK cells) expressing the molecular switch-regulated CAR polypeptide of the present invention, it also comprises the situation of pre-incubating component (i) and component (ii) together prior to administration to a subject. Thus, both components may be pre-incubated for 1 minute, 5 minutes, 10 minutes, 15 minutes, 30 minutes, 45 minutes, or 1 hour, or any suitable time readily determined by those skilled in the art, prior to administration.

The pharmaceutical combination of the present invention may be administered to a subject at appropriate doses. The dosing regimen will be determined by the physician and according to clinical factors. As is well known in the medical field, the dose to be administered to any one patient depends on many factors, including the patient's body weight, body surface area, age, the particular compound to be administered, gender, time and route of administration, general health status, and other drugs to be administered in parallel.

In some embodiments, where component (i) in the pharmaceutical composition of the present invention is immune effector cells (e.g., T cells, NK cells) expressing the molecular switch-regulated CAR polypeptide of the present invention, component (i) is administered intravenously one or more times to a subject at a dose of 1×10⁶ cells/kg body weight - 10×10⁶ cells/kg body weight, for example 1×10⁶ cells/kg body weight, 2×10⁶ cells/kg body weight, 3×10⁶ cells/kg body weight, 5×10⁶ cells/kg body weight, 7×10⁶ cells/kg body weight, 9×10⁶ cells/kg body weight, 10×10⁶ cells/kg body weight; and component (ii) is administered, preferably parenterally, more preferably intravenously, to the subject in the form of a dose unit of 0.1-10mg/kg, preferably 0.1mg/kg, 0.3mg/kg, 0.5mg/kg, 1mg/kg, 3mg/kg, 5mg/kg, 7mg/kg, 9mg/kg, 10mg/kg.

In some embodiments, the administration of the pharmaceutical combination of the present invention to an individual suffering from cancer results in complete disappearance of the tumor. In some embodiments, the administration of the pharmaceutical combination of the present invention to an individual suffering from cancer results in a reduction in tumor cells or tumor size of at least 85% or more. The reduction in tumor can be measured by any method known in the art, such as X-ray, positron emission tomography (PET), computed tomography (CT), magnetic resonance imaging (MRI), cytology, histology, or molecular genetic analysis.

In some embodiments, the pharmaceutical combination of the present invention can reduce the "on-target/off tumor" toxicity of CAR-T cells.

### VII. Kit of the invention

The present invention provides a kit of parts, comprising the pharmaceutical combination of the present invention, preferably in the form of a dosage unit of a medicament. The dosage unit can thus be provided according to a dosing regimen or drug administration interval.

In one embodiment, the kit of the present invention comprises in the same package:
(i) selected from an immune effector cell (e.g., a T cell and an NK cell) expressing a molecular switch-regulated CAR polypeptide of the present invention, a nucleic acid molecule encoding the CAR polypeptide of the present invention, a vector comprising the nucleic acid, and any combination thereof; and
(ii) P329G mutated antibody that specifically bind to BCMA molecule.

The various embodiments/technical solutions described herein and the features in the various embodiments/technical solutions should be understood as being capable of arbitrarily combined with each other, and the various solutions resulting from these combinations are included within the scope of the present invention as if the solutions are specifically and individually set forth herein, unless the context clearly indicates otherwise.

The following examples are described to assist in understanding the present invention. The examples are not intended to be and should not be interpreted in any way as limiting the protection scope of the present invention.

### Examples

### Example 1. Synthesis of CAR gene, construction of viral expression vector, preparation of P329G CAR-T cell, and detection of CAR expression

### (1-1) Synthesis of CAR gene, and construction of viral expression vector

A P329G CAR molecule (SEQ ID NO: 1), also named as HuR968B CAR, was constructed by fusing a signal peptide (SP) shown in SEQ ID NO: 11, a single-chain antibody fragment specifically recognizing a P329G antibody (VH-linker-VL, having the VH shown in SEQ ID NO: 12, the linker sequence shown in SEQ ID NO:41, the VL shown in SEQ ID NO: 13), a G4S hinge region shown in SEQ ID NO: 14, a CD8 transmembrane domain (CD8TM) shown in SEQ ID NO:15, a 41BB co-stimulatory domain (41BB-CSD) shown in SEQ ID NO:16, and a CD3ζ molecule intracellular activation domain (CD3ζ SSD) shown in SEQ ID NO: 17.

In addition, Blue21 CAR (SEQ ID NO: 8), which directly targeted BCMA, was constructed and used as a control. Blue21 CAR from N-terminal to C-terminal comprises a signal peptide shown in SEQ ID NO: 11, an anti-BCMA single chain antibody (from clone 11D53), a CD8α molecule hinge region shown in SEQ ID NO:18 and a CD8 transmembrane domain shown in SEQ ID NO: 15, a 4-1BB co-stimulatory domain shown in SEQ ID NO: 16, and a CD3ζ chain intracellular activation domain shown in SEQ ID NO: 17.

The above DNA fragments encoding the CAR polypeptides were inserted downstream of the EF1α promoter of the pRK lentiviral expression vector (modified from pRRLSIN.cPPT.PGK-GFP.WPRE vector (Addgene, 12252, purchased from Biofeng) by replacing the promoter and the resistance gene in the vector), respectively, to replace the EGFR sequence in the vector, thus obtain the CAR expression plasmids pRK-HuR968B, pRK-Blue21.

### (1-2) Preparation of lentivirus concentrates

The CAR expression plasmid prepared in Example 1-1 was mixed with a structural plasmid pMDLg/pRRE(Addgene, 12251, purchased from Biofeng), a regulatory plasmid pRSV-rev (Addgene, 12253, purchased from Biofeng) and an envelope plasmid pMD2G (Addgene, 12259, purchased from Biofeng) in a mass ratio of 3:3:2:2, transfected into Lenti-X-293T cells (Takara) using PEI transfection method. 16 h after the transfection, the medium was replaced with fresh DEME medium comprising 2% fetal bovine serum (FBS) and the cells were further cultured for 48 h. Cell supernatant was collected, with cell debris removed by centrifugation. PEG8000 was added to the cell supernatant and incubated for 16-64 h at 4°C for lentivirus concentration. ed again. The supernatant was removed after centrifugation again, and the lentiviral precipitate was resuspended using T-cell medium to obtain a lentivirus concentrate, which was divided and stored frozen at -80°C.

### (1-3) P329G CAR-T cell preparation and CAR expression assay

A recombinant human interleukin-2 for injection ((China) Drug Administration Code S20040020) was added to TexMACS GMP Medium (Miltenyi Biotec, 170-076-309) to prepare T-cell medium with IL-2 concentration of 200 IU/ml.

PBMC cells from several donors were obtained from ORiCELLS. Specific information was as shown in Table 1 below.

**Table 1. Relevant source information of donor PBMC cells**

| PBMC cell NO. | Catalog NO. | Lot NO. | Donor ID number |
|---|---|---|---|
| Donor 3 PBMC | FPB004F-C | PCH2020110004 | Z0052 |
| Donor 4 PBMC | FPB005F-C | PCH20201200002 | Z0066 |
| Donor 5 PBMC | FPB004F-C | PCH20210100004 | Z0086 |
| Donor 6 PBMC | FPB004F-C | PCH20201200043 | Z0084 |
| Donor 7 PBMC | FPB004F-C | PCH20210100012 | Z0091 |

On day 0, the resuscitated PBMCs from each donor were sorted using a Pan T Cell Isolation Kit (human) (Miltenyi, 130-096-535) to obtain T cells, which were resuspended to a certain density using the T cell medium and activated by adding TransAct (Miltenyi, 130-111-160).

On day 1, a certain amount of T cells were isolated and cultured without the addition of lentivirus concentrate, which were un-transduced cells (UNT, un-transduced T cells), and the remaining T cells were added with lentivirus concentrate obtained from Example 1-2 (the lentivirus was a lentivirus encoding P329G CAR (SEQ ID NO: 1) or control conventional CAR (SEQ ID NO:8)) at MOI = 1~5 and the T cells were aspirated evenly; on day 2, the virus supernatant was removed by centrifugation and the cells were resuspended in a fresh T-cell medium. On day 3, all cells were transferred to G-Rex (WILSONWOLF, Catalog no. 80040S) with appropriate amount of fresh T-cell medium added and placed in a 37°C CO₂ incubator; every 2-3 days, cells were cultured with half the amount of medium replaced for fresh medium or directly supplemented with IL-2, where IL-2 was added until the IL-2 concentration in the cell medium was 200 IU/ml. The cells were harvested when the number of cells expanded to about 20-80 times to meet the demand (generally 2-8×10⁸ cells). After centrifugation and removal of the medium, CAR-T cells were resuspended using CryoStor^{®} CS10 (Stemcell, 07930) and divided into portions, and programmed to -80° C for freezing and storage.

Appropriate amount of CAR-T cells were taken, washed once with FACS buffer (PBS+ 2% FBS), and resuspended, then added FACS buffer comprising LIVE/DEAD Fixable Dead Cell Stain, stained for 10-15 min at room temperature, washed twice, and added PerCP-Cy5.5-CD3, BUV805-CD, Biotin-F(ab')₂ Fragment goat anti-human IgG (Jackson ImmunoResearch, 109-066-006, for PG CAR assay)or Biotin-F(ab')₂ Fragment goat anti-mouse IgG (Jackson ImmunoResearch, 115-066-006, for Blue 21 CAR assay)antibody combination, stained at 4°C for 30-45 min, washed twice and then added APC-Streptavidin, stained at 4°C for 30-45 min; cells were washed twice and resuspended with FACS buffer and detected by flow cytometry.

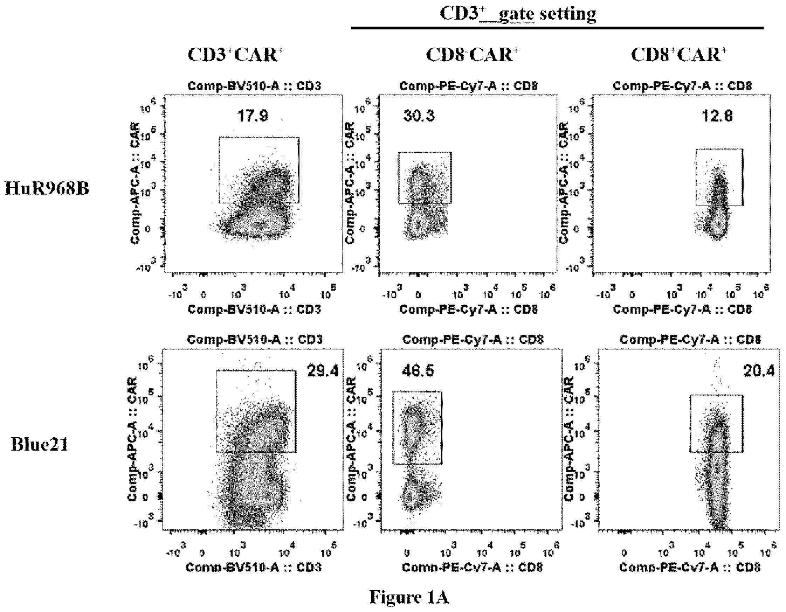

Figure 1A showed the expression of CARs in CD3⁺ cells, CD4⁺, and CD8⁺ T cell subsets after transduction of T cells with the 2 CARs constructed in Example 1-1, respectively, and the results showed that the positive CAR expression rates in these transduced T cells were about 18% to 29%.

### Example 2. BCMA-specific P329G mutated antibody and antigen binding activity assay

### (2-1) Synthesis of BCMA-specific antibodies

The heavy and light chain variable region sequences of BCMA parent antibody ADI-34861(VH shown in SEQ ID NO:37, VL shown in SEQ ID NO:38, respectively) and ADI-34857 (VH shown in SEQ ID NO:39, VL shown in SEQ ID NO:40, respectively) were obtained from International Application No. PCT/CN2019/074419 (BCMA antibody-related patent application). The heavy and light chain variable region sequences of ADI-38497 (SEQ ID NO:2, SEQ ID NO:3) were obtained by mutating the CDR regions of the parent antibody ADI-34861, and the heavy and light chain variable region sequences of ADI-38484 (SEQ ID NO: 9, SEQ ID NO: 10) were obtained by mutating the CDR regions of the parent antibody ADI-34857. Compared with the corresponding parent antibodies, the affinities of the mutated antibodies showed significant increases, and the specific experimental data were shown in Table 2 below.

**Table 2 Binding affinity of parent and mutated antibodies to BCMA**

| Antibodies | ForteBio IgG KD Human BCMA-IHM (M) monovalent | ForteBio IgG KD Human BCMA monomer (M) monovalent | ForteBio IgG KD Cynomolgus monkey BCMA-Fc (M) Avid | ForteBio IgG KD Mouse BCMA-Fc (M) Avid | Biacore Fab KD (M) Human BCMA-IHM monovalent | Cell Binding, Human BCMA CHO-S (FOB(Fold Over Background)) | Cell Binding, Human BCMA H929 (FON(Fold Over Negative)) |
|---|---|---|---|---|---|---|---|
| ADI-34861 | 5.14E-08 | 1.73E-08 | 2.11E-09 | 3.68E-09 | N.A. | 4 | 6 |
| ADI-38497 | 1.02E-09 | 6.21E-10 | 6.02E-10 | 5.03E-10 | 1.04E-10 | 8 | 26 |
| ADI-34857 | 2.77E-08 | N.B. | 5.93E-09 | N.B. | N.A. | 2 | 5 |
| ADI-38484 | 5.39E-10 | 7.94E-11 | 1.07E-09 | 8.50E-10 | 1.32E-11 | 11 | 35 |

The light and heavy chain variable region sequences of BCMA antibody clone J6M0 of GSK company were obtained from US9273141B2 patent, and used in a control antibody (designated as GSK IgG).

The light and heavy chain variable region sequences of GSK IgG, ADI-38497, and ADI-38484 antibodies were synthesized using the whole gene, and inserted into pcDNA3.4 expression vectors (purchased from Shanghai Bio-Innovation Biotechnology Co., Ltd.) comprising WT human IgG1 heavy chain constant region (SEQ ID NO:4) or comprising P329G site-mutated human IgG1 heavy chain constant region (SEQ ID NO:5), and comprising κ light chain constant region (SEQ ID NO:6). The obtained light and heavy chain expression vectors were co-transfected via PEI into HEK293 cells at a molar ratio of 2:3, and the medium supernatant was collected after 5-7 days of culture. The antibody-comprising supernatant was further purified through Protein A column and then dialyzed against PBS. The concentration was detected by reading the absorbance at 280 nm using a NanoDrop instrument, and the purity of the sample was detected by SDS-PAGE and SEC-HPLC. GSK WT antibody, GSK PG antibody; ADI-38497 WT antibody, ADI-38497 PG antibody; and ADI-38484 WT antibody, ADI-38484 PG antibody were obtained. The antibody having the heavy chain variable region sequence(SEQ ID NO:2) and the light chain variable region sequence(SEQ ID NO:3) of the BCMA antibody clone ADI-38497 was also named in this application as ADI-38497 antibody, including ADI-38497 PG antibody and ADI-38497 WT antibody; and the antibody having the heavy chain variable region sequence (SEQ ID NO:9) and the light chain variable region sequence (SEQ ID NO:10) of BCMA antibody clone ADI-38484 was also named in this application as ADI-38484 antibody, including ADI-38484 PG antibody and ADI-38484 WT antibody.

### (2-2) Antibody affinity assay

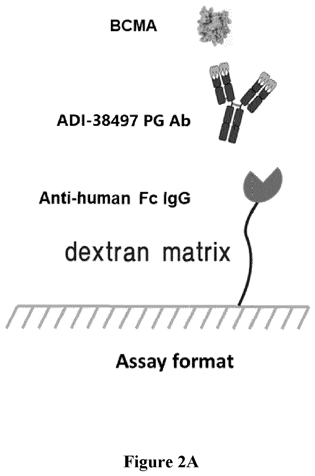

The affinity of ADI-38497 PG antibody to BCMA derived from different species was detected using Biacore T200. Figure 2A showed a schematic diagram of the method for detecting antibody affinity by surface plasmon resonance (SPR).

The particular method was as follows: After coupling anti-human Fc IgG (Ab97221, Abcam) to the surface of a CM5 chip (29149603, Cytiva), the ADI-38497 PG antibody was captured on the chip surface. Affinity and kinetic constants were obtained by detecting the association and dissociation between the antibody on the chip surface and the BCMA antigen in the mobile phase. 10-fold diluted 10×HBS-EP+ (BR-1006-69, Cytiva) was used as the experimental buffer during the assay. Each cycle in the affinity detection consists of capture of the ADI-38497 PG antibody, binding of one concentration of antigen, and regeneration of the chip. The gradient diluted antigens (1.25-40 nM of antigen concentration gradients, 2-fold dilution) flew over the surface of the chip from low concentration to high concentration at a flow rate of 30 µl/min, and the association time was 180s. A suitable dissociation time (900s or 600s or 60s) was set. Finally, the chip was regenerated with 10 mM glycine-HCl, pH 1.5 (BR-1003-54, Cytiva).

The data results were analyzed by Biacore T200 analysis software (version 3.1) using a 1:1 binding model.

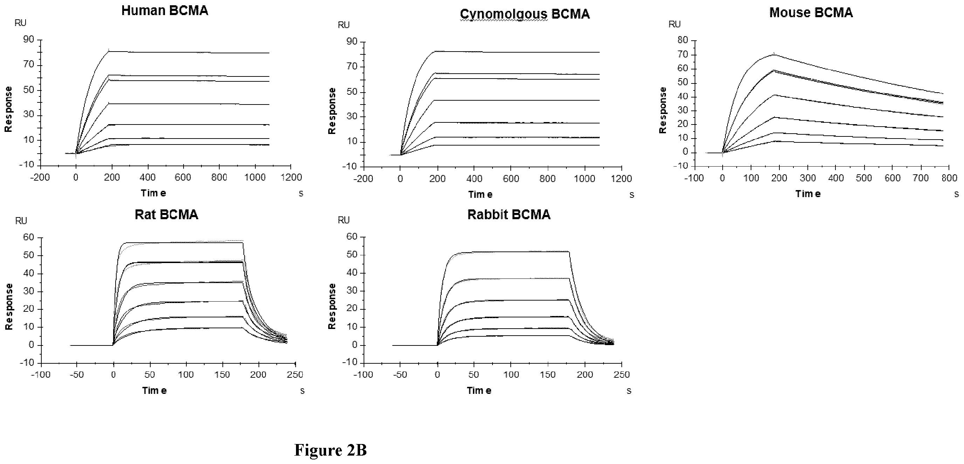

Figure 2B showed representative affinity profiles of ADI-38497 PG antibody to the recombinant human, cynomolgus monkey, mouse, rat and rabbit BCMA proteins, as measured by SPR. The results showed that ADI-38497 PG antibody could bind to BCMA proteins derived from above-mentioned different species, and the order of binding activity was human BCMA > monkey BCMA > mouse BCMA > rat BCMA > rabbit BCMA.

**Table 3 Binding affinity of ADI-38497 PG antibody to BCMA proteins from different species**

| Antigen | Antibody | ka (1/Ms) | kd (1/s) | KD (M) |
|---|---|---|---|---|
| Human BCMA | ADI-38497 PG Antibody | 2.878E+5 | 1.756E-5 | 6.101E-11 |
| cynomolgus monkey BCMA | | 3.402E+5 | 1.025E-5 | 3.012E-11 |
| Mouse BCMA | | 4.920E+5 | 8.716E-4 | 1.772E-9 |
| Rat BCMA | | 1.001E+6 | 8.314E-2 | 8.306E-8 |
| Rabbit BCMA | | 5.830E+4 | 5.309E-2 | 9.106E-7 |

### (2-3) Detection of binding activity of P329G BCMA antibody to BCMA antigens from different species

First, CHO GS cells expressing BCMA antigens from different species were prepared. Particularly, the BCMA genes from human, mouse, and cynomolgus monkey sources were synthesized and cloned into lentiviral vectors, and then lentiviruses comprising BCMA genes from different species were packaged, and CHO GS cells were infected with the lentiviruses. Then CHO GS cell lines expressing BCMA antigens from different species were obtained by flow cytometry sorting, named as hBCMA-CHO GS, mBCMA-CHO GS and cynoBCMA-CHO GS cells.

Then, FACS buffer was used to dilute ADI-38497 PG antibody and GSK-derived BCMA antibody (i.e., GSK PG IgG was used as Benchmark) into antibody solutions of different concentrations with 10-fold gradient dilution, which were respectively incubated with 1E5 CHO GS cells expressing BCMA antigens from different species at 4°C for 30 minutes. After washing with FACS buffer, the cells were then incubated with Fcγ fragment-specific APC-goat anti-human IgG (Jackson ImmunoResearch, 109-136-098) at 4°C for 30 minutes. The P329G antibody bound to the cells was detected by flow cytometry, and MFI of the APC channel was analyzed. Plotting was carried out with the antibody concentration as the X axis and the APC channel MFI as the Y axis and the binding EC50 was calculated.

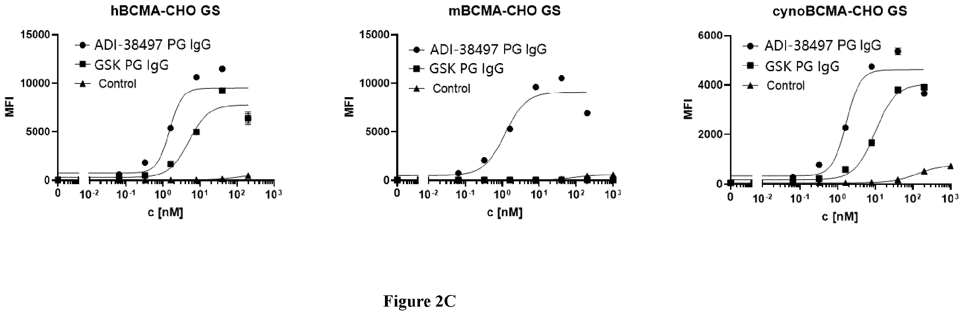

Figure 2C showed the binding ability of P329G BCMA antibody in different concentrations to CHO-GS cells stably expressing human BCMA, cynomolgus monkey BCMA or mouse BCMA. It could be seen from Figure 2C that ADI-38497 PG IgG antibody bound to different species derived BCMA expressed on the cell surface, while GSK-derived BCMA antibody (Benchmark) had higher specificity to species derived BCMA, which did not recognize mouse BCMA, and this result was consistent with the SPR detection result.

**Table 4 EC50 value of P329G BCMA antibody binding to CHO-GS cells expressing BCMA derived from different species**

| | Cell line | | | EC50(nM) of Antibody | | |
|---|---|---|---|---|---|---|
| | | ADI-38497 PG IgG | | | GSK PG IgG | |
| | hB CMA-CHO-GS | | 1.490 | | | 5.002 |
| mBCMA-CHO-GS | | | 1.183 | | | 131.9 |
| cynoBCMA-CHO-GS | | | 1.692 | | | 10.01 |

### (2-4) Detection of binding activity of P329G BCMA antibody to BCMA antigen on tumor cell surface

A proper amount of tumor cells in logarithmic growth phase was taken, and washed with FACS buffer twice. ADI-38497 PG antibody, ADI-38484 PG antibody, or GSK PG IgG used as Benchmark was added. Isotype hIgG 1 antibody was added to cells used as dyeing control. Dyeing was carried out at 4°C for 30 minutes and washing was performed twice. APC-F(ab')₂ Fragment Goat Anti-Human IgG antibody was added and dyeing was performed at 4°C for 30 minutes. Cells were washed twice, then suspended with FACS buffer, and detected using flow cytometry.

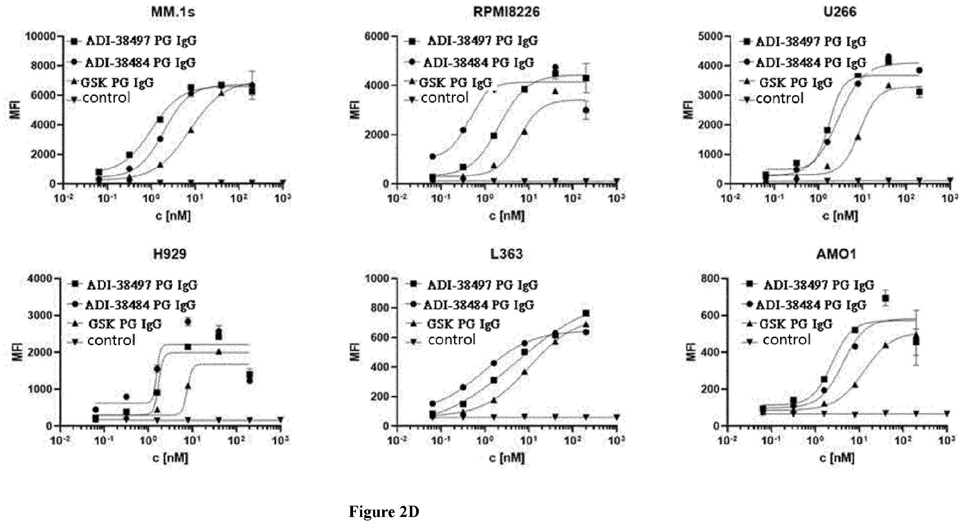

Figure 2D showed the binding activities of different concentrations of P329G BCMA antibody to positive multiple myeloma cell lines MM.1s, RPMI8226, U266, H929, L363 and AMO1 expressing BCMA (MM.1s was purchased from Nanjing Cobioer Biosciences Co., Ltd., CBP60239; RPMI8226 was purchased from Nanjing Cobioer Biosciences Co., Ltd., CBP60244; U266 was purchased from Wuhan Procell Life Science &Technology Co., Ltd., CL-0510; H929 was purchased from Nanjing Cobioer Biosciences Co., Ltd., CBP60243; L363 was purchased from Nanjing Cobioer Biosciences Co., Ltd., CBP6024; AMO1 was purchased from Nanjing Cobioer Biosciences Co., Ltd., CBP60242). ADI-38497 PG antibody, ADI-38484 PG antibody could bind to positive tumor cells expressing BCMA and exhibit antibody concentration dependency. Among the positive tumor cell lines expressing BCMA, MM.1s cells had the highest level of BCMA expression, RPMI8226, U266 and H929 cells expressed BCMA at a medium level, and L363 and AMO1 cells expressed BCMA at a low level.

**Table 5. EC50 value of P329G BCMA antibody binding to BCMA expressing positive tumor cells**

| | EC50(nM) of Antibody | | |
|---|---|---|---|
| Name of cell line | **ADI-38497 PG IgG** | **ADI-38484 PG IgG** | **GSK PG IgG** |
| MM.1s | 1.007 | 1.953 | 7.535 |
| RPMI8226 | 2.117 | 0.4280 | 5.972 |
| U266 | 1.795 | 2.789 | 8.736 |
| H929 | ∼ 1.713 | ∼ 1.548 | ∼ 7.686 |
| L363 | 5.113 | 0.9341 | 10.39 |
| AMO1 | 2.428 | 4.250 | 12.92 |

### Example 3. Binding activity assay of BCMA-specific P329G mutated antibody to the extracellular domain of P329G CAR

### (3-1) Affinity assay of P329G mutated antibody binding to the extracellular domain of P329G CAR

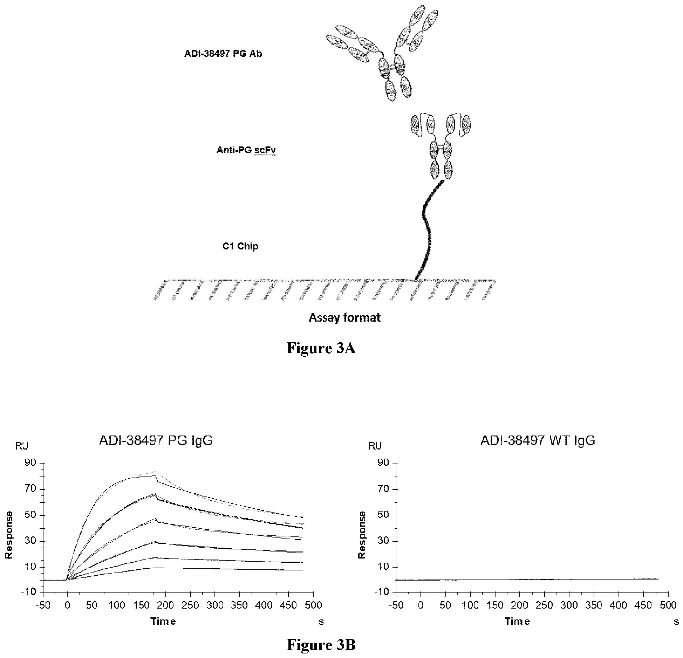

The binding affinity of ADI-38497 molecule (P329G mutated antibody or wild-type antibody) for anti-P329G mutation-specific single chain antibody-rabbit Fc fusion protein (also named as "anti-PG scFv fusion protein" or simply as "anti-PG scFv") was detected using Biacore T200. Figure 3A showed a schematic diagram of the method for detecting the affinity of anti-P329G mutation-specific single chain antibody-rabbit Fc fusion protein for ADI-38497 P329G mutation antibody by surface plasmon resonance (SPR).

The particular method was as follows: After coupling the synthesized anti-PG scFv fusion protein (SEQ ID NO:7) to the surface of a C1 chip (BR100535, Cytiva), affinity and kinetic constants were obtained by detecting the association and dissociation between the antibody on the chip surface and the ADI-38497 molecule in the mobile phase. 10-fold diluted 10×HBS-EP+ (BR-1006-69, Cytiva) was used as the experimental buffer during the assay. Each cycle in the affinity detection included binding of one concentration of antigen, and regeneration of the chip. The gradient diluted ADI-38497 molecule (3.125 nM-100 nM of concentration gradients, 2-fold dilution) was flowed over the surface of the chip from low concentration to high concentration at a flow rate of 30 µl/min, and the association time was 180s, the dissociation time was 300s. Finally, the chip was regenerated with 10 mM glycine-HCl, pH 1.5 (BR-1003-54, Cytiva).

The data results were analyzed by Biacore T200 analysis software (version 3.1) using a 1:1 binding model.

Figure 3B showed representative affinity profiles of the binding of ADI-38497 PG antibody and wild-type antibody to anti-PG scFv fusion protein, as measured by SPR. The results showed that only ADI-38497 PG antibody specifically bound to anti-PG scFv as the extracellular domain of P329G CAR.

**Table 6. Affinity of P329G BCMA antibody and wild type antibody for anti-PG scFv as determined by SPR**

| Analyte | Ligand | **Ka(1/Ms)** | **Kd(1/s)** | **KD(M)** |
|---|---|---|---|---|
| ADI-38497 PG IgG | Anti-PG scFv | 3.367E+5 | 1.929E-3 | 5.727E-9 |
| ADI-38497 WT IgG | Anti-PG scFv | No binding | | |

### (3-2) Binding avidity assay of ADI-38497 molecular for anti-PG scFv fusion protein

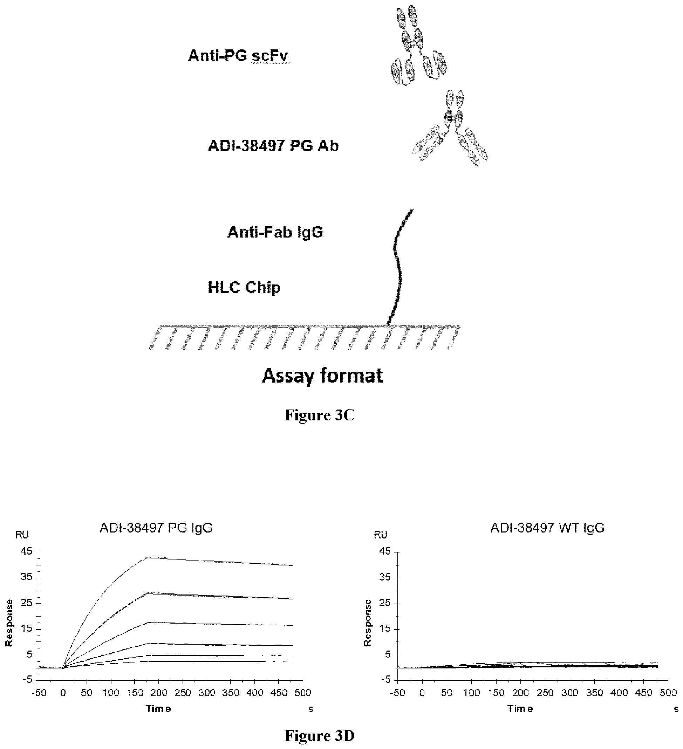

The binding avidity of ADI-38497 molecule (P329G mutated antibody or wild-type antibody) to anti-PG scFv fusion protein was detected using Biacore T200. Figure 3C showed a schematic diagram of the method for detecting the antibody avidity by surface plasmon resonance (SPR).

The particular method was as follows: After coupling anti-Fab IgG (15260, Sigma) to the surface of HLC chip (HLC30M, Xantec), ADI-38497 molecules were captured on the surface of the chip, and the avidity and kinetic constants were obtained by detecting the binding and dissociation of antibodies on the surface of the chip to anti-PG scFv fusion proteins in the mobile phase. The assay procedure was performed using a 10-fold dilution of 10× HBS-EP+ (BR-1006-69, Cytiva) as the experimental buffer. Each cycle of the avidity assay included capturing ADI-38497 molecules, binding one concentration of anti-PG scFv and chip regeneration. A gradient dilution of anti-PG scFv (concentration gradient of 1.25 nM-40 nM, 2-fold dilution) was flowed over the chip surface from low to high concentration in a flow rate of 30 µl/min, with an association time of 180 s and a dissociation time of 300 s. Finally, the chip was regenerated with 10 mM glycine-HCl, pH 1.5 (BR-1003-54, Cytiva).

The data results were analyzed by Biacore T200 analysis software (version 3.1) using a 1:1 binding model.

Figure 3D showed a representative avidity profile of ADI-38497 PG antibody and wild-type antibody for anti-PG scFv as the extracellular domain of the P329G CAR, as determined using SPR. The results showed that only ADI-38497 PG antibody specifically bound to P329G CAR.

**Table 7. Avidity of the P329G BCMA antibody and the wild-type antibody to anti-PG scFv as determined by SPR**

| Analyte | Ligand | Ka(1/Ms) | Kd(1/s) | KD(M) |
|---|---|---|---|---|
| Anti-PG scFv | ADI-38497 PG IgG | 2.292E+5 | 2.349E-4 | 1.025E-9 |
| Anti-PG scFv | ADI-38497 WT IgG | No binding | | |

### (3-3) Detection of binding activity of P329G BCMA antibody to P329 CAR

P329G CAR-T cells prepared in Example 1 were resuscitated, and resuspended in RPMI 1640 medium comprising 10% FBS, and incubated at 37° C for 24 hours to stabilize. The ADI-38497 PG antibody and the wild-type ADI-38497 WT antibody were prepared in FACS buffer as 5-fold gradient dilutions of different concentrations of antibody solutions, incubated respectively with 1E5 CAR-positive cells for 30 min at 4°C, washed with FACS buffer, and then incubated with Fcy fragment-specific APC goat anti-human IgG (Jackson ImmunoResearch, 109-136-098) for 30 min at 4°C. Antibody bound to cells was detected by flow cytometry, and APC channel MFI was analyzed, plotted with antibody concentration as the X-axis and APC channel MFI as the Y-axis and bound EC50 was calculated.

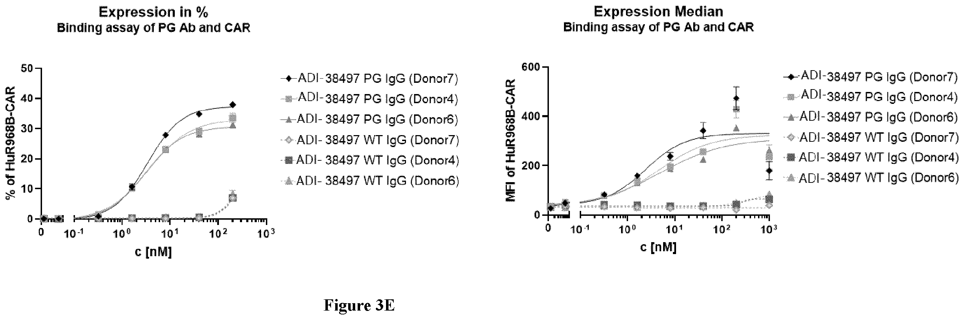

Figure 3E showed the binding ability of ADI-38497 WT antibody and ADI-38497 PG antibody to P329G CAR-T cells. The results showed that only P329G mutated antibody bound to CAR, while the WT antibody did not bind to CAR, which results were consistent with the SPR results.

Table 8 summarized the EC50 values and EC90 values of the binding of ADI-38497 PG antibody and WT antibody to P329G CAR-T cells from different donor sources.

**Table 8. EC50 values and EC90 values of the binding of BCMA IgG antibody to CAR-T cells**

| | Antibodies | EC₅₀ (nM) | EC₉₀ (nM) |
|---|---|---|---|
| Donor 4-HuR968B (26.2%) | ADI-38497 PG | 3.58 | 32.94 |
| | ADI-38497 WT | 1867 | |
| Donor 6-HuR968B (24.5%) | ADI-38497 PG | 3.01 | 21.87 |
| | ADI-38497 WT | 1135 | |
| Donor 7-HuR968B (29.3%) | ADI-38497 PG | 3.38 | 22.95 |
| | ADI-38497 WT | 486 | |

### Example 4. Functional assay of the Fc domain of BCMA-specific P329G mutated antibody

### (4-1) ADCC effector function assay of ADI-38497 PG antibody

PBMC cells (Peripheral Blood Mononuclear Cells) derived from donor 3 were resuscitated, resuspended in RPMI 1640 medium, and stabilized at 37°C for 1-2 hours. The PBMCs and target cells were mixed in terms of an effector to target ratio of 25:1, and mixed with different concentrations of BCMA antibodies, cultured at 37°C for 4 hours and 24 hours respectively. The antibody mediated killing effect of PBMCs on target cells was detected using LDH detection kit (Promega, G1780). The antibody concentrations were taken as the X axis, and the cell lysis ratios were taken as the Y axis for plotting and analysis. Cells were collected at the same time, washed twice with FACS buffer, and CD3 antibody, CD56 antibody, CD16 antibody and CD107a antibody were added, of which CD107a antibody should be added in advance, and incubated with cells at 37°C for 1 hour. The above mixtures of cells and antibodies were dyed at 4°C for 30 minutes, washed twice, suspended in FACS buffer, and detected using flow cytometry.

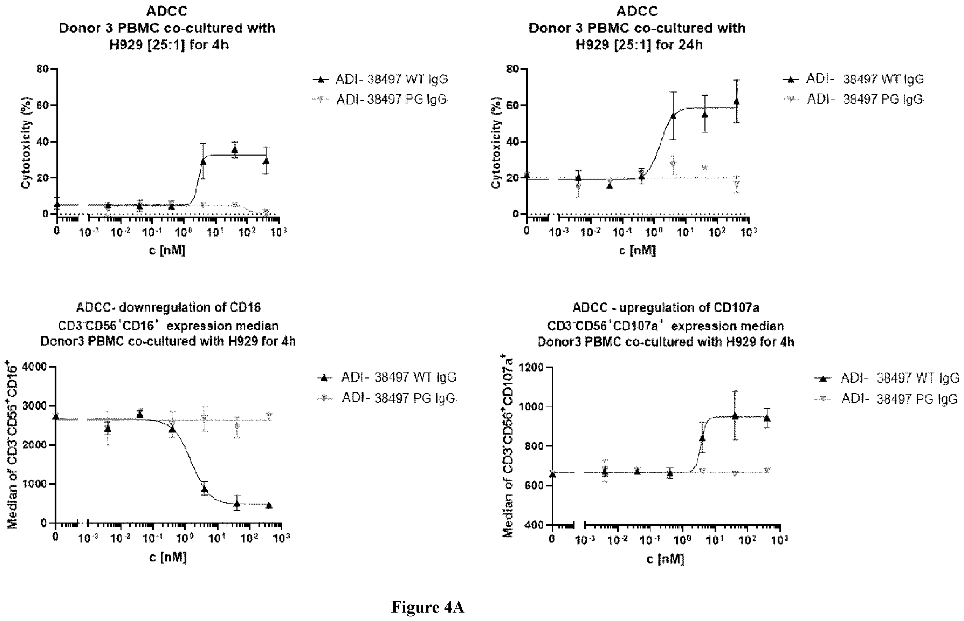

Figure 4A showed the abilities of ADI-38497 WT antibody and ADI-38497 PG antibody to mediate ADCC killing. The results showed that when testing different incubation time (4 hours, 24 hours) and using different detection indicators (cytotoxicity to target cells, influence on the expressions of CD3, CD56, CD16 and CD107a), only WT antibody could mediate the ADCC cytotoxicity killing effect on positive H929 tumor cells expressing BCMA, while P329G mutated antibody lacked the ability to mediate ADCC effect.

### (4-2) ADCP effector Function assay of ADI-38497 PG antibody

ADCP report cell line (Promega, G9871) and H929 cells in logarithmic growth phase were taken. ADCP report cells and H929 target cells were mixed in terms of an effector to target ratio of 2:1 or 5:1, and mixed with different concentrations of BCMA antibodies, continued to culture at 37°C for 20 hours. A luciferase detection kit (Promega, E2620) was used to detect the antibody mediated and target cell dependent activation effect of report cells. The antibody concentrations were used as the X axis, and fluorescence reading changes were taken as the Y axis for plotting and analysis.

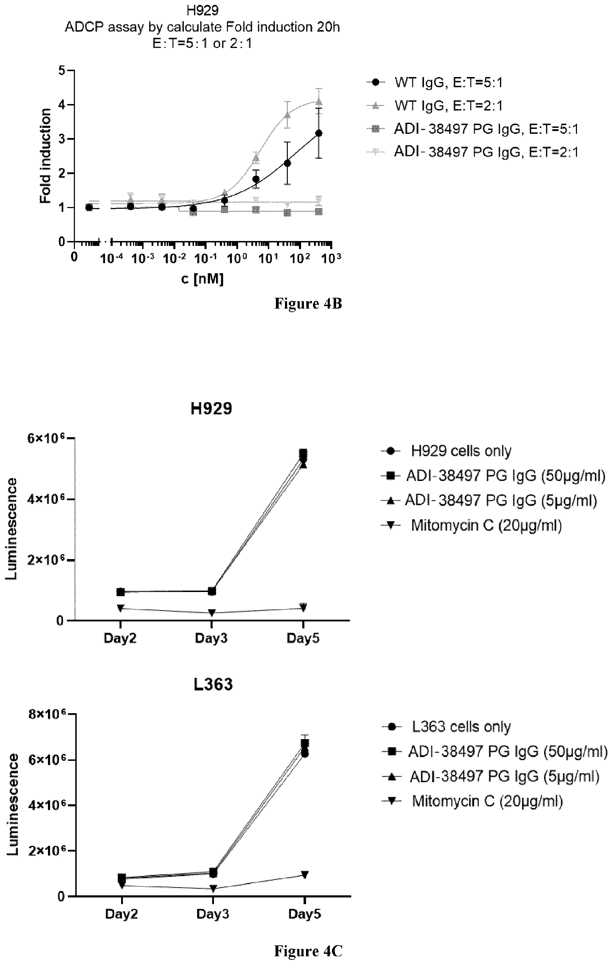

Figure 4B showed the ability of ADI-38497 WT antibody and ADI-38497 PG antibody to mediate ADCP killing. The results showed that when tested with different ratios of effector to target (2:1 or 5:1), only ADI-38497 WT antibody mediated ADCP killing effect on BCMA positive H929 tumor cells, while P329G mutated antibody lacked the ability to mediate ADCP killing effect on BCMA positive H929 tumor cells.

### (4-3) Functional assay of whether target cell lysis was mediated by using ADI-38497 PG antibody alone

H929 cells and L363 cells in logarithmic growth phase were taken, and a certain number of the cells were placed in a multiple-well plate; a part of H929 cells and a part of L363 cells in logarithmic growth phase as target cells were treated with mitomycin C, and used as negative controls. Different concentrations of ADI-38497 PG antibodies were added and mixed. They were cultured at 37°C for 48 hours, 72 hours and 120 hours respectively. The proportion of living cells was detected with CellTiter-Glo (Promega, G9242). The co-incubation time was taken as the X axis and the fluorescence reading value was taken as the Y axis for plotting and analysis.

Figure 4C showed whether ADI-38497 PG antibody was capable of mediating target cell lysis. The results showed that different incubation times (48 hours, 72 hours, and 120 hours) and different ADI-38497 PG antibody concentrations (5µg/ml, 50µg/ml) under test all indicated that using ADI-38497 PG antibody alone lacked the ability mediate target cell lysis.

### Example 5. Study of P329G CAR-T in vitro functions

### (5-1) CAR-T cell activation assay

CAR-T cells prepared from donor 5 in Example 1 were resuscitated, and stabilized by incubation at 37°C overnight. H929 or L363 tumor target cells and CAR-T cells were mixed in an E:T ratio of 2:1, and different concentrations of ADI-38497 PG antibody solution at 5-fold or 10-fold gradient dilutions were added to a total volume of 200 µL, placed in culture at 37° C for about 24 h and then centrifuged, cells were collected, washed twice with FACS buffer, resuspended, and added a FACS buffer comprising LIVE/DEAD Fixable Dead Cell Stain, Biotin-F(ab')₂ Fragment goat anti-human IgG (Jackson ImmunoResearch, 109-066-006), stained for 30 min at 4°C, washed twice, and added CD4, CD8, CD25, CD69 and APC-Streptavidin antibodies in combination., the above cell and antibody mixtures were stained at 4°C for 30 min, washed twice, and resuspended in FACS buffer, then detected by flow cytometry.

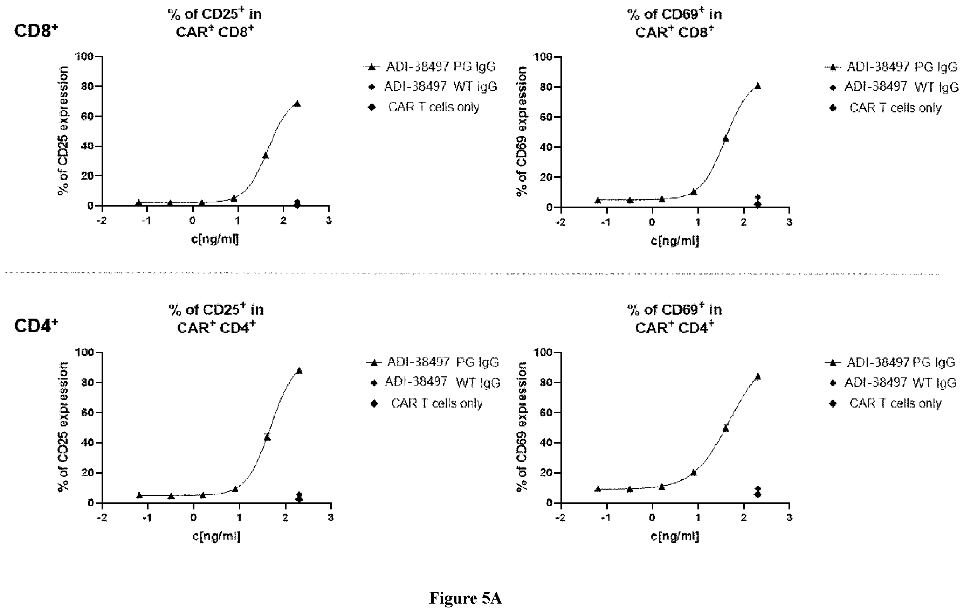

Figure 5A showed only ADI-38497 PG antibody comprising the P329G mutation against H929 cells via specifically mediating the activation of CAR⁺ -T, upregulating the expression levels of CD25, CD69, and activating CD4⁺ CAR⁺ and CD8⁺ CAR⁺ cells without difference and exhibiting a concentration gradient-dependence of ADI-38497 PG antibody.

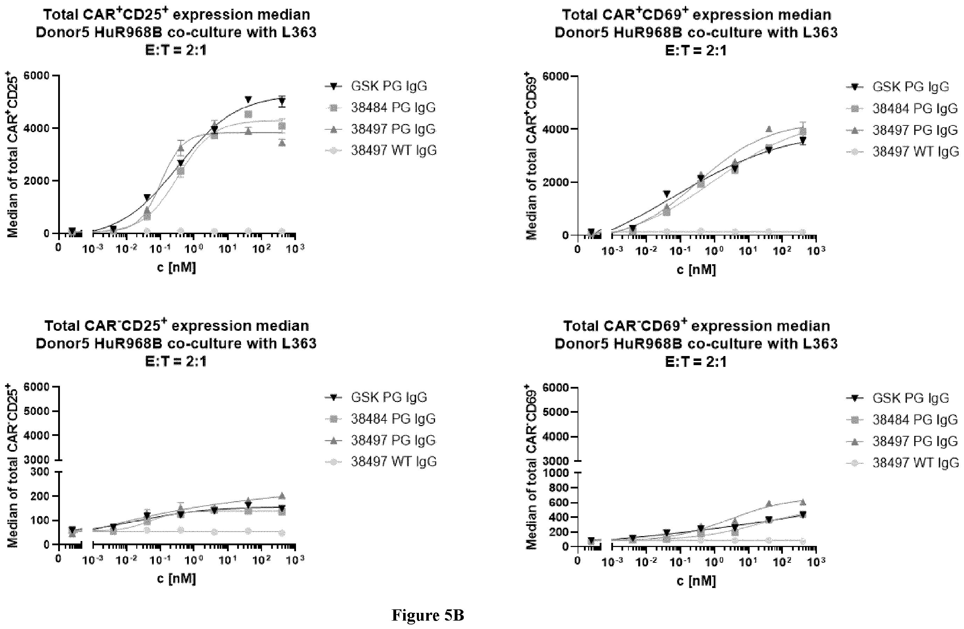

Figure 5B showed whether different BCMA antibodies (ADI-38497 PG antibody, ADI-38484 PG antibody, ADI-38497 WT antibody, GSK PG IgG) induced the activation effect of L363 target cells on HuR968B CAR-T cells. As seen in Figure 5B, BCMA antibodies comprising the P329G mutation (ADI-38497 PG antibody, ADI-38484 PG antibody, GSK PG IgG) specifically mediated the activation of CAR+ T cells, significantly upregulating the expression levels of CD25 and CD69. There was also a weak upregulation of CD25 and CD69 expressions in the CAR-negative cell population, indicating a lower level of activation, but this level of activation was insignificant and negligible.

Therefore, only BCMA antibodies comprising the P329G mutation (ADI-38497 PG antibody, ADI-38484 PG antibody, GSK PG IgG) specifically mediated the activation of CAR⁺ -T both in the case of H929 cells with high BCMA expression (Figure 5A) as target cells and in the case of L363 cells with low BCMA expression (Figure 5B) as target cells, significantly upregulating CD25, CD69 expression levels, with a concentration gradient-dependence of the P329G mutant BCMA antibody.

### (5-2) CAR-T cell proliferation assay

The UNT cells and CAR-T cells prepared from Donor 4 in Example 1 were resuscitated, and stabilized by incubation at 37°C overnight. ADI-38497 WT antibody or ADI-38497 PG antibody was diluted using PBS, added to a 96-well plate. The plate was coated by incubating overnight at 4°C. CAR-T cells recovered from overnight incubation were added to the antibody-coated 96-well plate, and used as a positive control. CD3/CD28-coupled magnetic beads were added directly to the CAR-T cells (the ratio of magnetic beads : cells was 3:1); incubated at 37°C for 72 h and 120 h, respectively, and the luminescence values were measured using CellTiter-Glo^{®} Luminescent Cell Viability Assay (Promega, G7572).

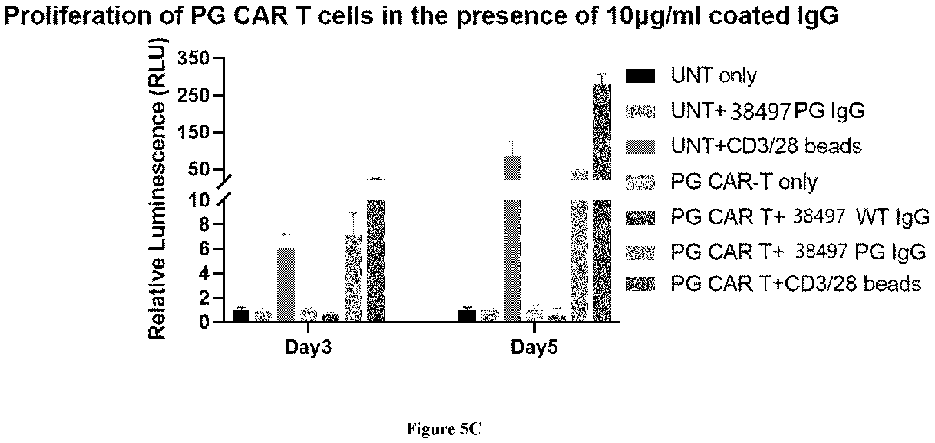

Figure 5C showed if HuR968B CAR-T cells were proliferated after stimulating with the coated ADI-38497 WT antibody or the coated ADI-38497 PG antibody. HuR968B CAR-T cells were proliferated under the coated ADI-38497 P329G antibody stimulation, compared to the coated ADI-38497 WT antibody stimulation. After 3 or 5 days of the stimulation, ADI-38497 PG antibody caused HuR968B CAR-T cells to proliferate approximately 7-fold or 43.5-fold, respectively, while UNT cells were not significantly proliferated when stimulated with ADI-38497 PG antibody, compared to stimulation with ADI-38497 WT antibody. Both HuR968B CAR -T cells and UNT cells were proliferated significantly under CD3/CD28 antibody-coupled magnetic bead stimulation.

### (5-3) Cytokine assay

CAR-T cells prepared in Example 1 were resuscitated, and stabilized by incubation at 37°C overnight. Tumor target cells and CAR-T cells were mixed in an E:T ratio of 2:1, and different concentrations of BCMA antibody (ADI-38497 PG antibody, ADI-38484 PG antibody, ADI-38497 WT antibody, or GSK PG IgG as Benchmark) diluted in 5-fold or 10-fold gradient dilutions were added to a total volume of 200 µL, incubated at 37°C for approximately 24 hours and then centrifuged to collect the supernatant. The cytokines were assayed using BD^{™} Cytometric Bead Array (CBA) Human Th1/Th2 Cytokine Kit II (BD, 551809). An equal volume of Capture Beads in the kit was mixed, and added to a plate at 25 µL/well. An equal volume of supernatant or supernatant dilution or standard was added. After mixing, 25 µL of an equal volume of human Th1/Th2 PE detection agent was added and incubated for 3 hours at room temperature and away from light. Wash buffer was used to wash twice, resuspended, and detected by flow cytometry. Cytokine concentration was calculated using the PE channel MFI value.

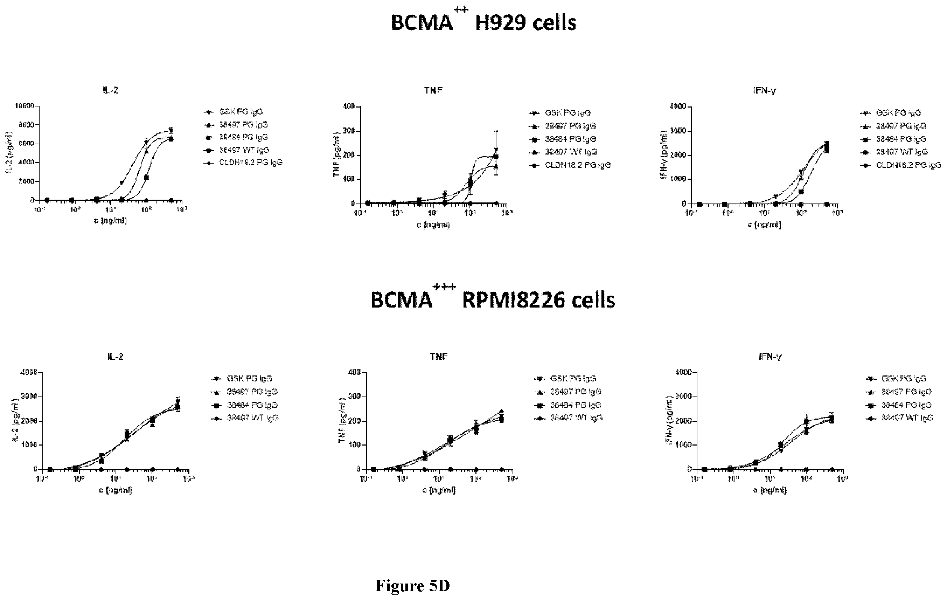

Figure 5D showed the results from effector cytokine release secreted by CAR-T cells, after co-culturing HuR968B CAR-T cells from Donor 4 of Example 1 with H929 cells, RPMI8226 cells, and adding different concentrations of BCMA antibodies (ADI-38497 PG antibody, ADI-38484 PG antibody, ADI-38497 WT antibody, or GSK PG IgG as a positive control).

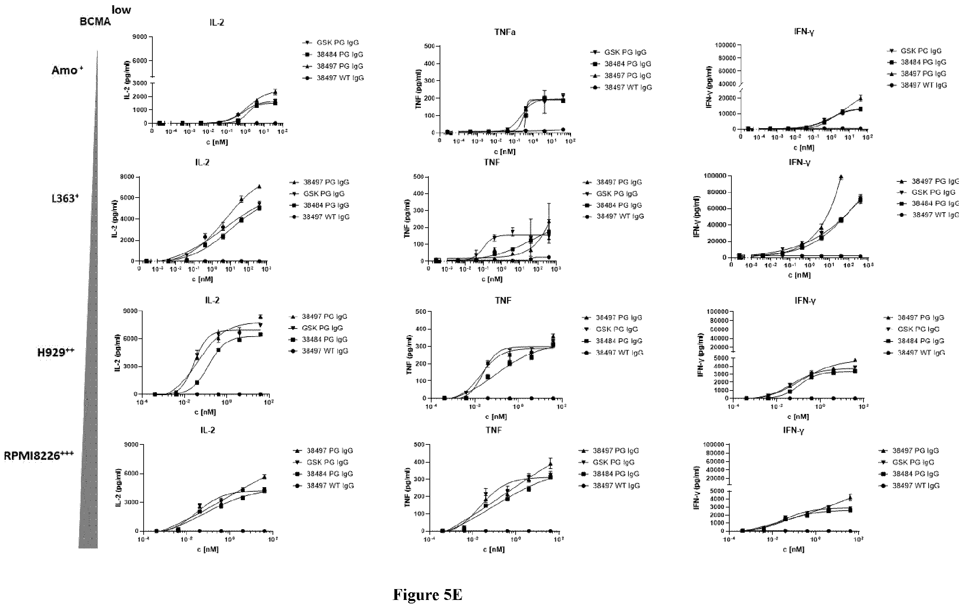

Figure 5E showed the results from effector cytokine release secreted by CAR-T cells, after co-culturing HuR968B CAR-T cells from donor 5 (purchased from ORiCELLS, Cat NO.: FPB004F-C, Lot NO.: PCH20210100004, Donor ID: Z0086) with different tumor cells, and adding different concentrations of BCMA antibodies (ADI-38497 PG antibody, ADI-38484 PG Antibody, ADI-38497 WT antibody, or Benchmark GSK PG IgG).

As seen in Figure 5D and Figure 5E, HuR968B CAR-T cells were not activated and did not secrete effector cytokines such as IL-2, IFN-γ, and TNFα under the addition of WT BCMA antibody (ADI-38497 WT antibody), while HuR968B CAR-T cells were activated and secreted effector cytokines only under the addition of BCMA antibody comprising P329G mutation (ADI-38497 PG antibody, ADI-38484 PG antibody, or GSK PG IgG as positive control). Based on the results of cytokine secretion under different target cell conditions, there was no significant correlation between the level of cytokine secretion by effector cells and the level of BCMA expression in target cells.

### (5-4) Killing efficiency detection

CAR-T cells prepared in Example 1 were resuscitated, and stabilized by incubation at 37°C overnight. Tumor target cells and CAR-T cells were mixed in an E:T ratio of 2:1, and different concentrations of BCMA antibody (ADI-38497 PG antibody, ADI-38484 PG antibody, ADI-38497 WT antibody, or Benchmark GSK PG IgG) solutions at 10-fold gradient dilutions were added to a total volume of 200 µL, incubated at 37°C for approximately 24 hours and centrifuged. The cell supernatant was transferred to a 96-well white bottom ELISA plate. The LDH values in the supernatant were measured using CytoTox 96 Non-Radioactive Cytotoxicity Assay (Promega, G1780) and Microplate Reader (Molecular Devices, SpectraMax i3x) to calculate the killing efficiency.

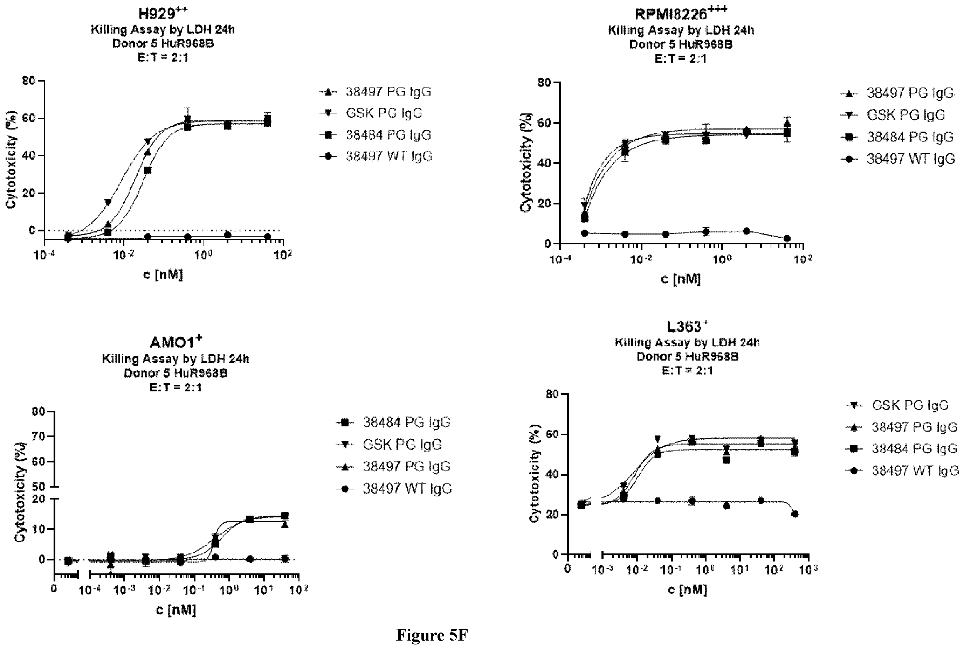

Figure 5F showed the killing effect of different BCMA antibodies (ADI-38497 PG antibody, ADI-38484 PG antibody, ADI-38497 WT antibody, or Benchmark GSK PG IgG) induced HuR968B CAR-T cells on tumor cells with different BCMA expression levels (H929⁺⁺ cells, RPMI8226⁺⁺⁺ cells, AMO1⁺ cells, and L363⁺ cells). In the case of adding WT BCMA antibody, HuR968B cells were not activated and did not lead to a killing effect on tumor cells, and only in the case of adding BCMA antibody comprising P329G mutation, P329G CAR-T cells were activated and thus killed target cells with antibody concentration dependence. The results were consistent with the above CAR-T cell activation and effector cytokine in the Example. In addition, the killing effects of P329G BCMA antibody-mediated HuR968B CAR-T cells on H929 and RPMI8226 cells as target cells with higher BCMA expression were higher than those of lower BCMA-expressing AMO1 and L363 target cells.

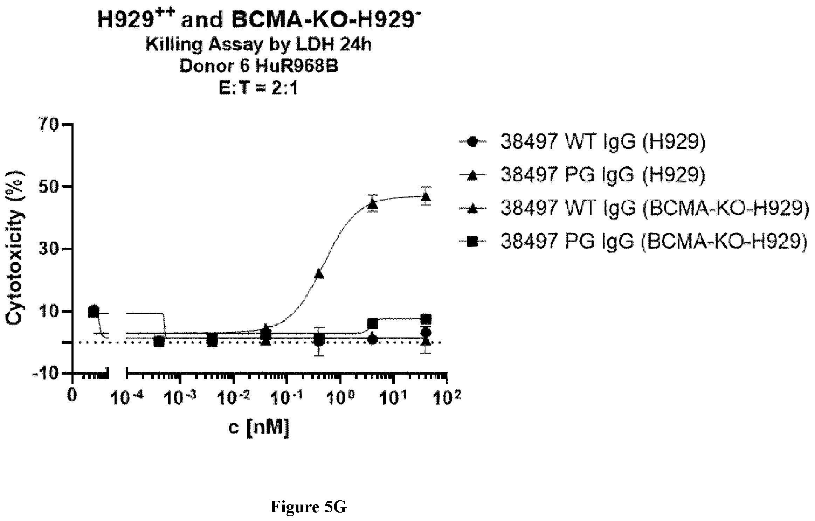

Figure 5G showed that neither P329G BCMA antibody nor WT BCMA antibody mediated the killing effect of CAR-T on BCMA-negative target cells (e.g. BCMA-KO-H929 cells), and WT BCMA antibody also failed to mediate the killing effect on BCMA-expressing H929 cells (H929). Only P329G BCMA antibody could induce the killing effect of CAR-T on tumor cells .

**Table 9. EC50 values of the killing effect of P329G BCMA antibody-mediated HuR968B CAR-T cells on H929 target cells**

| Antibody | EC50 (nM) |
|---|---|
| ADI-38497 PG | 0.3502 to 0.7433 |
| ADI-38497 WT | Very wide range |

### Example 6. Effect of free BCMA protein on PG CAR-T function

### (6-1) Killing efficiency detection

CAR-T cells prepared from donor 6 in Example 1 were resuscitated, and stabilized by incubation at 37°C overnight. Tumor target cells and CAR-T cells were mixed in an E:T ratio of 2:1, and certain concentrations of PG BCMA antibody were added. For conventional Blue21 CAR-T, no BCMA antibody was added. Different concentrations of BCMA protein solution as free BCMA protein at 10-fold gradient dilutions were added to a total volume of 200 µL, incubated at 37°C for approximately 24 hours and centrifuged. The cell supernatant was transferred to a 96-well white bottom ELISA plate. The LDH values in the supernatant were measured using CytoTox 96 Non-Radioactive Cytotoxicity Assay (Promega, G1780) and Microplate Reader (Molecular Devices, SpectraMax i3x) to calculate the killing efficiency.

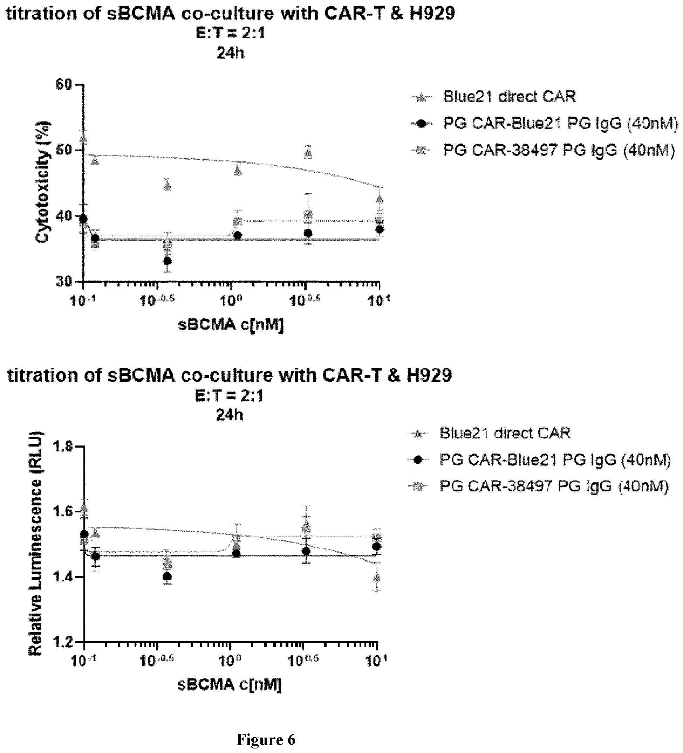

Figure 6 showed the effect of different concentrations of free BCMA protein on the killing activity of HuR968B CAR-T and Blue21 CAR-T cells. In the case of adding different concentrations of free BCMA protein, the function of conventional CAR T cells was blocked once conventional CAR T cells bound to free BCMA protein (due to the high affinity of conventional CAR molecules for BCMA), whereas PG CAR-T was still able to exert normal killing effect when PG BCMA antibody bound to soluble BCMA. Thus, the advantage of PG CAR-T cells was that the function thereof was less affected by soluble ligands (e.g. soluble BCMA).

### Example 7. Study on in vivo pharmacokinetics of ADI-38497 PG antibody

### (7-1) Antibody injection and sampling

BALB/c mice (age of 4-6 weeks, weight of 15-17g, female) were divided into three groups, i.e., 1 mg/kg of ADI-38497 PG antibody group; 10 mg/kg of ADI-38497 PG antibody group; and 200 mg/kg of ADI-38497 PG antibody group, 9 mice in each group. 1×PBS was used to dilute the antibody to 0.1 mg/mL, 1 mg/mL and 20 mg/mL respectively, and the administration volume of each mouse was 10 mL/kg, i.e., the dosage of the antibody was 1 mg/kg, 10 mg/mL, or 200 mg/mL respectively. The administration mode was intravenous injection, and the administration frequency was single. 100 µL blood samples were collected from the retroorbital venous plexus of mice 5 minutes, 30 minutes, 2 hours, 6 hours, 24 hours, 48 hours, 96 hours, 168 hours, 336 hours and 504 hours after the antibody administration, centrifugated at 3000 g, and the supernatants were taken for determination of blood drug concentration.

### (7-2) ADI-38497 PG antibody detection

A 96 well ELISA plate was coated one day in advance. BCMA antigen was diluted to 1 µg/ml using a coating solution (a bag of carbonate (Thermo, 28382) powder was dissolved in 400mL ultrapure water, then diluted to 500 mL at constant volume, and mixed well to obtain a coating solution), 100 µL per well. The plate was sealed with a plate sealing film and placed at room temperature overnight. The coating solution was removed out. The plate was patted and dried on an absorbent paper, then 300 µL washing solution was added per well, mixed under shaking for 10 seconds. After the plate was patted and dried out of washing solution, washing was repeated for 3 times. Blocking solution was added into the plate using a multichannel pipette gun, 200 µL per well. The plate was sealed with a plate sealing film and placed at room temperature for 2h. Then the plate was washed once. In standard curve (the standard curve was plotted using serially diluted BCMA antibody of known concentrations (for example, using ADI-38497 PG antibody of known concentrations to plot a standard curve) diluted, quality control samples and samples to be tested were added to the plate, 100 µL per well, incubated at room temperature for 2 h. The pre-coating solution was removed out. The plate was patted and dried on an absorbent paper, then 300 µL washing solution was added per well, mixed under shaking for 10 seconds. After the plate was patted and dried out of washing solution, washing was repeated for 3 times. Then, the washing was repeated once more. A goat anti-human IgG-Fc-HRP antibody (BETHYL) was diluted at 1: 100000, and added into each well at 100 µL, incubated at room temperature and away from light for 1h. Then the plate was washed once. TMB substrate was added to the 96-well ELISA plate at 100 µL per well. Color development was performed at room temperature and away from light for 5 minutes. 50 µL ELISA stop solution was added per well. The plate was shaked for 10 seconds, and the OD450 nm and OD620 nm values were read within 30 minutes.

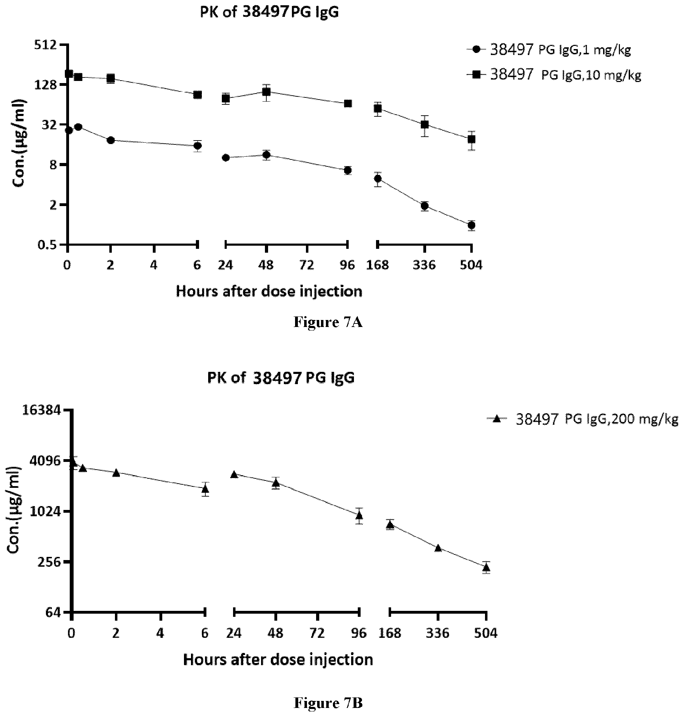

Figures 7A and 7B showed the pharmacokinetic results of ADI-38497 PG antibody in mice (hereinafter referred to as PG Ab for *in vivo* experiments in mice). After intravenous injection of ADI-38497 PG antibody at 1 mg/kg, 10 mg/kg, or 200 mg/kg, the exposure amount (Cmax and AUClast) of ADI-38497 PG antibody in serum showed a dose-dependent effect, while other pharmacokinetic parameters had no significant difference. As shown in Table 10, in 1 mg/kg of ADI-38497 PG antibody group: AUCO-inf, Cmax, CL, T1/2 were respectively 2480 µg×h/mL, 30 ug/ml, 0.40 ml/kg/h, 145 h ; in 10 mg/kg of ADI-38497 PG antibody group: AUCO-inf, Cmax, CL, T1/2 were respectively 24720 µg×h/mL, 187 ug/ml, 0.32 ml/kg/h, 219 h; in 200 mg/kg of ADI-38497 PG antibody group: AUCO-inf, Cmax, CL, T1/2 were respectively 397734 µg×h/mL, 3895 ug/ml, 0.43 ml/kg/h, 197 h. The half-life using 1 mg/kg of ADI-38497 PG antibody was slightly shorter than those using 10 mg/kg and 200 mg/kg of ADI-38497 PG antibody.

**Table 10. Pharmacokinetic test results of ADI-38497 PG antibody**

| Groups | Cₘₐₓ (µg/mL) | AUC₀₋ₜ (µg*h/mL) | AUC_{0-inf_obs} (µg*h/mL) | MRT_{0-inf_obs} (h) | t_{1/2} (h) | CL (ml/kg/h) | Vₛₛ (ml/kg) |
|---|---|---|---|---|---|---|---|
| ADI-38497 PG antibody, 1 mg/kg | 30 | 2273 | 2480 | 186 | 145 | 0.40 | 75 |
| ADI-38497 PG antibody, 10 mg/kg | 187 | 24720 | 30837 | 305 | 219 | 0.32 | 99 |
| ADI-38497 PG antibody, 200 mg/kg | 3895 | 397734 | 460828 | 229 | 197 | 0.43 | 100 |

### Example 8. Effect of PG CAR-T cells combined with different doses of PG antibody on higher BCMA-expressing tumors in vivo

### (8-1) Tumor inoculation and treatment in mice

H929 cells were suspended by using 1×PBS and prepared into a cell suspension with a cell density of 5 × 10⁶ cells/mL. NOG mice (age of 4-6 weeks, weight of 15-17g, female) were shaved on the right back, then subcutaneously injected with H929 cell suspension in a volume of 0.2 mL/mouse, i.e., the inoculation amount was 1 × 10⁶ cells/mouse. Seven days after tumor cell inoculation, mice with tumor volume of 50.82-104.36 mm³ were divided into seven groups, which were PBS vehicle group, PG Ab group, only PG CAR-T group, conventional CAR-T group, PG Ab + PG CAR-T, 3 mg/kg antibody group, PG Ab + PG CAR-T, 1 mg/kg antibody group, and PG Ab + PG CAR-T, 0.3 mg/kg antibody group, respectively. Each group consisted of 7 mice. The antibody was prepared at a concentration of 0.3 mg/mL, 0.1 mg/mL, or 0.03 mg/mL, respectively. After grouping, antibody administration was carried out on the 7th day, with a volume of 10 mL/kg per mouse. The administration frequency was once a week, and the administration pattern was intraperitoneal injection. CAR-T cells prepared from donor 4 were resuspended with 1 × PBS, and a CAR⁺ cell suspension of 25 × 10⁶ cells/mL was prepared. On the 7th day, 0.2 mL of cell suspension per mouse was injected into the tail vein, i.e., 5×10⁶ CAR⁺ cells/mouse were reinfused. The body weight of mouse, maximum long axis (L) and maximum wide axis (W) of tumor tissue were monitored twice a week.

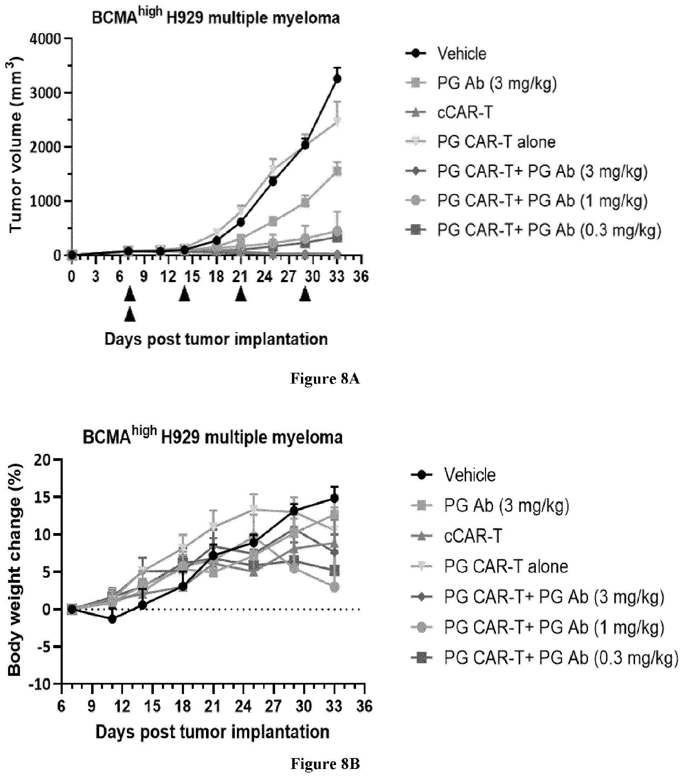

Figure 8A showed the therapeutic effects of different doses of PG antibody combined with PG CAR-T cells on immunodeficient tumor bearing mice inoculated subcutaneously with human H929 tumor cells having a higher BCMA expression. The results showed that in the high BCMA expression tumor model, administration of PG CAR-T cells alone did not lead to significant anti-tumor effect, and administration of PG antibody alone led to some anti-tumor effect; and only mice treated with both PG CAR-T cells and PG antibody led to significant anti-tumor effect and showed antibody dose-dependent effect. PG CAR-T cells combined with 0.3mg/kg antibody, 1mg/kg antibody and 3 mg/kg antibody resulted in tumor growth inhibition (TGI) of 92%, 88% and 101%, respectively, while mice treated with PG CAR-T cells only or PG antibody only resulted in anti-tumor effect TGI of 25% and 53%, respectively. In addition, PG CAR-T cells combined with 3 mg/kg of PG antibody resulted in antitumor effects comparable to those of conventional Blue21 CAR-T cells at equivalent doses of administration, with TGI of 101% and 102%, respectively.

Figure 8B showed the changes in body weight of mice in this experiment. The results showed that the body weight of mice treated with PG CAR-T cells in combination with PG antibody remained stable after treatment, with average increases of 5.2%, 3.0%, and 7.6% after treatment with the combination of PG antibody at 0.3 mg/kg, 1 mg/kg and 3 mg/kg. The results showed that PG CAR-T cells combined with PG antibody treatment resulted in significant anti-tumor effects and no significant toxic side effects.

### (8-2) In vivo CAR-T cell detection in mice.

30 µL of mouse blood samples from Example 8-1 were added to a V-shaped 96-well plate, and labeled as sample detection wells. 10 µL of mouse blood samples were added to the V-shaped 96-well plate, and labeled as control wells. 100 µL of FACS buffer comprising LIVE/DEAD Fixable Dead Cell Stain and TruStain FcX^{™} (anti-mouse CD16/32) (Biolegend) was added to each of all wells, mixed gently, incubated at 4°C for 15 min and away from light; then Biotin-F(ab')₂ fragment goat anti-human IgG antibody was added to the sample wells, incubated at 4°C for 30 min and away from light. Next, 100 µL of FACS buffer was added to each well, centrifuged at 400 g and discarded the supernatant; 100 µL of FACS buffer comprising APC-Cy7 anti-human CD45 (Biolegend), PerCP-Cy5.5-CD3 (BD Biosciences) and APC-streptavidin (Biolegend) to each well, mixed gently, and incubated at 4 °C for 30 min and away from light; then 200 µL/well of FACS buffer was added to all wells, centrifuged at 400 g, and discarded the supernatant; 250 µL/well of 1×RBC Lysis/Fixation solution (Biolegend) was added, mixed well, and incubated at room temperature for 20 min and away from light; centrifuged at 400 g and discarded the supernatant; resuspended the cells with 100 µL FACS buffer, then 10 µL of 123count ebeads was added per well; and detection was performed using flow cytometry.

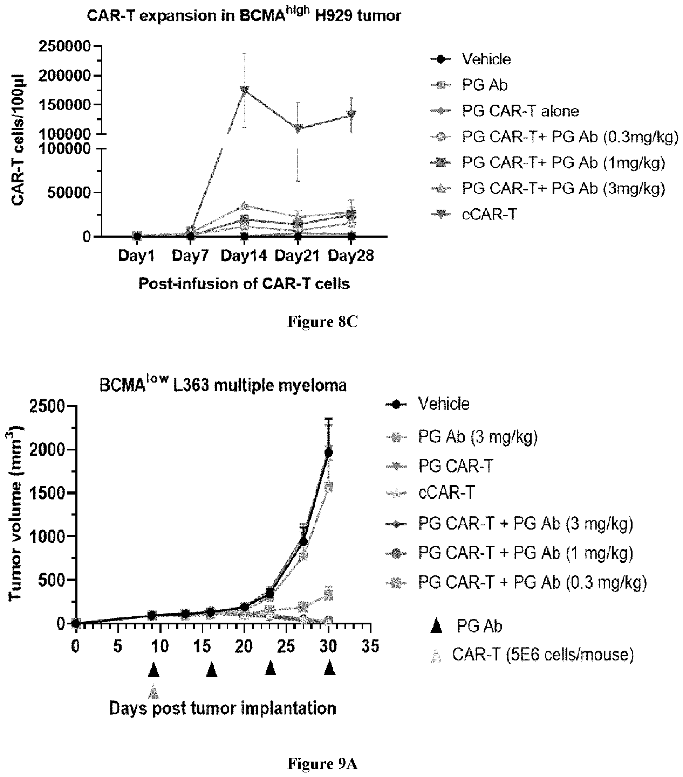

Figure 8C showed the in vivo expansion of PG CAR-T cells in mice in the experiment of Example 8-1. The results showed that PG CAR-T cell expansion in vivo was dependent on PG antibody, showing an antibody dose dependence, with higher levels of PG CAR-T cell expansion in mice in the higher antibody dose group. When PG CAR-T cells were administered only, they started to expand at week 1 in the reinfused mice, then expanded to 466 cells/100 µL peripheral blood after 2 weeks, reached a high level (3644 cells/100 µL peripheral blood) after 3 weeks, and remained at a high level (3214 cells/100 µL peripheral blood) after 4 weeks. When PG CAR-T cells were administered in combination with different doses, i.e. 0.3 mg/kg, 1 mg/kg, 3 mg/kg, of PG antibody, the peak expansion levels of PG CAR-T cells respectively reached 11428 cells/100 µL peripheral blood, 19299 cells/100 µL peripheral blood, and 35368 cells/100 µL peripheral blood after 2 weeks, and remained at high levels after 4 weeks, at 15486 cells/100 µL peripheral blood, 25073 cells/100 µL peripheral blood, and 27666 cells/100 µL peripheral blood respectively, which were much higher than those in the groups without co-administered antibody during the same period. In addition, conventional Blue21 CAR-T cells, which were used as positive controls, showed similar expansion kinetics, also starting to expand at week 1 and reaching a peak level (174769 cells/100 µL peripheral blood) after 2 weeks, and remaining at a very high level (131963 cells/100 µL peripheral blood) after 4 weeks.

### Example 9. Effect of PG CAR-T cells combined with different doses of PG antibody on lower BCMA-expressing tumors in vivo

### (9-1) Tumor inoculation and treatment in mice

L363 cells were suspended by using 1×PBS and prepared into a cell suspension with a cell density of 5 × 10⁶ cells/mL. NOG mice (age of 4-6 weeks, weight of 15-17g, female) were shaved on the right back, then subcutaneously injected with L363 cell suspension in a volume of 0.2 mL/mouse, i.e., the inoculation amount was 1 × 10⁶ cells/mouse. Nine days after tumor cell inoculation, mice with tumor volume of 74.14-110.29 mm³ were divided into seven groups, which were vehicle group, PG Ab group, PG CAR-T group, conventional CAR-T group, PG Ab + PG CAR-T, 3 mg/kg antibody group, PG Ab + PG CAR-T, 1 mg/kg antibody group, and PG Ab + PG CAR-T, 0.3 mg/kg antibody group, respectively. Each group consisted of 7 mice. The antibody was prepared at a concentration of 0.3 mg/mL, 0.1 mg/mL, or 0.03 mg/mL, respectively. After grouping, antibody administration was carried out on the 9th day, with a volume of 10 mL/kg per mouse. The administration frequency was once a week, and the administration pattern was intraperitoneal injection. CAR-T cells prepared from donor 4 were resuspended with 1 × PBS, and a CAR⁺ cell suspension of 25 × 10⁶ cells/mL was prepared. On the 9th day, 0.2 mL of cell suspension per mouse was injected into the tail vein, i.e., 5×10⁶ CAR⁺ cells/mouse were reinfused. The body weight of mouse, maximum long axis (L) and maximum wide axis (W) of tumor tissue were monitored twice a week.

Figure 9A showed the therapeutic effects of different doses of PG antibody combined with PG CAR-T cells on immunodeficient tumor bearing mice inoculated subcutaneously with human L363 tumor cells having a lower BCMA expression. The results showed that in the low BCMA expression tumor model, administration of PG CAR-T alone did not lead to an antitumor effect, while administration of PG antibody alone had a non-significant anti-tumor effect, with a TGI of 21%. Only mice treated with both PG CAR-T cells and PG antibody led to a significant anti-tumor effect and showed antibody dose-dependent effect. When PG CAR-T cells were combined with a dose of 0.3 mg/kg PG antibody, PG CAR-T cells induced a significant anti-tumor effect, with a TGI of 87%. When the combined dose of PG antibody was increased to 1 mg/kg and 3 mg/kg, the maximum anti-tumor effects induced by PG CAR-T cells were significantly increased, with TGI of 103% and 103%, respectively, showing the same anti-tumor effect as that of conventional Blue21 CAR-T cells.

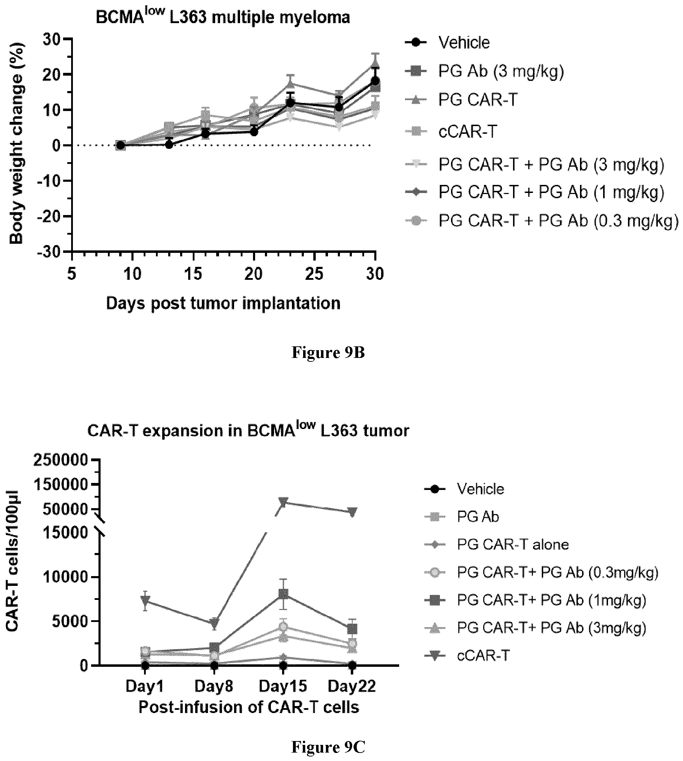

Figure 9B showed the changes in body weight of mice in the experiment. The results showed that the body weight of mice treated with PG CAR-T cells in combination with PG antibody maintained a steady increase after treatment, with an average increase of 18.2%, 10.5% and 8.5% respectively after the combination of PG antibody at 0.3 mg/kg, 1 mg/kg and 3 mg/kg. The results showed that PG CAR-T cells combined with PG antibody treatment led to a significant anti-tumor effect and did not induce any significant toxicity.

### (9-2) In vivo CAR-T cell detection in mice: Methods were the same as those of Example 8-2.

Figure 9C showed the in vivo expansion of PG CAR-T cells in mice in the experiment of Example 9-1. The results showed that when administered PG CAR-T cells only, the PG CAR-T cells started to expand at week 1 in the reinfused mice, expanded to 919 cells/100 µL peripheral blood after 2 weeks, and rapidly decreased to 204 cells/100 µL peripheral blood at week 3. When PG CAR-T cells were administered in combination with different doses of PG antibody at 0.3 mg/kg, 1 mg/kg and 3 mg/kg, the peak expansion levels in vivo reached 4380 cells/100 µL peripheral blood, 8049 cells/100 µL peripheral blood, and 3347 cells/100 µL peripheral blood after 2 weeks, and remained high after 3 weeks, and at 2475 cells/100 µL peripheral blood, 4121 cells/100 µL peripheral blood, and 1969 cells/100 µL peripheral blood, respectively, which were much higher than those in the group without co-administration of the antibody during the same period. In addition, the conventional Blue21 CAR-T cells, which served as a positive control, also reached a peak level of expansion in the reinfused mice (76836 cells/100 µL peripheral blood) after 2 weeks and remained at a high level (36328 cells/100 µL peripheral blood) after 3 weeks.

### Example 10. Anti-tumor effect of different doses of PG CAR-T cells combined with PG antibody in vivo

### (10-1) Tumor inoculation and treatment in mice

H929 cells were suspended by using 1×PBS and prepared into a cell suspension with a cell density of 5 × 10⁶ cells/mL. NOG mice (age of 4-6 weeks, weight of 15-17g, female) were shaved on the right back, then subcutaneously injected with H929 cell suspension in a volume of 0.2 mL/mouse, i.e., the inoculation amount was 1 × 10⁶ cells/mouse. Nine days after tumor cell inoculation, mice with tumor volume of 59.50-105.82 mm³ were divided into seven groups, which were vehicle group, PG Ab group, only PG CAR-T group, PG Ab + PG CAR-T, 10×10⁶ cells group, PG Ab + PG CAR-T, 1×10⁶ cells group, PG Ab + PG CAR-T, 0.1×10⁶ cells group, and PG Ab + PG CAR-T, 0.01×10⁶ cells group. Each group consisted of 7 mice. The antibody was prepared at a concentration of 0.3 mg/mL. After grouping, antibody administration was carried out on the 9th day, with a volume of 10 mL/kg per mouse. The administration frequency was once a week, and the administration pattern was intraperitoneal injection. CAR-T cells prepared from donor 4 were resuspended with 1 × PBS, and a CAR⁺ cell suspension of 50 × 10⁶ cells/mL was prepared. Then, 10-fold gradient dilutions were made to obtain cell suspensions of 5×10⁶, 0.5×10⁶ and 0.05×10⁶ cells/mL. On the 9th day, 0.2 mL of cell suspension per mouse was injected into the tail vein. The body weight of mouse, maximum long axis (L) and maximum wide axis (W) of tumor tissue were monitored twice a week.

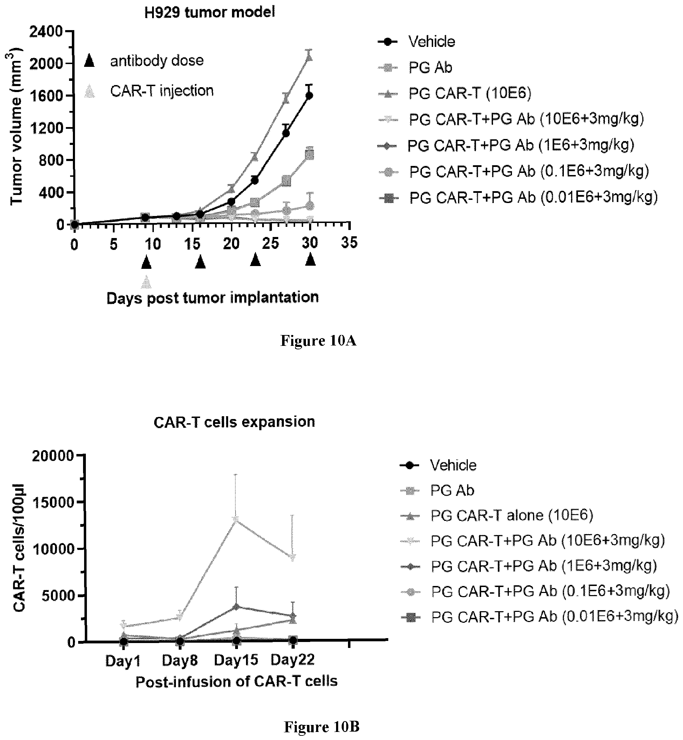

Figure 10A showed the therapeutic effects of PG antibody combined with different doses of PG CAR-T cells on immunodeficient tumor bearing mice inoculated subcutaneously with human H929 tumor cells. The results showed that at a very low dose of 0.01×10⁶ CAR-T cells, CAR-T cells in the combination produced similar anti-tumor effects to those of only PG antibody administration, with TGI of 49% and 50%, respectively. Increasing the CAR-T cell doses to 0.1×10⁶, 1×10⁶, and 10×10⁶ CAR-T cells significantly increased the anti-tumor effects induced by PG CAR-T cells in the combination, with TGI of 91%, 104%, and 103%, respectively. Administration of CAR-T cells only did not show anti-tumor effect.

### (10-2) In vivo CAR-T cell detection: Methods were the same as those of Example 8-2.

Figure 10B showed the expansion of PG CAR-T cells in mice in the experiments of Example 10-1. The results showed that CAR-T cells reinfused into mice started to expand at week 1 under PG antibody induction, reached peak levels of expansion after 2 weeks, and remained at high levels after 3 weeks. In addition, when combined with PG antibody, CAR-T cell expansion in vivo was dependent on CAR-T cell dose, and mice in higher CAR-T dose group had a higher level of CAR-T cell expansion. 0.01×10⁶, 0.1x 10⁶, 1×10⁶, and 10×10⁶ CAR-T cell dose groups had peak expansion levels of 6 cells/100 µL peripheral blood, 338 cells/100 µL peripheral blood, 3640 cells/100 µL peripheral blood, and 12895 cells/100 µL peripheral blood, respectively.

### Example 11. Study of in vivo anti-tumor effect of PG CAR-T cells combined with PG antibody at different dosing frequencies

### (11-1) Tumor inoculation and treatment in mice

H929 cells were suspended by using 1×PBS and prepared into a cell suspension with a cell density of 5 × 10⁶ cells/mL. NOG mice (age of 4-6 weeks, weight of 15-17g, female) were shaved on the right back, then subcutaneously injected with H929 cell suspension in a volume of 0.2 mL/mouse, i.e., the inoculation amount was 1 × 10⁶ cells/mouse. Seven days after tumor cell inoculation, mice with tumor volume of 51.25-94.97 mm³ were divided into seven groups, which were vehicle group, only PG CAR-T group, PG Ab + PG CAR-T, Q3/4×2 group, PG Ab + PG CAR-T, QW×4 group, PG Ab + PG CAR-T, Q2W×2 group, PG Ab + PG CAR-T, Q3W×2 group, and conventional CAR-T group. Each group consisted of 7 mice. The antibody was prepared at a concentration of 0.1 mg/mL. After grouping, antibody administration was carried out, with a volume of 10 mL/kg per mouse. The administration pattern was intraperitoneal injection. CAR-T cells prepared from donor 4 were resuspended with 1 × PBS, and a CAR⁺ cell suspension of 10 × 10⁶ cells/mL was prepared, and 0.2 mL of cell suspension per mouse was injected into the tail vein. The body weight of mouse, maximum long axis (L) and maximum wide axis (W) of tumor tissue were monitored twice a week.

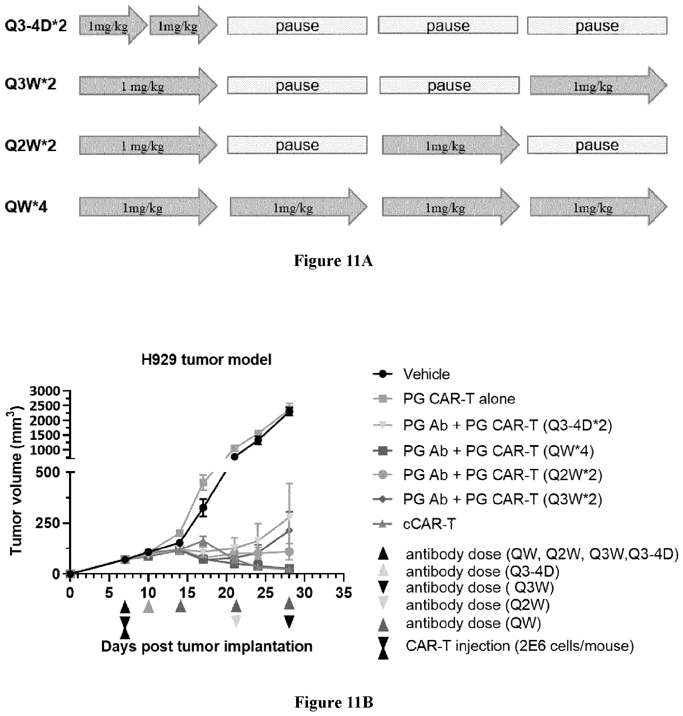

Figure 11A showed the administration frequency of PG antibody in the experiment of Example 11-1. Q3-4D×2: PG antibody was administered twice in week 1 with a dosing interval of 3-4 days on a 4-week cycle; Q3W×2: PG antibody was administered twice with a dosing frequency of 3 weeks/dose; Q2W×2: PG antibody was administered twice with a dosing frequency of 2 weeks/dose; QW×4: PG antibody was administered 4 times with a dosing frequency of 1 week/dose.

Figure 11B showed the therapeutic effects of different dosing frequencies of PG antibody in combination with PG CAR-T cells on immunodeficient tumor-bearing mice inoculated subcutaneously with human H929 tumor cells. The results showed that when combined with different dosing frequencies of PG antibody, PG CAR-T cells could induce significant antitumor effects, and the antitumor effects induced by PG CAR-T cells were significantly increased by increasing the dosing frequency of PG antibody. The most significant antitumor effect induced by CAR-T cells was in the QW×4 group, which showed the same antitumor effect as that of conventional Blue21 CAR-T cells.

### (11-2) In vivo CAR-T cell detection: Methods were the same as those of Example 8-2.

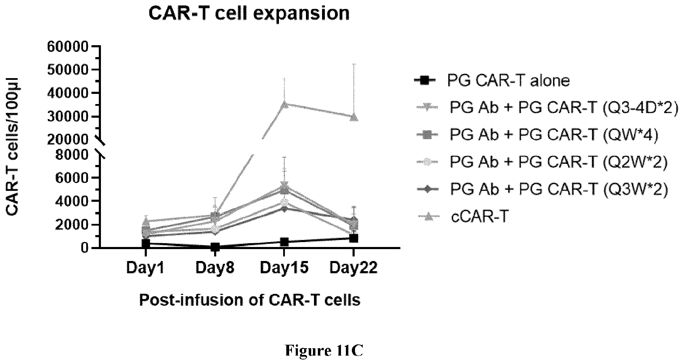

Figure 11C showed the expansion of PG CAR-T cells in mice in the experiment of Example 11-1. The results showed that when PG CAR-T cells were administered in combination with PG antibody administered at different frequencies, the kinetics of CAR-T cell expansion were similar, with CAR-T cells starting to expand at week1 in the CAR-T cell reinfused mice, reaching peak levels after 2 weeks, and decreasing to baseline levels after 3 weeks. In addition, conventional Blue21 CAR-T cells also reached peak expansion after 2 weeks in the conventional Blue21 CAR-T cell reinfused mice and remained at high levels after 3 weeks.

### Example 12. In vivo anti-systemic-tumor effect study of PG CAR-T cells combined with different doses of PG antibody

### (12-1) Tumor inoculation and treatment in mice

First, H929-luc cells were prepared. Particularly, H929 cells (purchased from Nanjing Cobioer Biosciences Co., Ltd.) were used to package lentiviruses comprising GFP-luciferase gene, then the obtained lentiviruses were used to infect H929 cells. Subsequently, H929-luc cell line with dual expression of GFP and luciferase was obtained through flow cytometry sorting.

Next, H929-luc cells were resuspended by using 1 × PBS and prepared into a cell suspension with a cell density of 25 × 10⁶ cells/mL. NOG mice (age of 4-6 weeks, weight of 15-17g, female) were injected with H929-luc cell suspension via tail vein, in an injection volume of 0.2 mL per mouse. Fourteen days after tumor cells were inoculated, the substrate D-Luciferin (15 mg/mL) was intraperitoneally injected in a volume of 10 mL/kg/mouse. 10 minutes after the substrate injection, IVIS Spectrum was used for imaging analysis. Mice with the fluorescent signal at 1.17 × 10⁷ - 1.43 × 10⁸ photons/sec were divided into 7 groups, which were vehicle group, PG Ab, 0.3 mg/kg group, PG Ab, 3 mg/kg group, PG CAR-T group, PG Ab + PG CAR-T, 0.3 mg/kg + 2 × 10⁶ group, PG Ab + PG CAR-T, 3 mg/kg + 2 × 10⁶ group, and Blue21 CAR-T group, respectively. 6-7 mice in each group. Antibody concentrations of 0.03 mg/mL and 0.3 mg/mL were prepared respectively. After grouping, antibody administration was started on the 14th day. The administration volume of each mouse was 10 mL/kg, the administration frequency was once a week, and the administration mode was intraperitoneal injection. The CAR-T cells prepared from donor 4 were resuspended with 1×PBS to prepare 10×10⁶ CAR⁺ cells/mL cell suspension, and 0.2 mL of cell suspension/mouse was injected into the tail vein on day 7.

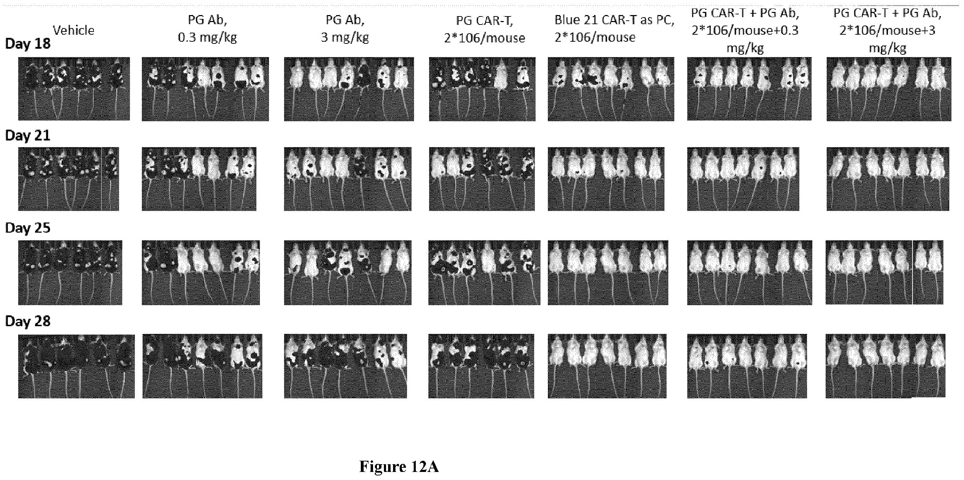

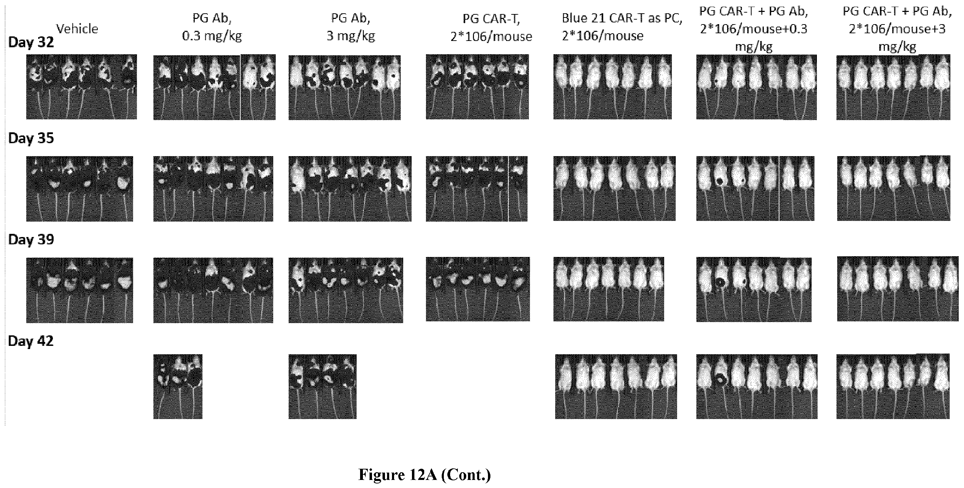

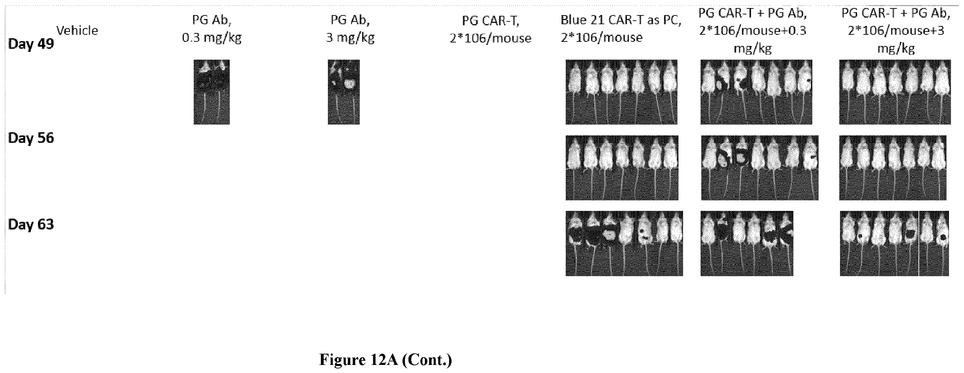

Figure 12A showed the fluorescence images of the therapeutic effects of different doses of PG antibody combined with PG CAR-T cells on immunodeficient tumor bearing mice inoculated with human H929-luc tumor cells via the tail vein. The results showed that in the systemic tumor model, PG CAR-T cells combined with PG antibody also led to significant anti-tumor effects. On day 18, the fluorescence distribution and fluorescence intensity of mice in the PG CAR-T cells combined with PG antibody 0.3 mg/kg group were significantly reduced compared with the control group, and mice in the group given PG antibody 3 mg/kg combined with PG CAR-T cells had no significant fluorescence signal. However, the conventional Blue21 CAR-T treatment group still had a large amount of fluorescence distribution, indicating that the combined PG CAR-T cell groups induced anti-tumor effects faster than Blue21 CAR-T; The mice in the group treated with PG antibody 3mg/kg combined with PG CAR-T cells showed no significant fluorescence signal until day 56, and showed slight fluorescence signals from tumors in a subset of the mice on day 63, while at this time, more than half of the mice in the Blue21 CAR-T treatment group showed significant tumor recurrence and metastasized to the peritoneal cavity, indicating that the combination of PG CAR-T cells with PG antibody led to a more durable anti-tumor effect.

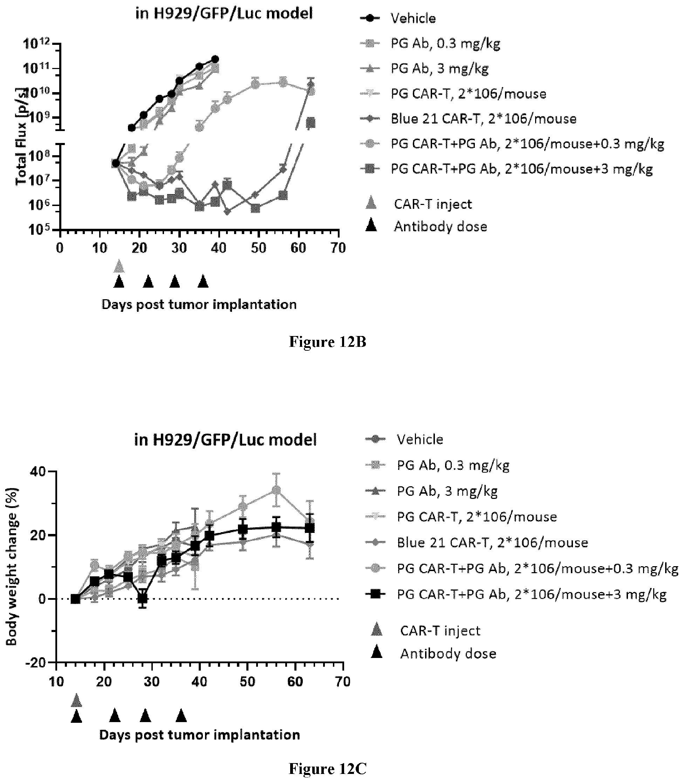

Figure 12B showed the therapeutic effects of different doses of PG antibody combined with PG CAR-T cells on immunodeficient tumor bearing mice inoculated with human H929-luc tumor cells via the tail vein. The results showed that PG CAR-T cells led to some anti-tumor effect when combined with 0.3 mg/kg of PG antibody, and had the anti-tumor effect increased significantly and maintained longer when increasing the combined PG antibody dose to 3 mg/kg; and had the tumor growth started to recur around 4 weeks after stopping the administration of PG antibody, while the tumor recurrence was faster when the same cell dose of conventional Blue21 CAR-T was administered, showing that PG CAR-T cells had a longer-lasting anti-tumor effect than conventional Blue21 CAR-T cells. The PG CAR-T cell group and the PG antibody group did not show any significant anti-tumor effect.

Figure 12C showed the changes in body weight of mice in the above experiments. The results showed that the body weight of mice in each treatment group increased steadily during the treatment period, suggesting that the treatment with different doses of PG antibody combined with PG CAR-T cells did not induce significant toxicity in the hematological tumor model.

### Example 13. In vivo toxicology study of PG CAR-T cells combined with PG antibody

### (13-1) Tumor inoculation and treatment in mice

H929 cells were suspended by using 1×PBS and prepared into a cell suspension with a cell density of 5 × 10⁶ cells/mL. NOG mice (age of 4-6 weeks, weight of 15-17g, female) were shaved on the right back, then subcutaneously injected with H929 cell suspension in a volume of 0.2 mL/mouse. Six days after tumor cell inoculation, mice with tumor volume of 38.49-104.77 mm³ were divided into eight groups as shown in Table 11, namely, the tumor-free vehicle group, the tumor-free PG Ab + PG CAR-T, 10 mg/kg + 10×10⁶ group, and the following tumor-bearing groups: vehicle group, PG CAR-T group, PG Ab group, PG Ab + PG CAR-T, 10 mg/kg + 10×10⁶ group, PG Ab + PG CAR-T, 3 mg/kg + 10×10⁶ group, and PG Ab + PG CAR-T, 3 mg/kg + 1×10⁶ group, 24 mice in each group. The antibody was prepared at a concentration of 1 mg/mL, or 0.3 mg/mL, respectively. After grouping, antibody administration was carried out, with a volume of 10 mL/kg per mouse. The administration frequency was once a week, the administrations were 3 times, and the administration pattern was intraperitoneal injection. One day after the first antibody administration, CAR-T cells prepared from donor 4 were resuspended with 1×PBS, and cell suspensions of 50×10⁶ CAR⁺ cells/mL and 5×10⁶ CAR⁺ cells/mL were prepared, respectively, and 0.2 mL of cell suspension per mouse was injected into the tail vein. The body weight of mouse, maximum long axis (L) and maximum wide axis (W) of tumor tissue were monitored twice a week. Peripheral bloods were collected before the 1st antibody administration, after CAR-T reinfusion, before the 3rd antibody administration, and at the endpoint of the experiment, using 4 mice in each group, and the collected bloods were used for hematological and blood biochemical assays.

**Table 11. Treatment and dose administered in each group**

| Group | Cell line | Treatment | Concentration |
|---|---|---|---|
| 1 | tumor-free | Vehicle | N/A |
| 2 | tumor-free | PG Ab + PG CAR-T | 10 mg/kg (QW×3) + 10 × 10⁶ cells/mouse (SD) |
| 3 | H929 | Vehicle | N/A |
| 4 | H929 | PGAb | 10 mg/kg (QW×3) |
| 5 | H929 | PG CAR-T | 10 × 10⁶ cells/mouse (SD) |
| 6 | H929 | PG Ab + PG CAR-T | 10 mg/kg (QW×3) + 10 × 10⁶ cells/mouse (SD) |
| 7 | H929 | PG Ab + PG CAR-T | 3 mg/kg (QW×3) + 10 × 10⁶ cells/mouse (SD) |
| 8 | H929 | PG Ab + PG CAR-T | 3 mg/kg (QW×3) + 1 × 10⁶ cells/mouse (SD) |

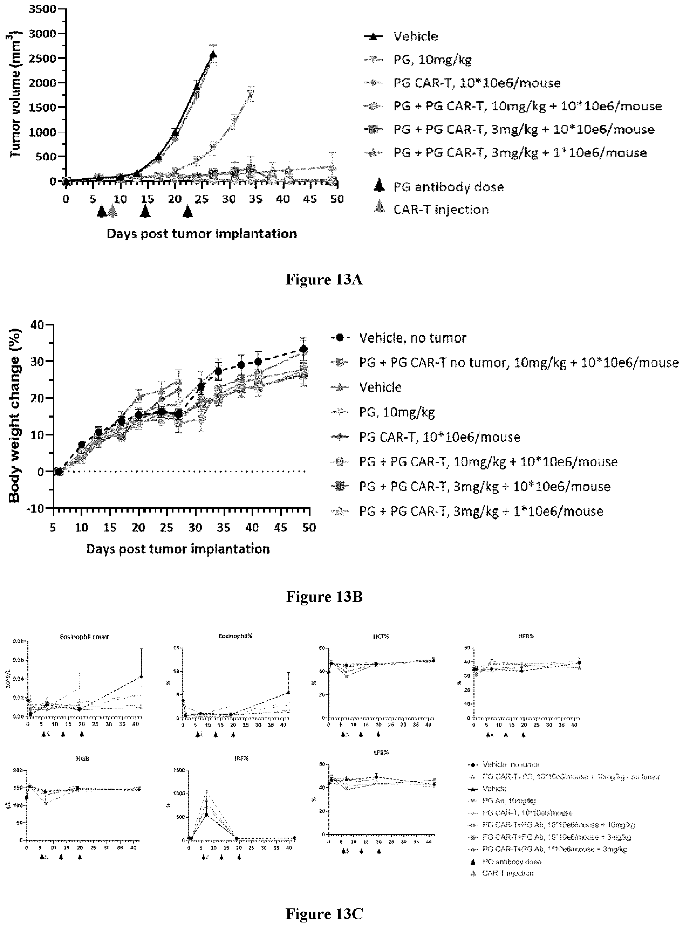

Figure 13A showed the therapeutic effect of PG antibody combined with PG CAR-T cells on immunodeficient tumor-bearing mice inoculated subcutaneously with human H929 tumor cells. The results showed that PG antibody combined with PG CAR-T cells led to significant anti-tumor effects in all treated groups, while the anti-tumor effects were weaker in the mice treated with PG antibody only and no anti-tumor effect was obtained in the mice treated with PG CAR-T only.

Figure 13B showed the changes in body weight of mice in the experiment. The results showed that the body weight of the mice in the tumor-bearing and tumor-free treatment groups increased steadily during the treatment period, and there were no significant changes in body weights compared with the control mice, suggesting that the treatment with different doses of PG antibody combined with different doses of PG CAR-T cells did not induce significant toxic responses.

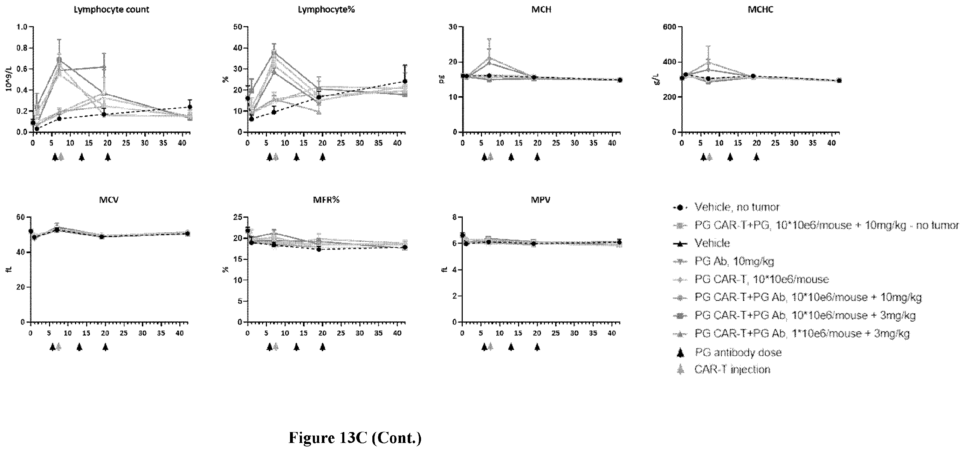

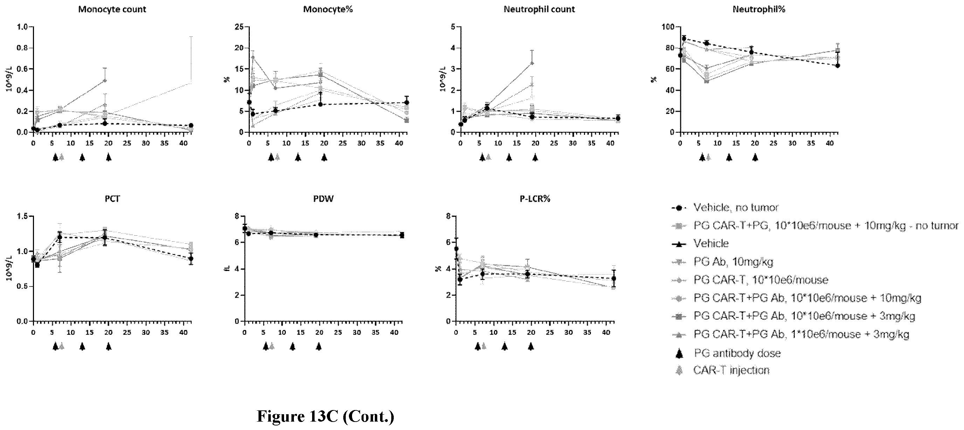

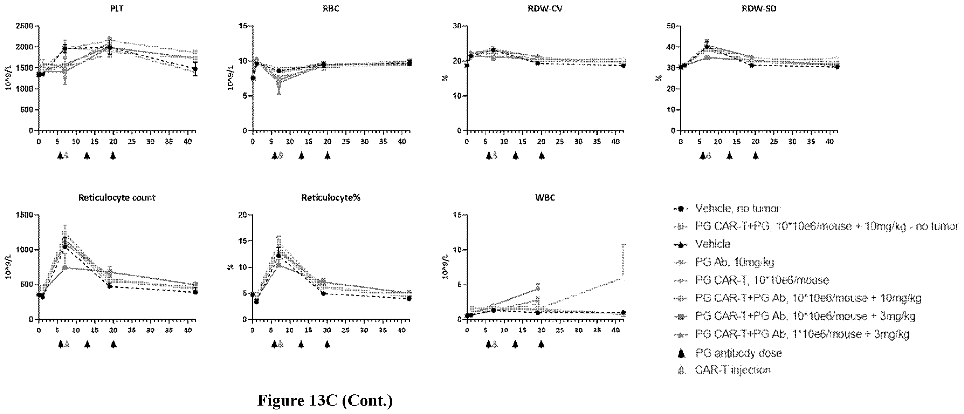

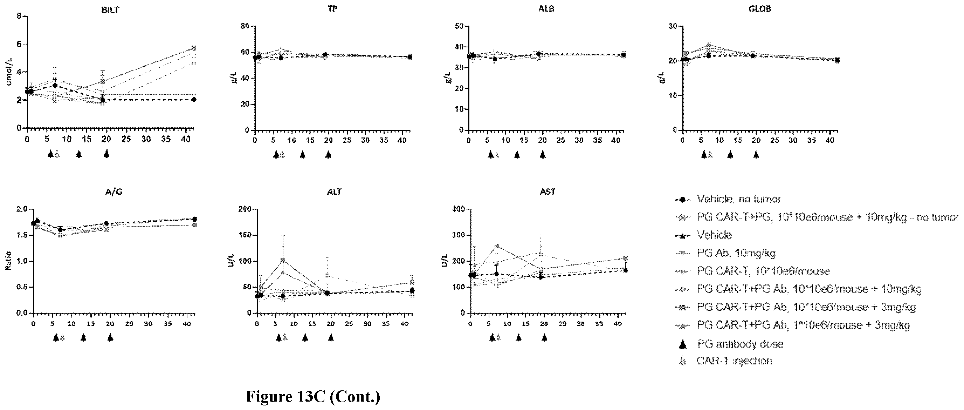

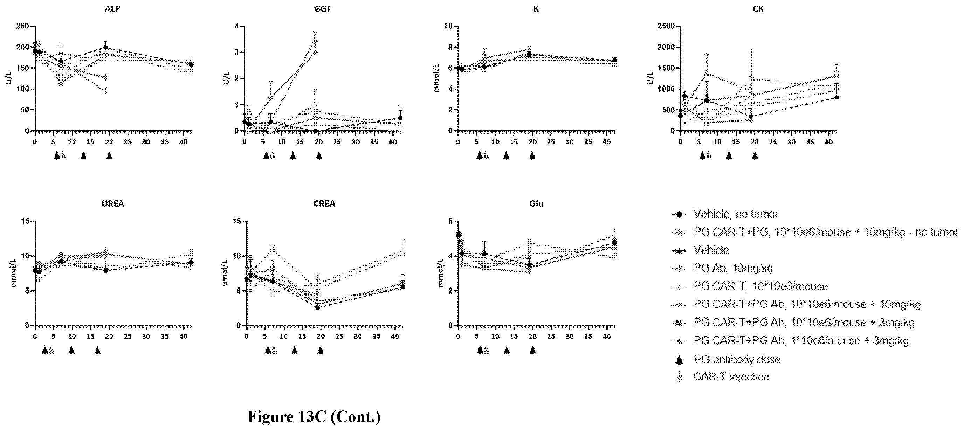

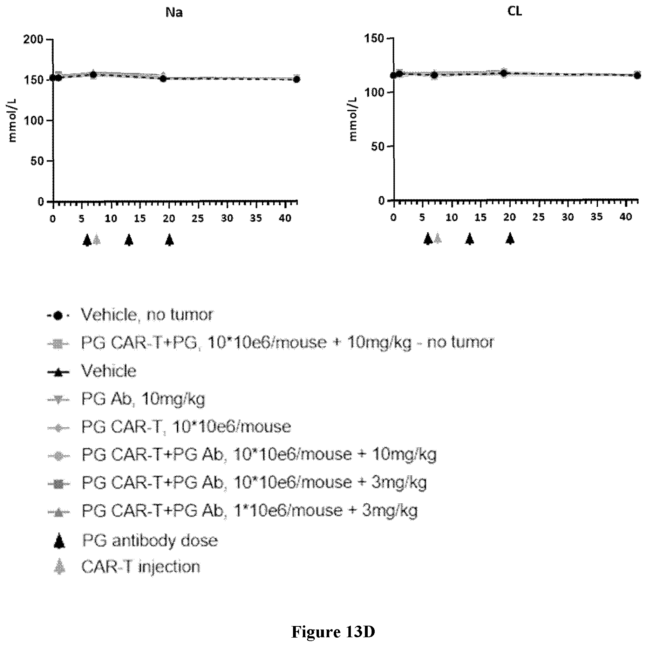

Figure 13C and Figure 13D showed the results of hematological and blood biochemical assays in mice in the above experiments. The results showed that the hematological and blood biochemical parameters of the mice in the tumor-bearing and tumor-free treatment groups did not change significantly during the treatment period compared with the control mice, indicating that PG antibody combined with PG CAR-T cell treatment did not lead to toxic responses.

### Example 14. Clinical study

### 1. Clinical study objective

1) Evaluation of the safety and tolerability of "PG 38497 antibody + PG CAR-T cells" infusion in the treatment of relapsed/refractory multiple myeloma (RRMM).
2) Evaluation of the clinical efficacy of "PG 38497 antibody + PG CAR-T cells" infusion in the treatment of RRMM.
3) Evaluation of the pharmacokinetic (PK) and pharmacodynamic (PD) characteristics of "PG 38497 antibody + PG CAR-T cells" after administration
4) Evaluation of the immunogenicity of "PG 38497 antibody + PG CAR-T cells" after administration.

### 2. Tested drug

The active components of the tested drug in the Example were P329G BCMA antibody (ADI-38497 PG antibody) and P329G CAR-T cells (obtained from modified patient's autologous PBMC cells).

The formulation of P329G BCMA antibody was 20.0 mg/ml P329G BCMA antibody, 0.76 mg/ml histidine, 1.08 mg/ml histidine hydrochloride, 50.00 mg/ml sorbitol, 0.20 mg/ml polysorbate 80, pH 6.0. Format of P329G BCMA antibody: 60 mg (3 mL)/vial.

Format of the P329G CAR-T cells: Each package contains 90-140 × 10⁶ anti-P329G CAR-T positive cells dissolved in 7.5% DMSO cryoprotectant.

The manufacturer of P329G BCMA antibody was INNOVENT BIOPHARMACEUTICALS (SU ZHOU) CO., LTD., and the manufacturer of the P329G CAR-T cells was INNOVENT CELLS PHARMACEUTICALS (SU ZHOU) CO., LTD.

### 3. Inclusion criteria

1) 18 years old and above, gender was not limited.
2) According to the International Myeloma Working Group (IMWG) diagnostic criteria for multiple myeloma, with supporting information from the examination for the initial diagnosis of multiple myeloma.
3) Previously treated with at least 3 lines of therapy, with at least one full treatment cycle per line (unless best outcome was documented as progressive disease (PD), according to IMWG criteria); and should include proteasome inhibitors and immunomodulators.
4) Progression of disease during the most recent anti-myeloma treatment or within 12 months after the treatment, as evidenced by examination data.
5) The presence of a measurable lesion at the time of screening, which was determined according to any of the following criteria.
   ① Proportion of monoclonal plasma cells ≥ 5% by bone marrow cytology, bone marrow biopsy tissue or flow cytometry assays.
   ② Serum monoclonal protein (M-protein) level ≥0.5 g/dL.
   ③ Urinary M protein level ≥200 mg/24 hours.
   ④ Multiple myeloma of light chain type with no measurable lesions in serum or urine: serum immunoglobulin free light chain ≥ 10 mg/dL and abnormal serum immunoglobulin κ/λ free light chain ratio.
6) ECOG score of 0 or 1.
7) Expected survival time ≥ 12 weeks.
8) Subjects should possess appropriate organ function and meet all of the following laboratory test results prior to enrollment.
   ① Blood routine: absolute neutrophil count (ANC) >_ 1×10⁹ /L; absolute lymphocyte count (ALC) >_ 0.3×10⁹ /L; blood platelets ≥ 50×10⁹ /L; hemoglobin ≥ 60 g/L.
   ② Liver function: ALT and AST ≤ 2.5 × upper limit of normal (ULN); serum total bilirubin ≤1.5 × ULN.
   ③ Renal function: serum creatinine <2.5×ULN; or creatinine clearance (CrCl) ≥ 40 ml/min, calculated according to the Cockcroft-Gault formula.
   ④ Coagulation function: Fibrinogen ≥1.0 g/L; activated partial thromboplastin time, activated partial thromboplastin time ≤1.5× ULN, prothrombin time ≤1.5× ULN.
   ⑤ Resting arterial oxygen saturation >91%.
   ⑥ Left ventricular ejection fraction (LVEF) ≥50 %.
9) Subjects and their spouses agreed to take effective tools or medication contraception measures (excluding safe period contraception) within one year from the signing of the informed consent form (ICF) to the last administration of "P329G BCMA antibody + PG CAR-T cells" treatment (including any component during the treatment).
10) Subjects should sign an ICF indicating that they understood the purpose and procedures of the study and that they were willing to participate in the study. Informed consent should be obtained prior to starting any test or procedure that was relevant to the study but was not part of the standard treatment of the subject's disease.

### 4. Clinical programs

The study was a clinical study to evaluate the safety, tolerability, pharmacokinetics and preliminary efficacy of the infusion of "P329G BCMA and P329G CAR-T cells" in the treatment of RRMM subjects.

Enrolled subjects would receive peripheral blood mononuclear cell (PBMC) apheresis (Day -28 to Day -21). Then, P329G CAR-T cell preparation (hereinafter referred to as PG CAR-T cells) was prepared using the subject's autologous T cells. After successful preparation of PG CAR-T cells, subjects would first receive a clearing lymphatic pretreatment regimen using cyclophosphamide and fludarabine (Day -5 to Day -3). One day after the completion of the pretreatment and rest assessment, one dose of P329G BCMA antibody (hereinafter briefly referred to as ADI-38497 PG antibody) was infused, followed by PG CAR-T cell infusion two days later (Day 0). Starting from the day of PG CAR-T cell infusion, ADI-38497 PG antibody was administered periodically every 21 days, up to a maximum of 24 months, until disease progression, intolerable toxicity, subject withdrawal of informed consent, or other reasons for discontinuing study treatment (whichever occurs first).

The study was divided into two parts: dose escalation and dose extension.
1) In the dose-escalation phase, the classical "3+3" approach was used to fix the dose of PG CAR-T cells and fully explore the optimal dose of ADI-38497 PG antibody. ADI-38497 PG antibody was administered in 3 dose groups: 0.3 mg/kg, 1 mg/kg, and 3 mg/kg. The PG CAR-T cell dose was 2*10⁶ cells/kg. The DLT observation window was 21 days after the first dose of ADI-38497 PG antibody, and during the DLT observation period, 1 dose of PG CAR-T cells was administered.
2) PG CAR-T cells were fixed, and ADI-38497 PG antibody was subjected to dose escalation. Three subjects were first enrolled in each dose group. If DLT was not observed in the DLT observation window in the first 3 enrolled subjects, then the administration of the next dose group could be started; if DLT occurred in ≥2 out of 3 cases, then the dose escalation would be terminated; if DLT occurred in 1 of the first 3 enrolled subjects in a dose group, then 3 additional subjects was needed to be added to the same dose group (total 6 subjects in the dose group at this time), and if no DLT occurred in the additional 3 subjects, then the next dose group could be started; if DLT occurred in ≥1 of the additional 3 subjects, then no further dose increment was allowed. At the same time, 6 additional subjects should be tested in the previous dose group, until the maximum tolerated dose (MTD) was determined, so at least 6 subjects were required to confirm the MTD dose group. If DLT occurs in ≥2 of the first 3 subjects in the starting antibody dose of 0.3 mg/kg group, then the dose will be backed off to a lower antibody dose of 0.1 mg/kg for dose exploration; if DLT occurs in 1 of the first 3 enrolled subjects in the group, then 3 additional subjects will be added to the dose group (for a total of 6 subjects in the dose group), and if no DLT occurs in the additional 3 subjects, then the administration of the next dose group can be started, and if DLT occurs in ≥1 of the supplemented 3 subjects, then the dose exploration will be reverted to a lower antibody dose of 0.1 mg/kg.

The investigator might choose to continue the study at a dose that was predicted to have a potentially higher benefit/risk ratio based on the data accumulated in study and in agreement with the sponsor. For example, if the overall safety profile of the above trial was favorable and further increase in PG CAR-T cell dose was predicted to have the potential to increase the benefit to the subject, then it was continued to explore the safety of the 5*10⁶ PG CAR-T cell dose/kg. During the dose-escalation phase, safety data would be reviewed by the investigator and combined with PK data to determine if the next dose group was needed to be explored and the dose-escalation regimen was adjusted if necessary. A staggered entry strategy wound be used for the first 3 subjects. An observation period of at least 2 weeks must elapse between subjects 1 and 2, and between subjects 2 and 3.

Subjects who completed the DLT observation period and did not develop DLT during the study of drug dose escalation phase continued receiving the current dose group until disease progression, intolerable toxicity, withdrawal of informed consent by the subject, or other reasons to discontinue study treatment (whichever occurred first), for a maximum of 24 months.

### 3) Dose extension phase.

In order to fully explore the efficacy and safety of "P329G BCMA antibody and P329G CAR-T cells", during the study or at the end of the overall dose-climbing, investigators could select one to two dose groups for dose extension based on the pre-acquired safety and efficacy information, PK data of PG CAR-T cells and antibody, and each dose group might be supplemented with 3-6 subj ects.

### 5. Clinical therapeutic dosing regimen

Information on the administration of P329G BCMA and P329G CAR-T cell preparations was shown in the following Table 12. Subjects should be hospitalized for at least 2 weeks following infusion of the P329G CAR-T cell preparation, and the exact discharge time was determined by the researcher based on the evaluation of the subject's condition.

**Table 12 P325G BCMA and P329G CAR-T cell infusion dosing**

| **Components in the drug to be studied** | P329G BCMA antibody | P329G CAR-T cells |
|---|---|---|
| **Dosage** | As previously described, subjects were dosed at one of 0.3, 1, or 3 mg/kg per infusion of 38497 PG antibody depending on the stage of their entry into the study. Based on newly available data, the dose and dosing schedule might be changed for safety reasons. | As previously described, the target dose of cells to be reinfused to the subject was 2 × 10⁶ cells/kg. Based on newly available data, the dose and dosing schedule might be changed for safety reasons. If the number of the P329G CAR-T cells was not sufficient to reach the lower limit of the administered dose range after apheresis and CAR-T cell preparation, the subject could be treated provided that the generated and quality-test-passed live CAR-positive T cells reached a measurable number. |
| **Route of administration/ protocol** | The 38497 PG antibody was infused intravenously under the supervision of the medical staff of the study center. | The P329G CAR-T cells were infused intravenously under the supervision of the medical staff of the study center. The infusion instructions for the P329G CAR-T cell preparation could be found in the drug handling manual. |
| **Dosing Instructions** | The actual dose administered in the study treatment depended on the body weight (kg) of the subject at the time of apheresis. | The actual dose administered in the study treatment depended on the body weight (kg) of the subject at the time of apheresis. |
| **Dosing schedule** | first dose was infused intravenously on day 4 after the start of clearing lymphatic pretreatment, and thereafter the same dose of antibody was infused intravenously every 3 weeks (from the date of cell infusion) until the onset of PD, intolerable toxicity, withdrawal of informed consent, or other circumstances requiring discontinuation of the drug. Used for a maximum of 24 months. | A total of 1 dose was administered by intravenous infusion on day 5 after clearing lymphatic pretreatment. Single intravenous infusion. |
| | | |

### 6. Experimental results

The Example was the first clinical study of the "P329G BCMA and P329G CAR-T cell" therapeutic method in humans. The results of the aforementioned in vivo and in vitro experiments showed that P329G CAR-T cells had good anti-tumor effects, in the presence of sufficient P329G BCMA antibody, and very low doses of the P329G CAR-T cells could induce full anti-tumor effects at least as effective as those of conventional CAR-T cells at the same dose; P329G CAR-T cell activity was regulated by P329G BCMA antibody and was antibody dose dependent. P329G CAR-T cell activity was regulated by P329G BCMA antibody and showed antibody dose-dependence, with different doses of P329G BCMA antibody inducing different antitumor effects.

The above describes exemplary embodiments of the present invention, and it should be understood by those skilled in the art that these disclosures are only exemplary and that various other substitutions, adaptations and modifications may be made within the scope of the present invention. Accordingly, the present invention is not limited to the specific embodiments enumerated in the specification.

### Sequence Listing

| Sequenc e No. | Name | Amino acid sequence |
|---|---|---|
| SEQ ID NO: 1 | PG CAR | MALPVTALLLPLALLLHAARPEVQLVESGGGLVQPGGSLRLSCAASGFTFSR YWMNWVRQAPGKGLEWVGEITPDSSTINYAPSLKGRFTISRDNAKNSLYLQ MNSLRAEDTAVYYCARPYDYGAWFASWGQGTLVTVSSGGGGSGGGGSGGG GSGGGGSQAVVTQEPSLTVSPGGTVTLTCRSSTGAVTTSNYANWVQEKPDHL FTGLIGGTNKRAPGTPARFSGSLLGGKAALTLSGAQPEDEAEYYCALWYSNH WVFGGGTKLTVLGGGGSIYIWAPLAGTCGVLLLSLVITLYCKRGRKKLLYIFK QPFMRPVQTTQEEDGCSCRFPEEEEGGCELRVKFSRSADAPAYQQGQNQLYN ELNLGRREEYDVLDKRRGRDPEMGGKPRRKNPQEGLYNELQKDKMAEAYS EIGMKGERRRGKGHDGLYQGLSTATKDTYDALHMQALPPR |
| SEQ ID NO: 2 | Amino acid sequence of ADI-38497 VH | QLQLQESGPGLVKPSETLSLTCTVSGGSIVSSSYYWTWIRQPPGKGLEWIGSIS IAGSTYYNPSLKSRVTISVDTSKNQFSLKLSSVTAADTAVYYCARDRGDQILD VWSQGTMVTVSS |
| SEQ ID NO: 3 | Amino acid sequence of ADI-38497 VL | DIQMTQSPSSLSASVGDRVTITCRASQSISRYLNWYQQKPGKAPKLLIYAASS LQSGVPSRFSGSGSGTDFTLTISSLQPEDFATYYCQQKYFDITFGGGTKVEIK |
| SEQ ID NO: 4 | Amino acid sequence of WT IgG1 heavy chain constant region | |
| SEQ ID NO: 5 | Amino acid sequence of P329G IgG1 heavy chain constant region | ASTKGPSVFPLAPSSKSTSGGTAALGCLVKDYFPEPVTVSWNSGALTSGVHTF PAVLQSSGLYSLSSVVTVPSSSLGTQTYICNVNHKPSNTKVDKKVEPKSCDKT HTCPPCPAPELLGGPSVFLFPPKPKDTLMISRTPEVTCVVVDVSHEDPEVKFN WYVDGVEVHNAKTKPREEQYNSTYRVVSVLTVLHQDWLNGKEYKCKVSN KALGAPIEKTISKAKGQPREPQVYTLPPSRDELTKNQVSLTCLVKGFYPSDIAV EWESNGQPENNYKTTPPVLDSDGSFFLYSKLTVDKSRWQQGNVFSCSVMHE |
| | | ALHNHYTQKSLSLSPGK |
| SEQ ID NO: 6 | Amino acid sequence of κ light chain constant region | |
| SEQ ID NO: 7 | Anti-PG scFv fusion protein | EVQLVESGGGLVQPGGSLRLSCAASGFTFSRYWMNWVRQAPGKGLEWVGE ITPDSSTINYAPSLKGRFTISRDNAKNSLYLQMNSLRAEDTAVYYCARPYDYG AWFASWGQGTLVTVSSGGGGSGGGGSGGGGSGGGGSQAVVTQEPSLTVSPG GTVTLTCRSSTGAVTTSNYANWVQEKPDHLFTGLIGGTNKRAPGTPARFSGS LLGGKAALTLSGAQPEDEAEYYCALWYSNHWVFGGGTKLTVLGGGGSSKP TCPPPELLGGPSVFIFPPKPKDTLMISRTPEVTCVVVDVSQDDPEVQFTWYIN NEQVRTARPPLREQQFNSTIRVVSTLPIAHQDWLRGKEFKCKVHNKALPAPIE KTISKARGQPLEPKVYTMGPPREELSSRSVSLTCMINGFYPSDISVEWEKNGK AEDNYKTTPAVLDSDGSYFLYSKLSVPTSEWQRGDVFTCSVMHEALHNHYT QKSISRSPGK |
| SEQ ID NO: 8 | Amino acid sequence of Blue21 CAR | MALPVTALLLPLALLLHAARPDIVLTQSPPSLAMSLGKRATISCRASESVTILG SHLIHWYQQKPGQPPTLLIQLASNVQTGVPARFSGSGSRTDFTLTIDPVEEDD VAVYYCLQSRTIPRTFGGGTKLEIKGSTSGSGKPGSGEGSTKGQIQLVQSGPEL KKPGETVKISCKASGYTFTDYSINWVKRAPGKGLKWMGWINTETREPAYAY DFRGRFAFSLETSASTAYLQINNLKYEDTATYFCALDYSYAMDYWGQGTSVT VSSAAATTTPAPRPPTPAPTIASQPLSLRPEACRPAAGGAVHTRGLDFACDIYI WAPLAGTCGVLLLSLVITLYCKRGRKKLLYIFKQPFMRPVQTTQEEDGCSCRF PEEEEGGCELRVKFSRSADAPAYQQGQNQLYNELNLGRREEYDVLDKRRGR DPEMGGKPRRKNPQEGLYNELQKDKMAEAYSEIGMKGERRRGKGHDGLYQ GLSTATKDTYDALHMQALPPR |
| SEQ ID NO: 9 | Amino acid sequence of ADI-38484 VH | QVQLVQSGAEVKKPGSSVKVSCKASGGTFSNDVISWVRQAPGQGLEWMGV IIPIFGIANYAQKFQGRVTITADESTSTAYMELSSLRSEDTAVYYCARGRGYYS SWLHDIWGQGTMVTVSS |
| SEQ ID NO: 10 | Amino acid sequence of ADI-38484 VL | DIQMTQSPSSLSASVGDRVTITCQASQDITNYLNWYQQKPGKAPKLLIYDAS NLETGVPSRFSGSGSGTDFTFTISSLQPEDIATYYCQQAFDLITFGGGTKVEIK |
| SEQ ID NO: 11 | SP | MALPVTALLLPLALLLHAARP |
| SEQ ID NO: 12 | Anti-PG antibody VH | EVQLVESGGGLVQPGGSLRLSCAASGFTFSRYWMNWVRQAPGKGLEWVGE ITPDSSTINYAPSLKGRFTISRDNAKNSLYLQMNSLRAEDTAVYYCARPYDYG AWFASWGQGTLVTVSS |
| SEQ ID NO: 13 | Anti-PG antibody VL | QAVVTQEPSLTVSPGGTVTLTCRSSTGAVTTSNYANWVQEKPDHLFTGLIGGT NKRAPGTPARFSGSLLGGKAALTLSGAQPEDEAEYYCALWYSNHWVFGGGT KLTVL |
| SEQ ID NO: 14 | G4S | GGGGS |
| SEQ ID NO: 15 | CD8 TMD | IYIWAPLAGTCGVLLLSLVITLYC |
| SEQ ID NO: 16 | 4-1BB CSD | KRGRKKLLYIFKQPFMRPVQTTQEEDGCSCRFPEEEEGGCEL |
| SEQ ID NO: 17 | CD3ζ SSD | RVKFSRSADAPAYQQGQNQLYNELNLGRREEYDVLDKRRGRDPEMGGKPR RKNPQEGLYNELQKDKMAEAYSEIGMKGERRRGKGHDGLYQGLSTATKDT YDALHMQALPPR |
| SEQ ID NO: 18 | CD8α hinge region | TTTPAPRPPTPAPTIASQPLSLRPEACRPAAGGAVHTRGLDFACD |
| SEQ ID NO: 19 | Anti-PG antibody CDR H1 | RYWMN |
| SEQ ID NO: 20 | Anti-PG antibody CDR H2 | EITPDS STINYAPSLKG |
| SEQ ID NO: 21 | Anti-PG antibody CDR H3 | PYDYGAWFAS |
| SEQ ID NO: 22 | Anti-PG antibody CDR L1 | RSSTGAVTTSNYAN |
| SEQ ID NO: 23 | Anti-PG antibody CDR L2 | GTNKRAP |
| SEQ ID NO: 24 | Anti-PG antibody CDR L3 | ALWYSNHWV |
| SEQ ID NO: 25 | ADI-38497 CDR H1 | SSSYYWT |
| SEQ ID NO: 26 | ADI-38497 CDR H2 | SISIAGSTYYNPSLKS |
| SEQ ID NO: 27 | ADI-38497 CDR H3 | DRGDQILDV |
| SEQ ID NO: 28 | ADI-38497 CDR L1 | RASQSISRYLN |
| SEQ ID NO: 29 | ADI-38497 CDR L2 | AASSLQS |
| SEQ ID NO: 30 | ADI-38497 CDR L3 | QQKYFDIT |
| SEQ ID NO: 31 | ADI-38484 CDR H1 | NDVIS |
| SEQ ID NO: 32 | ADI-38484 CDR H2 | VIIPIFGIANYAQKFQG |
| SEQ ID NO: 33 | ADI-38484 CDR H3 | GRGYYSSWLHDI |
| SEQ ID NO: 34 | ADI-38484 CDR L1 | QASQDITNYLN |
| SEQ ID NO: 35 | ADI-38484 CDR L2 | DASNLET |
| SEQ ID NO: 36 | ADI-38484 CDR L3 | QQAFDLIT |
| SEQ ID NO:37 | Amino acid sequence of ADI-34861 VH | QLQLQESGPGLVKPSETLSLTCTVSGGSISSSSYYWGWIRQPPGKGLEWIGSIS YSGSTYYNPSLKSRVTISVDTSKNQFSLKLSSVTAADTAVYYCARDRGDTILD VWGQGTMVTVSS |
| SEQ ID NO:38 | Amino acid sequence of ADI-34861 VL | DIQMTQSPSSLSASVGDRVTITCRASQSISRYLNWYQQKPGKAPKLLIYAASS LQSGVPSRFSGSGSGTDFTLTISSLQPEDFATYYCQQKYFDITFGGGTKVEIK |
| SEQ ID NO:39 | Amino acid sequence of ADI-34857 VH | QVQLVQSGAEVKKPGSSVKVSCKASGGTFSNYAISWVRQAPGQGLEWMGGI IPIFGTANYAQKFQGRVTITADESTSTAYMELSSLRSEDTAVYYCARGRGYYSS WLFDIWGQGTMVTVSS |
| SEQ ID NO:40 | Amino acid sequence of ADI-34857 VL | DIQMTQSPSSLSASVGDRVTITCQASQDITNYLNWYQQKPGKAPKLLIYDAS NLETGVPSRFSGSGSGTDFTFTISSLQPEDIATYYCQQAFDLITFGGGTKVEIK |
| SEQ ID NO:41 | Linker between VH and VL of scFv | GGGGSGGGGSGGGGSGGGGS |

## Claims

1. A pharmaceutical combination comprising
(i) a first component, selected from an immune effector cell (e.g., a T cell, an NK cell) expressing a molecular switch-regulated CAR polypeptide, a nucleic acid molecule encoding the CAR polypeptide, a vector comprising the nucleic acid molecule, and any combination thereof; and
(ii) a second component, which is an antibody or an antigen binding fragment that comprises a P329G mutation and specifically binds to BCMA molecule (also designated as a P329G mutated antibody), for example, the P329G mutated antibody comprises a mutated Fc domain, wherein the amino acid at P329 position according to the EU numbering is mutated into glycine (G), and the mutated Fc domain has a decreased binding to Fcy receptor, compared with the binding of a parent antibody Fc domain without the mutation to Fcy receptor; and
Optionally a pharmaceutically acceptable auxiliary material;
Wherein, the molecular switch-regulated CAR polypeptide comprises
(1) a humanized anti-P329G mutation scFv sequence, wherein the scFv sequence comprises the following sequences capable of specifically binding to an antibody Fc domain comprising a P329G mutation, but not capable of specifically binding to a parent antibody Fc domain without the mutation:
(i) a heavy chain variable region, which comprises according to the Kabat numbering
(a) a heavy chain complementarity determining region CDR H1 shown in amino acid sequence of RYWMN (SEQ ID NO: 19), or a variant of the CDR H1 with no more than 2 amino acid changes or no more than 1 amino acid change;
(b) a CDR H2 shown in amino acid sequence of EITPDSSTINYAPSLKG(SEQ ID NO: 20); or a variant of the CDR H2 with no more than 2 amino acid changes or no more than 1 amino acid change; and
(c) a CDR H3 shown in amino acid sequence of PYDYGAWFAS(SEQ ID NO: 21); or a variant of the CDR H3 with no more than 2 amino acid changes or no more than 1 amino acid change; and
(ii) a light chain variable region, which comprises according to the Kabat numbering
(d) a light chain complementarity determining region (CDR L)1 shown in amino acid sequence of RSSTGAVTTSNYAN(SEQ ID NO: 22); or a variant of the CDR L1 with no more than 2 amino acid changes or no more than 1 amino acid change;
(e) a CDR L2 shown in amino acid sequence of GTNKRAP(SEQ ID NO: 23); or a variant of the CDR L2 with no more than 2 amino acid changes or no more than 1 amino acid change; and
(f) a CDR L3 shown in amino acid sequence of ALWYSNHWV(SEQ ID NO: 24); or a variant of the CDR L3 with no more than 2 amino acid changes or no more than 1 amino acid change; wherein the amino acid change is an addition, deletion or substitution of an amino acid;
(2) a hinge region/a spacer region, selected from a group consisting of
(i) a (G₄S)ₙ, (SG₄)ₙ, or G₄(SG₄)ₙ peptide linker, wherein "n" is an integer from 1 to 10, such as an integer from 1 to 4; for example, a sequence shown in SEQ ID NO: 14;
(ii) a CD8α hinge region, or a variant thereof having 1-5 amino acid modifications, for example, a sequence shown in SEQ ID NO: 18 or a variant thereof having 1-2 amino acid modifications;
(3) a transmembrane region (TM), selected from a group consisting of CD8 transmembrane domain, or a variant thereof having 1-5 amino acid modifications, for example, a sequence shown in SEQ ID NO: 15, or a variant thereof having 1-2 amino acid modifications;
(4) a co-stimulatory signal domain (CSD), selected from a group consisting of 4-1BB co-stimulatory domain, or a variant thereof having 1-5 amino acid modifications, for example, a sequence shown in SEQ ID NO: 16, or a variant thereof having 1-2 amino acid modifications;
(5) a stimulatory signal domain (SSD), which is a CD3ζ signaling domain, or a variant thereof having 1-10 amino acid modifications, for example, a sequence shown in SEQ ID NO: 17, or a variant thereof having 1-10, or 1-5 amino acid modifications;
Preferably, the CAR polypeptide comprises
(1) a humanized anti-P329G mutation scFv sequence, wherein the scFv sequence comprises the following sequences capable of specifically binding to an antibody Fc domain comprising a P329G mutation, but not capable of specifically binding to a parent antibody Fc domain without the mutation:
(i) a heavy chain variable region, which comprises according to the Kabat numbering
(a) CDR H1 shown in amino acid sequence of RYWMN(SEQ ID NO: 19);
(b) CDR H2 shown in amino acid sequence of EITPDSSTINYAPSLKG(SEQ ID NO: 20); and
(c) CDR H3 shown in amino acid sequence of PYDYGAWFAS(SEQ ID NO: 21); and
(ii) a light chain variable region, which comprises according to the Kabat numbering
(d) CDR L1 shown in amino acid sequence of RSSTGAVTTSNYAN(SEQ ID NO: 22);
(e) CDR L2 shown in amino acid sequence of GTNKRAP(SEQ ID NO: 23); and
(f) CDR L3 shown in amino acid sequence of ALWYSNHWV(SEQ ID NO: 24);
for example, (i) a heavy chain variable region, which comprises a sequence shown in SEQ ID NO: 12, or a sequence having at least 90%, 91%, 92%, 93%, 94%, 95%, 96%, 97%, 98% or 99% identity thereto, and
(ii) a light chain variable region, which comprises a sequence shown in SEQ ID NO: 13, or a sequence having at least 90%, 91%, 92%, 93%, 94%, 95%, 96%, 97%, 98% or 99% identity thereto;
for example, (i) a heavy chain variable region, which comprises a sequence shown in SEQ ID NO: 12, and (ii) a light chain variable region, which comprises a sequence shown in SEQ ID NO: 13;
(2) a hinge region/a spacer region, selected from a group consisting of
(i) a (G₄S)ₙ, (SG₄)ₙ, or G₄(SG₄)ₙ peptide linker, wherein "n" is an integer from 1 to 4, for example, a sequence shown in SEQ ID NO: 14;
(ii) a CD8α hinge region sequence shown in SEQ ID NO: 18, or a variant thereof having 1 amino acid modification
(3) a transmembrane region (TM), selected from a group consisting of CD8 transmembrane domain shown in SEQ ID NO: 15, or a variant thereof having 1 amino acid modification
(4) a co-stimulatory signal domain (CSD), selected from a group consisting of 4-1BB co-stimulatory domain shown in SEQ ID NO: 16, or a variant thereof having 1 amino acid modification
(5) a stimulatory signal domain (SSD), selected from a group consisting of CD3ζ signaling domain shown in SEQ ID NO: 17, or a variant thereof having 1 amino acid modification wherein the amino acid modification is an addition, deletion or substitution of an amino acid.

2. The pharmaceutical combination according to claim 1, wherein the antibody or the antigen binding fragment that comprises a P329G mutation and specifically binds to BCMA molecule comprises a heavy chain variable region and a light chain variable region, wherein:
(a) the heavy chain variable region comprises according to the Kabat numbering CDR H1 shown in amino acid sequence of SSSYYWT (SEQ ID NO: 25); or a variant of the CDR H1 with no more than 2 amino acid changes or no more than 1 amino acid change; CDR H2 shown in amino acid sequence of SISIAGSTYYNPSLKS(SEQ ID NO: 26); or a variant of the CDR H2 with no more than 2 amino acid changes or no more than 1 amino acid change; and CDR H3 shown in amino acid sequence of DRGDQILDV (SEQ ID NO: 27); or a variant of the CDR H3 with no more than 2 amino acid changes or no more than 1 amino acid change; the light chain variable region comprises according to the Kabat numbering CDR L1 shown in amino acid sequence of RASQSISRYLN(SEQ ID NO: 28); or a variant of the CDR L1 with no more than 2 amino acid changes or no more than 1 amino acid change; CDR L2 shown in amino acid sequence of AASSLQS(SEQ ID NO: 29); or a variant of the CDR L2 with no more than 2 amino acid changes or no more than 1 amino acid change; and CDR L3 shown in amino acid sequence of QQKYFDIT(SEQ ID NO: 30); or a variant of the CDR L3 with no more than 2 amino acid changes or no more than 1 amino acid change;
(b) the heavy chain variable region comprises according to the Kabat numbering CDR H1 shown in amino acid sequence of NDVIS (SEQ ID NO: 31); or a variant of the CDR H1 with no more than 2 amino acid changes or no more than 1 amino acid change; CDR H2 shown in amino acid sequence of VIIPIFGIANYAQKFQG(SEQ ID NO: 32); or a variant of the CDR H2 with no more than 2 amino acid changes or no more than 1 amino acid change; and CDR H3 shown in amino acid sequence of GRGYYSSWLHDI(SEQ ID NO: 33); or a variant of the CDR H3 with no more than 2 amino acid changes or no more than 1 amino acid change; the light chain variable region comprises according to the Kabat numbering CDR L1 shown in amino acid sequence of QASQDITNYLN(SEQ ID NO: 34); or a variant of the CDR L1 with no more than 2 amino acid changes or no more than 1 amino acid change; CDR L2 shown in amino acid sequence of DASNLET(SEQ ID NO: 35); or a variant of the CDR L2 with no more than 2 amino acid changes or no more than 1 amino acid change; and CDR L3 shown in amino acid sequence of QQAFDLIT(SEQ ID NO: 36); or a variant of the CDR L3 with no more than 2 amino acid changes or no more than 1 amino acid change;
wherein the amino acid change is an addition, deletion or substitution of an amino acid;
for example, wherein:
(a) the heavy chain variable region comprises according to the Kabat numbering CDR H1 shown in amino acid sequence of SSSYYWT (SEQ ID NO: 25); CDR H2 shown in amino acid sequence of SISIAGSTYYNPSLKS(SEQ ID NO: 26); and CDR H3 shown in amino acid sequence of DRGDQILDV (SEQ ID NO: 27); the light chain variable region comprises according to the Kabat numbering CDR L1 shown in amino acid sequence of RASQSISRYLN(SEQ ID NO: 28); CDR L2 shown in amino acid sequence of AASSLQS(SEQ ID NO: 29); and CDR L3 shown in amino acid sequence of QQKYFDIT(SEQ ID NO: 30);
(b) the heavy chain variable region comprises according to the Kabat numbering CDR H1 shown in amino acid sequence of NDVIS(SEQ ID NO: 31); CDR H2 shown in amino acid sequence of VIIPIFGIANYAQKFQG(SEQ ID NO: 32); and CDR H3 shown in amino acid sequence of GRGYYSSWLHDI(SEQ ID NO: 33); the light chain variable region comprises according to the Kabat numbering CDR L1 shown in amino acid sequence of QASQDITNYLN(SEQ ID NO: 34); CDR L2 shown in amino acid sequence of DASNLET(SEQ ID NO: 35); and CDR L3 shown in amino acid sequence of QQAFDLIT(SEQ ID NO: 36);
for example, wherein:
(a) the heavy chain variable region comprises a sequence shown in SEQ ID NO: 2, or a sequence having at least 90%, 91%, 92%, 93%, 94%, 95%, 96%, 97%, 98% or 99% identity thereto, and the light chain variable region comprises a sequence shown in SEQ ID NO: 3, or a sequence having at least 90%, 91%, 92%, 93%, 94%, 95%, 96%, 97%, 98% or 99% identity thereto;
(b) the heavy chain variable region comprises a sequence shown in SEQ ID NO: 9, or a sequence having at least 90%, 91%, 92%, 93%, 94%, 95%, 96%, 97%, 98% or 99% identity thereto, and the light chain variable region comprises a sequence of SEQ ID NO: 10, or a sequence having at least 90%, 91%, 92%, 93%, 94%, 95%, 96%, 97%, 98% or 99% identity thereto;
for example, wherein:
(a) the heavy chain variable region comprises a sequence shown in SEQ ID NO: 2, and the light chain variable region comprises a sequence shown in SEQ ID NO: 3;
(b) the heavy chain variable region comprises a sequence shown in SEQ ID NO: 9, and the light chain variable region comprises a sequence shown in SEQ ID NO: 10;
for example, the antibody is IgG1, IgG2, IgG3, or IgG4 antibody; preferably, IgG1 or IgG4 antibody; more preferably, IgG1 antibody;
for example, the antigen binding fragment is Fab, Fab', F(ab')₂, Fv, single chain Fv, single chain Fab or diabody.

3. The pharmaceutical combination according to claim 1 or 2, wherein the mutated Fc domain is a mutated Fc domain of IgG1, IgG2, IgG3, or IgG4 antibody, preferably, the mutated Fc domain is a mutated Fc domain of IgG1, or IgG4 antibody; more preferably, the mutated Fc domain is a mutated Fc domain of IgG1 antibody;
for example, the antibody or antigen binding fragment comprises a heavy chain constant region sequence shown in SEQ ID NO: 5, or a sequence having at least 90%, 91%, 92%, 93%, 94%, 95%, 96%, 97%, 98% or 99% identity thereto, and wherein the amino acid at P329 position according to the EU numbering is mutated into G;
for example, the antibody or antigen binding fragment comprises a heavy chain constant region sequence shown in SEQ ID NO: 5, or a sequence having at least 90%, 91%, 92%, 93%, 94%, 95%, 96%, 97%, 98% or 99% identity thereto, and wherein the amino acid at P329 position according to the EU numbering is mutated into G; and a light chain constant region sequence shown in SEQ ID NO: 6, or a sequence having at least 90%, 91%, 92%, 93%, 94%, 95%, 96%, 97%, 98% or 99% identity thereto;
for example, the antibody or antigen binding fragment comprises a heavy chain constant region sequence shown in SEQ ID NO: 5 and a light chain constant region sequence shown in SEQ ID NO: 6.

4. The pharmaceutical combination according to any one of claims 1-3, wherein the molecular switch-regulated CAR polypeptide further comprises a signal peptide sequence at N-terminus, for example, a signal peptide sequence shown in SEQ ID NO: 11,
preferably, the molecular switch-regulated CAR polypeptide has an amino acid sequence shown in SEQ ID NO: 1, or a sequence having at least 90%, 91%, 92%, 93%, 94%, 95%, 96%, 97%, 98% or 99% identity thereto.

5. The pharmaceutical combination according to any one of claims 1-4, wherein the nucleic acid molecule encoding CAR polypeptide is one encoding the CAR polypeptide in the pharmaceutical combination of any one of claims 1-4, for example, the nucleic acid molecule is one encoding an amino acid sequence shown in SEQ ID NO: 1, or is one encoding an amino acid sequence having at least 90%, 91%, 92%, 93%, 94%, 95%, 96%, 97%, 98%, or 99% identity thereto.

6. The pharmaceutical combination according to any one of claims 1-4, wherein the vector comprises the nucleic acid molecule encoding the CAR polypeptide in the pharmaceutical combination of any one of claims 1-4, for example, the vector is selected from a group consisting of a DNA vector, an RNA vector, a plasmid, a lentiviral vector, an adenovirus vector, or a retroviral vector.

7. The pharmaceutical combination according to any one of claims 1-4, wherein the immune effector cell is a T cell, NK cell prepared from an autologous T cell, NK cell, or an allogeneic T cell, NK cell, and expressing the molecular switch-regulated CAR polypeptide in the pharmaceutical combination of any one of claims 1-4, for example, the immune effector cell is prepared from T cell, NK cell isolated from human PBMC and expressing the molecular switch-regulated CAR polypeptide in the pharmaceutical combination of any one of claims 1-4.

8. The pharmaceutical combination according to any one of claims 1-7, wherein
(i) the immune effector cell expressing the CAR polypeptide in the pharmaceutical combination of any one of claims 1-4 is administered intravenously one or more times to a subject at a dose of 1×10⁶ cells/kg body weight - 10×10⁶ cells/kg body weight, for example 1×10⁶ cells/kg body weight, 2×10⁶ cells/kg body weight, 3×10⁶ cells/kg body weight, 5×10⁶ cells/kg body weight, 7×10⁶ cells/kg body weight, 9×10⁶ cells/kg body weight, 10×10⁶ cells/kg body weight; and
(ii) the P329G mutated antibody in the pharmaceutical combination of any one of claims 1-4 is administered, preferably parenterally, more preferably intravenously, to a subject in a form of a dose unit of 0.1-10mg/kg, preferably 0.1mg/kg, 0.3mg/kg, 0.5mg/kg, 1mg/kg, 3mg/kg, 5mg/kg, 7mg/kg, 9mg/kg, 10mg/kg.

9. The pharmaceutical combination according to claim 8, wherein (i) and (ii) are administered separately, simultaneously, or sequentially, e.g., (ii) is administered on the first day, (i) is administered intravenously on the same day, and then (ii) is administered multiple times at a certain frequency, meanwhile an in vivo PK concentration of (i) and a desired therapeutic efficacy endpoint are monitored to determine whether to administer (i) multiple times; or
(i) is administered on the first day, (ii) is administered intravenously on the second day, and then
(ii) is administered multiple times at a certain frequency, meanwhile an in vivo PK concentration of (i) and a desired therapeutic efficacy endpoint are monitored to determine whether to administer (i) multiple times; or
(ii) is administered on the first day, (i) is administered intravenously on the second day, and then
(ii) is administered multiple times at a certain frequency, meanwhile an in vivo PK concentration of (i) and a desired therapeutic efficacy endpoint are monitored to determine whether to administer (i) multiple times;
For example, (i) and (ii) are each administered once, and then (ii) is administered multiple times at a frequency of once every 3-4 days, once a week, once every two weeks, once every three weeks, or once every four weeks, meanwhile an in vivo PK concentration of (i) and a desired therapeutic efficacy endpoint are monitored to determine whether to administer (i) multiple times.

10. Use of the pharmaceutical combination according to any one of claims 1-9 for treating a BCMA-related disease in a subject, comprising administering to the subject a therapeutically effective amount of the pharmaceutical combination according to any one of claims 1-9, preferably, the BCMA-related disease is, for example, a cancer expressing or overexpressing BCMA, for example, the cancer is relapsed/refractory multiple myeloma (RRMM).

11. Use of the pharmaceutical combination according to any one of claims 1-9 in the manufacture of a medicament for the treatment of a BCMA-related disease, for example, the BCMA-related disease is a cancer expressing or overexpressing BCMA, for example, the cancer is relapsed/refractory multiple myeloma (RRMM).

12. A method for treating a BCMA-related disease, comprising administering to a subject a therapeutically effective amount of the pharmaceutical combination of any one of claims 1-9, for example, the BCMA-related disease is a cancer expressing or overexpressing BCMA, for example, the cancer is relapsed/refractory multiple myeloma (RRMM).

13. A kit of parts, comprising the pharmaceutical combination of any one of claims 1-9, preferably the kit of parts is in the form of a pharmaceutical dose unit.

14. A pharmaceutical complex which is formed by the binding of
(i) an immune effector cell (e.g., a T cell and an NK cell) expressing a molecular switch-regulated CAR polypeptide in the pharmaceutical combination of any one of claims 1-4; to
(ii) an antibody or an antigen binding fragment that comprises a P329G mutation and specifically binds to BCMA molecule (also designated as a P329G mutated antibody), for example, the P329G mutated antibody comprises a mutated Fc domain, wherein the amino acid at P329 position according to the EU numbering is mutated into glycine (G), and the mutated Fc domain has a decreased binding to Fcy receptor, compared with the binding of a parent antibody Fc domain without the mutation to Fcy receptor;
Wherein the complex is formed by binding of the humanized anti-P329G mutation scFv sequence in the extracellular domain of the CAR polypeptide in the immune effector cell to the Fc domain of the P329G mutated antibody;
For example, wherein the immune effector cell is a T cell, NK cell prepared from an autologous T cell, NK cell, or an allogeneic T cell, NK cell, and expressing the molecular switch-regulated CAR polypeptide in the pharmaceutical combination of any one of claims 1-4, for example, the immune effector cell is prepared from T cell, NK cell isolated from human PBMC and expressing the molecular switch-regulated CAR polypeptide in the pharmaceutical combination of any one of claims 1-4, for example, wherein the P329G mutated antibody is ADI-38497 PG Ab and/or ADI-38484 PG Ab.

15. Use of the pharmaceutical complex according to claim 14 for the treatment of a BCMA-related disease in a subject, preferably the BCMA-related disease is, for example, a cancer expressing or overexpressing BCMA, for example, the cancer is relapsed/refractory multiple myeloma (RRMM).
